(19) Europäisches Patentamt
European Patent Office
Office européen des brevets

(11) EP 2 289 891 A2

(12) **DEMANDE DE BREVET EUROPEEN**

(43) Date de publication:
**02.03.2011 Bulletin 2011/09**

(51) Int Cl.:
***C07D 401/06*** $^{(2006.01)}$ ***C07D 401/14*** $^{(2006.01)}$
***C07D 401/12*** $^{(2006.01)}$ ***A61K 31/47*** $^{(2006.01)}$

(21) Numéro de dépôt: **10185850.4**

(22) Date de dépôt: **04.08.2006**

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **04.08.2005 FR 0508351**

(62) Numéro(s) de document de la (des) demande(s) initiale(s) en application de l'article 76 CBE:
**06794293.8 / 1 919 894**

(71) Demandeurs:
- **Palumed S.A.**
  **31320 Castanet-Tolosan (FR)**
- **Centre National de la Recherche Scientifique (C.N.R.S)**
  **75016 Paris (FR)**

(72) Inventeurs:
- **Deraeve, Céline**
  **31400 TOULOUSE (FR)**
- **Pitie, Marguerite**
  **31520 RAMONVILLE (FR)**
- **Boldron, Christophe**
  **31400 TOULOUSE (FR)**
- **Meunier, Bernard**
  **31320 CASTANET (FR)**

(74) Mandataire: **Blot, Philippe Robert Emile**
**Cabinet Lavoix**
**2, place d'Estienne d'Orves**
**75441 Paris Cedex 09 (FR)**

Remarques:
Cette demande a été déposée le 01-10-2010 comme demande divisionnaire de la demande mentionnée sous le code INID 62.

(54) **Nouveaux dérivés de polyquinoléines et leur utilisation thérapeutique.**

(57) L'invention concerne des composés de formule générale (I), leur procédé de préparation, ainsi que leur utilisation en tant qu'agent thérapeutique.

EP 2 289 891 A2

**Description**

La présente invention concerne de nouveaux dérivés poly-quinoléines, leur procédé de préparation et leur utilisation en tant qu'agents thérapeutiques.

Plus précisément, les composés selon la présente invention présentent la propriété d'être des ligands de métaux et/ou de dissoudre les agrégats amyloïdes et sont particulièrement utiles dans le traitement de maladies neurodégénératives.

Différentes revues récapitulent un ensemble de données mettant en évidence que beaucoup de désordres neurodégénératifs progressifs et lents sont associés à : (i) un stress oxydant, (ii) un mauvais repliement de protéines conduisant à la formation d'agrégats, de fibrilles, de profibrilles ou de plaques, (iii) une accumulation de ces protéines, (iv) une perte de synapses, (v) une homéostasie des ions métalliques altérée, (vi) des défauts dans les axones et le transport dendritique, (vii) une mort neuronale. (E. Bossy-Wetzel et al., Nature Medecine, 2004, S2-S9 ; K. J. Barnham et al., Nature Rev. Drug Discov., 2004, 3, 205-214; M. P. Mattson, Nature, 2004, 430, 631-639; P. M. Doraiswamy et al., The Lancet Neurol., 2004, 3, 431-434).

En effet, de nombreuses études ont récemment mis en évidence le rôle déterminant des ions métalliques (cuivre, zinc, fer, aluminium, manganèse ...) dans la modification du repliement ou de l'agrégation de protéines, conduisant ainsi à des pathologies graves. Ce rôle néfaste d'interactions hors normes d'ions métalliques avec des protéines vient d'être souligné dans de nombreuses maladies neurodégénératives (par exemple : la maladie d'Alzheimer, des encéphalopathies spongiformes, la maladie de Parkinson, la maladie d'Huntington, la sclérose latérale amyotrophique, etc.) ou lors de l'évolution néfaste de certains handicaps comme dans le cas de la trisomie 21. Une ou des protéines spécifiques sont associées à chaque maladie et il a été mis en évidence que des chélateurs d'ions métalliques peuvent être actifs pour limiter leurs mauvais repliements induits par les métaux.

Dans certaines encéphalopathies comme la maladie de Kreutzfeld-Jacob et de son nouveau variant, il est maintenant admis que ces maladies sont liées à la transformation d'une protéine de type prion (PrP) en sa forme pathologique et infectieuse dite "scrapie" ($PrP^{Sc}$). Les ions cuivriques sont impliqués dans cette modification conformationnelle (formation de feuillets bêta) des prions qui deviennent résistants aux protéases et insolubles dans les détergents non dénaturants. Des travaux récents viennent de montrer qu'un ligand tel que le disulfate de la bathocuproïne peut restaurer *in vitro* la sensibilité de la protéine "scrapie" $PrP^{sc}$ aux protéases et sa solubilité (E. Quaglio et al., J. Biol. Chem., 2001, 276, 11432-11438).

Dans le cas de la maladie de Parkinson, l'$\alpha$-synucléine interagit avec des ions ferriques. Il a été proposé que ces ions facilitent la formation de radicaux hydroxyles, particulièrement oxydants et des études par IRM *post mortem* ont mis en évidence de fortes concentrations d'ions ferriques dans la *substancia nigra* de malades (une zone du cerveau où les neurones dopaminergiques sont plus sélectivement touchés dans cette maladie). L'emploi de chélateurs comme le Clioquinol réduit la toxicité du 1-méthyl-4-phényl-1,2,3,6-tétrapyridine, une toxine induisant le parkinsonisme, sur les souris (D. Kaur et al., Neuron 2003, 37, 899-909).

Dans le cas de la maladie d'Alzheimer (M. P. Mattson, Nature, 2004, 430, 631-639; M. Citron, Nature Rev. Neurosci., 2004, 5, 677-685), la pathologie est liée à l'agrégation dans le cerveau de peptides $\beta$-amyloïdes conduisant à la formation des plaques amyloïdes. Cette agrégation peut être induite par les ions Cu(II) et Zn(II) mais aussi, dans une moindre mesure, Fe(III). L'accumulation au sein de ces plaques d'ions métalliques à activité rédox est probablement à l'origine du stress oxydant important (*via* la production de $H_2O_2$) lui-même à l'origine des dommages créés sur les neurones du cerveau, conduisant à une perte irréversible des facultés intellectuelles (M. P. Cuajungco et al., Ann. N. Y. Acad. Sci., 2000, 920, 292-304; C. S. Atwood et al., Met. Ions Biol. Syst., 1999, 36, 309-364). Le fait que les premiers essais effectués avec un ligand d'ions métalliques comme le Clioquinol conduisent à des améliorations dans la maladie d'Alzheimer (R. A. Cherny et al., Neuron, 2001, 30, 665-676) indiquent que des approches thérapeutiques sont possibles à l'aide de chélateurs d'ions métalliques.

Toutefois, ces chélateurs doivent posséder les propriétés suivantes pour être susceptibles d'être utilisés comme médicaments dans le traitement de maladies neurodégénératives :

- (a) être de faible masse moléculaire et ne pas être trop fortement chargés afin de pouvoir passer les différentes barrières (d'abord intestinale dans le cas d'une molécule utilisée par voie orale et ensuite, et de manière réversible, la barrière hémato-méningée pour aller chélater les ions métalliques en excès dans les protéines pathogènes),
- (b) présenter une structure modifiable afin d'ajuster la sélectivité de chélation à certains ions métalliques (une forte chélation non spécifique conduirait à une déplétion généralisée des ions métalliques, y compris ceux des métalloenzymes essentielles au fonctionnement de l'organisme) ou de permettre de moduler leur biodistribution dans l'organisme.

Des chélateurs comportant un motif quinoléine substitué en position 8 par un hétéroatome (comme les dérivés de 8-hydroxyquinoléine par exemple) sont des candidats pour chélater les excès d'ions métalliques impliqués dans les

maladies neurodégénératives. On peut s'attendre à ce que ce type de ligand forme souvent des complexes de cuivre, de zinc ou de fer (des ions métalliques associés aux agrégations protéiques et même au stress oxydant pour le cuivre et le fer) comportant deux (et même trois pour le fer) ligands autour de l'ion métallique (Sillen, L. G. et al., Stability Constants of Metal-Ion Complexes, The Chemical Society London Publication, 1971).

Des dérivés de bis-quinoléines ont été décrits, mais rarement comme agents pour le traitement de maladies du système nerveux potentiels. Il en est ainsi de WO 2004/007461 qui décrit la propriété de chélateur métallique. Néanmoins, ce document décrit essentiellement des composés mono-quinoléine. Par ailleurs, EP 0 443 862 décrit des dérivés agonistes du récepteur NMDA et ne suggère aucunement l'activité de chélateur métallique des composés décrits. Enfin, Stockwell et al. dans J. Am. Chem. Soc. 1999, 10662-10663 décrivent l'activité biologique des composés 2, 2'-(imino)bis(8-quinoléinol) et ses dérivés 2, 2'-(methylimino)- et 2, 2'-(n-butylimino)-bis(8-quinoléinol).

Il a maintenant été découvert, et ce de façon totalement inattendue, que les composés 2,2'- ou 8,8'-poly-quinoléines selon l'invention présentaient une forte activité chélatrice pour les métaux et/ou une capacité à dissoudre les agrégats amyloïdes.

Par « aggrégats amyloïdes », on entend ici une structure polymérique de peptides Aβ générée par interaction secondaire, tertiaire ou quaternaire (de feuillet β par exemple) ou par coordination biométallique sur le peptide (E. Scarpini et al., The Lancet Neurology, 2003, 2, 539-547 ; E. Gaggeli et al., Chem. Rev., 2006, 106, 1995-2044 ; A. B. Clippingdale et al., J. Peptide Sc., 2001, 7, 227-249).

Ces composés sont utiles comme médicaments pour le traitement et/ou la prévention de maladies neurodégénératives, notamment de la maladie d'Alzheimer, de Parkinson, des encéphalopathies spongiformes, la maladie d'Huntington, la sclérose latérale amyotrophique ou la trisomie 21.

Les présents inventeurs ont ainsi développé des chélateurs comportant plusieurs motifs quinoléine substitués (en position 2 ou 8) de petite taille et suffisamment hydrophobes pour pouvoir passer les barrières. Ils ont ainsi démontré que ces structures favorisaient l'interaction des hétérocycles de la molécule sur le même ion métallique et que des substitutions sur ces ligands, introduites de façon contrôlée, pouvaient moduler leur action vis-à-vis des protéines impliquées dans les maladies neurodégénératives : les propriétés de chélation en particulier des ions Cu(II), Zn(II) et Fe (III) impliquées dans ces maladies, d'hydrophobicité, de capacité à désagréger les protéines impliquées dans les maladies neurodégénératives que ce soit ou non en présence d'ion métallique, ou de diminuer le stress oxydant qu'elles peuvent induire.

La présente invention concerne l'utilisation des composés de formule (I)

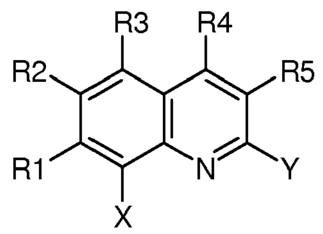

(I)

pour la préparation de compositions pharmaceutiques pour chélater les ions métalliques et/ou pour dissoudre les agrégats amyloïdes telle que dans la formule (I)

soit

X représente un groupe -OR, -NRR', $-S(O)_pR$, -OCOR, -OCOOR, et

Y représente un groupe de formule :

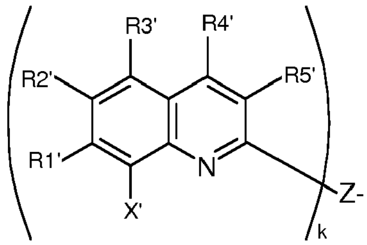

(IY)

dans laquelle X' représente un groupe -OR, -NRR', $-S(O)_pR$, -OCOR, -OCOOR et Z représente un groupe de formule $-(A)_m-(Alk)_n-(A')_{m'}-(Alk')_{n'}-(A'')_{m''}-(Alk'')_{n''}$ où m, n, m', n', m", n" identiques ou différents représentent indépendamment 0 ou 1, étant entendu que au moins un des m, n, m', n', m", n" soit égal à 1,
A, A' et A" identiques ou différents représentent indépendamment un groupe choisi parmi -NR-, $-S(O)_p-$, -O-, -C(=O)-, ou un cycle de 4 à 11 chaînons choisi parmi les cycloalkyles, hétérocycles, aryles, hétéroaryles, ledit cycle étant éventuellement substitué par un ou plusieurs substituants choisis parmi Alkyle, OR, NRR', $CF_3$, Hal, CN, $S(O)_pR$, COOR, OCOOR, CONRR', NRCOOR' ; Alk, Alk' et Alk" identiques ou différents représentent indépendamment un groupe -alkyle-éventuellement substitué par un ou plusieurs substituants choisis parmi OR, NRR', $CF_3$, Hal, CN, $S(O)_pR$, COOR, OCOOR, CONRR', NRCOOR' ;
ou soit
X représente un groupe de formule :

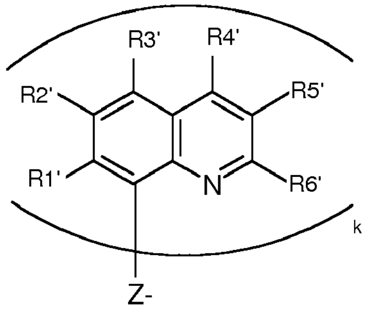

(IX)

dans laquelle Z représente un groupe de formule $-(A)_m-(Alk)_n-(A')_{m'}-(Alk')_{n'}-(A'')_{m''}-(Alk'')_{n''}$ où m, n, m', n', m", n" identiques ou différents représentent indépendamment 0 ou 1, étant entendu que au moins un des m, n, m', n', m", n" soit égal à 1, A, A' et A" identiques ou différents représentent indépendamment un groupe choisi parmi -NR-, $-S(O)_p-$, -O-, -C(=O)-, ou un cycle de 4 à 11 chaînons choisi parmi les cycloalkyles, hétérocycles, aryles, hétéroaryles, ledit cycle étant éventuellement substitué par un ou plusieurs substituants choisis parmi Alkyle, OR, NRR', $CF_3$, Hal, CN, $S(O)_pR$, COOR, OCOOR, CONRR', NRCOOR' ; Alk, Alk' et Alk" identiques ou différents représentent indépendamment un groupe -alkyle-éventuellement substitué par un ou plusieurs substituants choisis parmi OR, NRR', $CF_3$, Hal, CN, $S(O)_pR$, COOR, OCOOR, CONRR', NRCOOR' ; Y représente un groupe choisi parmi H, OR, NRR', Hal, -CN, $-CF_3$, alkyle éventuellement substitué par un ou plusieurs substituants choisis parmi OR, NRR', $CF_3$, Hal, CN, $S(O)_pR$, COOR, OCOOR, CONRR', NRCOOR' ; et
R et R' identiques ou différents représentent indépendamment un atome d'hydrogène ou un groupe cycloalkyle ou alkyle éventuellement substitué par un ou plusieurs groupes choisis parmi OR, NRR', Hal, -CN, $-CF_3$, $S(O)_pR$, COOR, OCOOR, CONRR', NRCOOR'; hétéroaryle ;
R1, R2, R3, R4, R5, R1', R2', R3', R4', R5', R6' identiques ou différents représente indépendamment un groupe ou atome choisi parmi H, OR, NRR', Hal, -CN, $-CF_3$, $S(O)_pR$, COOR, OCOOR, CONRR', NRCOOR', alkyle éventuellement substitué par un ou plusieurs groupes choisis parmi OR, NRR', Hal, -CN, $-CF_3$, $S(O)_pR$, COOR, OCOOR, CONRR', NRCOOR' ;
k représente 1 ou 2 ;
p représente 0, 1 ou 2 ;
ainsi que leurs stéréoisomères ou leurs mélanges, leurs formes tautomères, leurs hydrates, solvates, leurs sels, formes

libres et esters pharmaceutiquement acceptables,
à l'exception des composés pour lesquels :

X représente un groupe -OH, et
Y représente un groupe de formule :

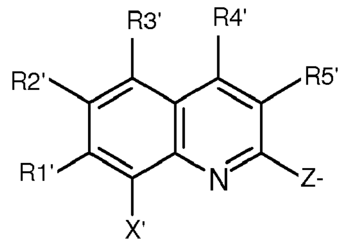

dans laquelle X' représente un groupe -OH, et
Z représente un groupe choisi parmi -NH-, -NBu-, -NMe-, et R1, R2, R3, R4, R5, R1', R2', R3', R4', R5', R6' sont égaux à H.

Plus préférentiellement, les composés pour la présente utilisation sont choisis parmi :

Diméthyl-bis[8-(acétyloxy)-2-quinoléinyl]propanedioate
2,2'-Méthanediyl-bis(8-hydroxyquinoléine)
2,2'-Méthanediyl-bis(5-chloro-8-hydroxyquinoléine)
2,2'-(2,2-Propanediyl)-bis(8-hydroxyquinoléine)
2,2'-(2,2-Propanediyl)-bis(5-chloro-8-hydroxyquinoléine)
2,2'-(Difluorométhanediyl)-bis(8-hydroxyquinoléine)
Bis(8-hydroxy-2-quinoléinyl)méthanone
2,2'-(1,2-Ethanediyl)-bis[8-(méthyloxy)quinoléine]
2,2'-(1,2-Ethanediyl)-bis(8-hydroxyquinoléine)
2,2'-(1,2-Ethanediyl)-bis(5-chloro-8-hydroxyquinoléine)
2,2'-(1,2-Ethanediyl)-bis(5-chloro-7-iodo-8-hydroxyquinoléine)
2,2'-(1,4-Butanediyl)-bis[8-(méthyloxy)quinoléine]
2,2'-(1,4-Butanediyl)-bis(8-hydroxyquinoléine)
2,2',2"-(1,2,3-Propanetriyl)-tris[8-(méthyloxy)quinoléine]
2,2',2"-(1,2,3-Propanetriyl)-tris(8-hydroxyquinoléine)
Bis(8-quinoléinyl)amine
*N,N'*-di-8-quinoléinyl-1,3-propanediamine
*N,N'*-bis(2-méthyl-8-quinoléinyl)-1,2-éthanediamine
8,8'-Oxydiquinoléine
Diméthyl bis{7-(méthyloxy)8-[phénylméthyl)oxy]-2-quinoléinyl}propanedioate
2,2'-(Méthanediyl)-bis(7-méthyloxy-8-hydroxy-2-quinoléinium) dichlorure
2,2'-(1,2-Ethanediyl)-bis[7-(méthyloxy)-8-quinoléinol]
8-Hydroxy-*N*-(8-hydroxy-2-quinoléinyl)-2-quinoléinecarboxamide
2-{[8-Hydroxy-2-quinoléinyl)amino]méthyl}-8-quinoléinol
2,2'-(Iminodiméthanediyl)di(*N*-boc-8-quinoléinamine)
2,2'-(Iminodiméthanediyl)di(8-quinoléinamine)
2,2',2"-(Nitrilotriméthanediyl)tri(*N*-boc-8-quinoléinamine)
2,2',2"-(Nitrilotriméthanediyl)tri(8-quinoléinamine)
2,2'-[(Butylimino)diméthanediyl]di(*N*-boc-8-quinoléinamine)
2,2'-[(Butylimino)diméthanediyl]di(8-quinoléinamine)
*N*-Butyl-2,2'-imino-bis(8-quinoléinamine)
*N*-8-Quinoléinyl-8-quinoléinecarboxamide
*N*-8-Quinoléinyl-8-quinoléinesulfonamide

ainsi que leurs stéréoisomères ou leur mélanges, leurs formes tautomères, leurs hydrates, solvates, leurs sels, formes libres et esters pharmaceutiquement acceptables.

Selon un autre objet, la présente invention concerne également l'utilisation des composés de formule (I)

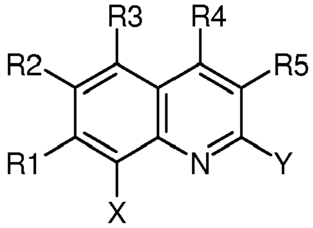

(I)

pour la préparation de compositions pharmaceutiques pour prévenir et/ou traiter les maladies affectant le système nerveux central, telles que les maladies neurodégénératives telle que dans la formule (I) :
soit
X représente un groupe -OR, -NRR', $-S(O)_pR$, -OCOR, -OCOOR et
Y représente un groupe de formule :

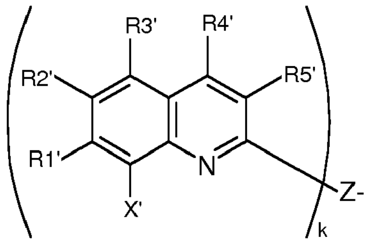

(IY)

dans laquelle X' représente un groupe -OR, -NRR', $-S(O)_pR$, -OCOR, -OCOOR et
Z représente un groupe de formule $-(A)_m-(Alk)_n-(A')_{m'}-(Alk')_{n'}-(A'')_{m''}-(Alk'')_{n''}$ où m, n, m', n', m", n" identiques ou différents représentent indépendamment 0 ou 1, étant entendu que au moins un des m, n, m', n', m", n" soit égal à 1,
A, A' et A" identiques ou différents représentent indépendamment un groupe choisi parmi -NR-, $-S(O)_p-$, -O-, -C(=O)-, ou un cycle de 4 à 11 chaînons choisi parmi les cycloalkyles, hétérocycles, aryles, hétéroaryles, ledit cycle étant éventuellement substitué par un ou plusieurs substituants choisis parmi Alkyle, OR, NRR', $CF_3$, Hal, CN, $S(O)_pR$, COOR, OCOOR, CONRR', NRCOOR' ; Alk, Alk' et Alk" identiques ou différents représentent indépendamment un groupe -alkyle- éventuellement substitué par un ou plusieurs substituants choisis parmi OR, NRR', $CF_3$, Hal, CN, $S(O)_pR$, COOR, OCOOR, CONRR', NRCOOR' ;
ou soit
X représente un groupe de formule :

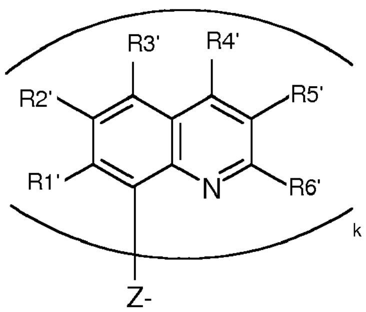

(IX)

dans laquelle Z représente un groupe de formule $-(A)_m-(Alk)_n-(A')_{m'}-(Alk')_{n'}-(A'')_{m''}-(Alk'')_{n''}$ où m, n, m', n', m", n" identiques

ou différents représentent indépendamment 0 ou 1, étant entendu que au moins un des m, n, m', n', m'', n'' soit égal à 1, A, A' et A'' identiques ou différents représentent indépendamment un groupe choisi parmi -NR-, -S(O)$_p$-, -O-, -C(=O)-, ou un cycle de 4 à 11 chaînons choisi parmi les cycloalkyles, hétérocycles, aryles, hétéroaryles, ledit cycle étant éventuellement substitué par un ou plusieurs substituants choisis parmi Alkyle, OR, NRR', $CF_3$, Hal, CN, S(O)$_p$R, COOR, OCOOR, CONRR', NRCOOR' ;
Alk, Alk' et Alk'' identiques ou différents représentent indépendamment un groupe -alkyle- éventuellement substitué par un ou plusieurs substituants choisis parmi OR, NRR', $CF_3$, Hal, CN, S(O)$_p$R, COOR, OCOOR, CONRR', NRCOOR' ;
Y représente un groupe choisi parmi H, OR, NRR', Hal, -CN, -CF3, alkyle éventuellement substitué par un ou plusieurs substituants choisis parmi OR, NRR', $CF_3$, Hal, CN, S(O)$_p$R, COOR, OCOOR, CONRR', NRCOOR' ;
et
R et R' identiques ou différents représentent indépendamment un atome d'hydrogène ou un groupe cycloalkyle ou alkyle éventuellement substitué par un ou plusieurs groupes choisis parmi OR, NRR', Hal, -CN, -$CF_3$, S(O)$_p$R, COOR, OCOOR, CONRR', NRCOOR' ; hétéroaryle ;
R1, R2, R3, R4, R5, R1', R2', R3', R4', R5', R6' identiques ou différents représente indépendamment un groupe ou atome choisi parmi H, OR, NRR', Hal, -CN, -$CF_3$, S(O)$_p$R, COOR, OCOOR, CONRR', NRCOOR', alkyle éventuellement substitué par un ou plusieurs substituants choisis parmi OR, NRR', $CF_3$, Hal, CN, S(O)$_p$R, COOR, OCOOR, CONRR', NRCOOR' ;
k représente 1 ou 2 ;
p représente 0, 1 ou 2 ;
ainsi que leurs stéréoisomères ou leur mélanges, leurs formes tautomères, leurs hydrates, solvates, leurs sels, formes libres et esters pharmaceutiquement acceptables,
à l'exception des composés pour lesquels :

X représente un groupe -OH, et
Y représente un groupe de formule :

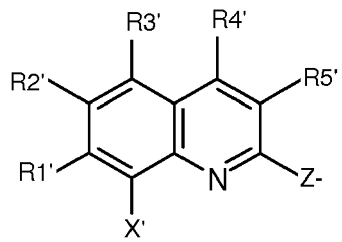

dans laquelle X' représente un groupe -OH,
Z représente un groupe choisi parmi -NBu-, -NMe-, et
R1, R2, R3, R4, R5, R1', R2', R3', R4', R5', R6' sont égaux à H et des composés pour lesquels
X représente un groupe de formule :

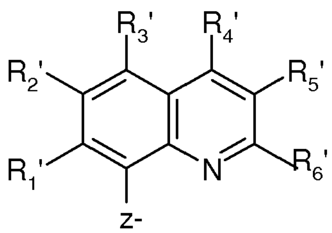

dans laquelle Z représente un groupe de formule -(NH)-$(CH_2)_2$-(NH)-$(CH_2)_2$-(NH)-,
Y représente H
R1, R2, R3, R4, R5, R1', R2', R3', R4', R5', R6' représentent H.

Plus préférentiellement, les composés pour la présente utilisation selon l'invention sont choisis parmi :

Diméthyl-bis[8-(acétyloxy)-2-quinoléinyl]propanedioate

2,2'-Méthanediyl-bis(8-hydroxyquinoléine)
2,2'-Méthanediyl-bis(5-chloro-8-hydroxyquinoléine)
2,2'-(2,2-Propanediyl)-bis(8-hydroxyquinoléine)
2,2'-(2,2-Propanediyl)-bis(5-chloro-8-hydroxyquinoléine)
2,2'-(Difluorométhanediyl)-bis(8-hydroxyquinoléine)
Bis(8-hydroxy-2-quinoléinyl)méthanone
2,2'-(1,2-Ethanediyl)-bis[8-(méthyloxy)quinoléine]
2,2'-(1,2-Ethanediyl)-bis(8-hydroxyquinoléine)
2,2'-(1,2-Ethanediyl)-bis(5-chloro-8-hydroxyquinoléine)
2,2'-(1,2-Ethanediyl)-bis(5-chloro-7-iodo-8-hydroxyquinoléine)
2,2'-(1,4-Butanediyl)-bis[8-(méthyloxy)quinoléine]
2,2'-(1,4-Butanediyl)-bis(8-hydroxyquinoléine)
2,2',2"-(1,2,3-Propanetriyl)-tris[8-(méthyloxy)quinoléine]
2,2',2"-(1,2,3-Propanetriyl)-tris(8-hydroxyquinoléine)
Bis(8-quinoléinyl)amine
*N,N'*-di-8-quinoléinyl-1,3-propanediamine
*N,N'*-bis(2-méthyl-8-quinoléinyl)-1,2-éthanediamine
8,8'-Oxydiquinoléine
Diméthyl bis{7-(méthyloxy)8-[phénylméthyl)oxy]-2-quinoléinyl}propanedioate
2,2'-(Méthanediyl)-bis(7-méthyloxy-8-hydroxy-2-quinoléinium) dichlorure
2,2'-(1,2-Ethanediyl)-bis[7-(méthyloxy)-8-quinoléinol]
8-Hydroxy-*N*-(8-hydroxy-2-quinoléinyl)-2-quinoléinecarboxamide
2-{[8-Hydroxy-2-quinoléinyl)amino]méthyl}-8-quinoléinol
2,2'-(Iminodiméthanediyl)di(*N*-boc-8-quinoléinamine)
2,2'-(Iminodiméthanediyl)di(8-quinoléinamine)
2,2',2"-(Nitrilotriméthanediyl)tri(*N*-boc-8-quinoléinamine)
2,2',2"-(Nitrilotriméthanediyl)tri(8-quinoléinamine)
2,2'-[(Butylimino)diméthanediyl]di(*N*-boc-8-quinoléinamine)
2,2'-[(Butylimino)diméthanediyl]di(8-quinoléinamine)
*N*-Butyl-2,2'-imino-bis(8-quinoléinamine)
*N*-8-Quinoléinyl-8-quinoléinecarboxamide
*N*-8-Quinoléinyl-8-quinoléinesulfonamide

ainsi que leurs stéréoisomères ou leur mélanges, leurs formes tautomères, leurs hydrates, solvates, leurs sels, formes libres et esters pharmaceutiquement acceptables.

L'invention concerne notamment une utilisation dans laquelle le composé est choisi parmi le Bis(8-quinoléinyl) amine et le 2,2'-(2,2-propanediyl)-bis-(8-hydroxyquinoléine), lesdits composés ne présentant pas d'effet mutagène ou d'effet bactéricide, en particulier selon le test de Ames adapté de D.M. Maron et B. N. Ames, Mutat. Res. 1983, 113, 173-215 ; D . E. Levin et al., Mutat. Res. 1982, 94, 315-330; D. E. Levin et al. Proc. Natl. USA 1982, 79, 7445-7449, notamment sur des souches de TA98 et/ou TA100 de salmonelle en absence ou en présence d'homogénat enzymatique (S9) de microsome de foie de rat.

Selon un autre objet, la présente demande concerne les compositions pharmaceutiques comprenant un composé de formule (I)

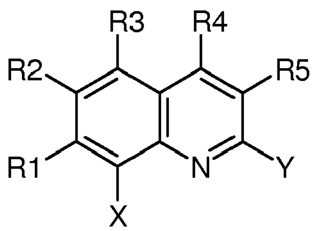

(I)

tels que dans la formule (I)

X représente un groupe -OR, -NRR', $-S(O)_pR$, -OCOOR et

Y représente un groupe de formule :

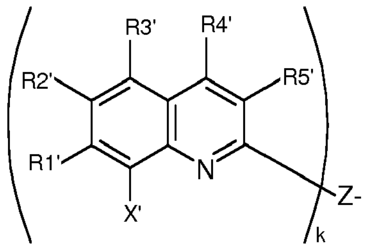

(IY)

dans laquelle X' représente un groupe -OR, -NRR', $-S(O)_pR$, -OCOOR et Z représente un groupe de formule $-(A)_m-(Alk)_n-(A')_{m'}-(Alk')_{n'}-(A'')_{m''}-(Alk'')_{n''}$ où

m, n, m', n', m'', n'' identiques ou différents représentent indépendamment 0 ou 1, étant entendu que au moins un des m, n, m', n', m'', n'' soit égal à 1,

A, A' et A'' identiques ou différents représentent indépendamment un groupe choisi parmi -NR-, $-S(O)_p-$, -O-, -C(=O)-, ou un cycle de 4 à 11 chaînons choisi parmi les cycloalkyles, hétérocycles, aryles, hétéroaryles, ledit cycle étant éventuellement substitué par un ou plusieurs substituants choisis parmi Alkyle, OR, NRR', $CF_3$, Hal, CN, $S(O)_pR$, COOR, OCOOR, CONRR', NRCOOR' ;

Alk, Alk'et Alk'' identiques ou différents représentent indépendamment un groupe -alkyle- éventuellement substitué par un ou plusieurs substituants choisis parmi OR, NRR', $CF_3$, Hal, CN, $S(O)_pR$, COOR, OCOOR, CONRR', NRCOOR' ; et

R et R' identiques ou différents représentent indépendamment un atome d'hydrogène ou un groupe cycloalkyle ou alkyle éventuellement substitué par un ou plusieurs groupes choisis parmi OR, NRR', Hal, -CN, $-CF_3$, $S(O)_pR$, COOR, OCOOR, CONRR', NRCOOR'; hétéroaryle;

R1, R2, R3, R4, R5, R1', R2', R3', R4', R5', R6' identiques ou différents représente indépendamment un groupe ou atome choisi parmi H, OR, NRR', Hal, -CN, $-CF_3$, alkyle éventuellement substitué par un ou plusieurs substituants choisis parmi OR, NRR', $CF_3$, Hal, CN, $S(O)_pR$, COOR, OCOOR, CONRR', NRCOOR' ;

k représente 1 ou 2 ;

p représente 0, 1 ou 2 ;

ainsi que leurs stéréoisomères ou leur mélanges, leurs formes tautomères, leurs hydrates, solvates, leurs sels, formes libres et esters pharmaceutiquement acceptables,

à l'exception des composés pour lesquels :

X représente un groupe -OH, et

Y représente un groupe de formule :

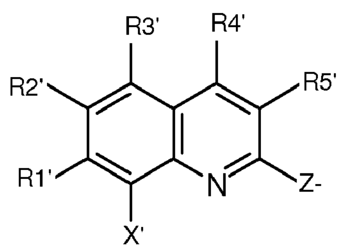

dans laquelle X' représente un groupe -OH, et

Z représente un groupe choisi parmi -NH-, -NBu-, -NMe-,

R1, R2, R3, R4, R5, R1', R2', R3', R4', R5', R6' sont égaux à H.

Plus préférentiellement, les composés pour les compositions pharmaceutiques selon l'invention sont choisis parmi :

Diméthyl-bis[8-(acétyloxy)-2-quinoléinyl]propanedioate
2,2'-Méthanediyl-bis(8-hydroxyquinoléine)
2,2'-Méthanediyl-bis(5-chloro-8-hydroxyquinoléine)
2,2'-(2,2-Propanediyl)-bis(8-hydroxyquinoléine)
2,2'-(2,2-Propanediyl)-bis(5-chloro-8-hydroxyquinoléine)
2,2'-(Difluorométhanediyl)-bis(8-hydroxyquinoléine)
Bis(8-hydroxy-2-quinoléinyl)méthanone
2,2'-(1,2-Ethanediyl)-bis[8-(méthyloxy)quinoléine]
2,2'-(1,2-Ethanediyl)-bis(8-hydroxyquinoléine)
2,2'-(1,2-Ethanediyl)-bis(5-chloro-8-hydroxyquinoléine)
2,2'-(1,2-Ethanediyl)-bis(5-chloro-7-iodo-8-hydroxyquinoléine)
2,2'-(1,4-Butanediyl)-bis[8-(méthyloxy)quinoléine]
2,2'-(1,4-Butanediyl)-bis(8-hydroxyquinoléine)
2,2',2"-(1,2,3-Propanetriyl)-tris[8-(méthyloxy)quinoléine]
2,2',2"-(1,2,3-Propanetriyl)-tris(8-hydroxyquinoléine)
Diméthyl bis{7-(méthyloxy)8-[phénylméthyl)oxy]-2-quinoléinyl}propanedioate
2,2'-(Méthanediyl)-bis(7-méthyloxy-8-hydroxyquinoléinium) dichlorure
2,2'-(1,2-Ethanediyl)-bis[7-(méthyloxy)-8-quinoléinol]
8-Hydroxy-*N*-(8-hydroxy-2-quinoléinyl)-2-quinoléinecarboxamide
2-{[8-Hydroxy-2-quinoléinyl)amino]méthyl}-8-quinoléinol
2,2'-(Iminodiméthanediyl)di(*N*-boc-8-quinoléinamine)
2,2'-(Iminodiméthanediyl)di(8-quinoléinamine)
2,2',2"-(Nitrilotriméthanediyl)tri(*N*-boc-8-quinoléinamine)
2,2',2"-(Nitrilotriméthanediyl)tri(8-quinoléinamine)
2,2'-[(Butylimino)diméthanediyl]di(*N*-boc-8-quinoléinamine)
2,2'-[(Butylimino)diméthanediyl]di(8-quinoléinamine)
*N*-Butyl-2,2'-imino-bis(8-quinoléinamine)

ainsi que leurs stéréoisomères ou leur mélanges, leurs formes tautomères, leurs hydrates, solvates, leurs sels, formes libres et esters pharmaceutiquement acceptables.

Selon un autre objet, la présente invention concerne les composés de formule (I) :

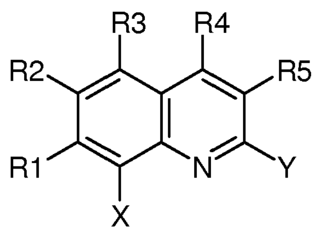

(I)

tels que dans la formule (I)
X représente un groupe -OR, -NRR', $-S(O)_pR$, -OCOOR,$-NO_2$ et
Y représente un groupe de formule :

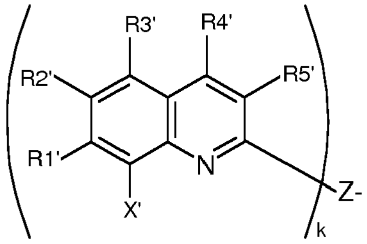

(IY)

dans laquelle X' représente un groupe -OR, -NRR', $-S(O)_pR$, -OCOOR, $-NO_2$ et Z représente un groupe de formule $-(A)_m-(Alk)_n-(A')_{m'}-(Alk')_{n'}-(A'')_{m''}-(Alk'')_{n''}$ où
m, n, m', n', m", n" identiques ou différents représentent indépendamment 0 ou 1, étant entendu que au moins un des m, n, m', n', m", n" soit égal à 1,
A, A' et A" identiques ou différents représentent indépendamment un groupe choisi parmi -NR-, $-S(O)_p-$, -O-, -C(=O)-, ou un cycle de 4 à 11 chaînons choisi parmi les cycloalkyles, hétérocycles, aryles, hétéroaryles, ledit cycle étant éventuellement substitué par un ou plusieurs substituants choisis parmi Alkyle, OR, NRR', $CF_3$, Hal, CN, $S(O)_pR$, COOR, OCOOR, CONRR', NRCOOR' ;
Alk, Alk' et Alk" identiques ou différents représentent indépendamment un groupe -alkyle- éventuellement substitué par un ou plusieurs substituants choisis parmi OR, NRR', $CF_3$, Hal, CN, $S(O)_pR$, COOR, OCOOR, CONRR', NRCOOR' ; et
R et R' identiques ou différents représentent indépendamment un atome d'hydrogène ou un groupe cycloalkyle ou alkyle éventuellement substitué par un ou plusieurs groupes choisis parmi OR, NRR', Hal, -CN, $-CF_3$, $S(O)_pR$, COOR, OCOOR, CONRR', NRCOOR'; hétéroaryle ;
R1, R2, R3, R4, R5, R1', R2', R3', R4', R5', R6' identiques ou différents représente indépendamment un groupe ou atome choisi parmi H, OR, NRR', Hal, -CN, $-CF_3$, alkyle éventuellement substitué par un ou plusieurs substituants choisis parmi OR, NRR', $CF_3$, Hal, CN, $S(O)_pR$, COOR, OCOOR, CONRR', NRCOOR' ;
k représente 1 ou 2 ;
p représente 0, 1 ou 2 ;
ainsi que leurs stéréoisomères ou leur mélanges, leurs formes tautomères, leurs hydrates, solvates, leurs sels, formes libres et esters pharmaceutiquement acceptables,
à l'exception des composés pour lesquels :

X représente un groupe -OH, et
Y représente un groupe de formule :

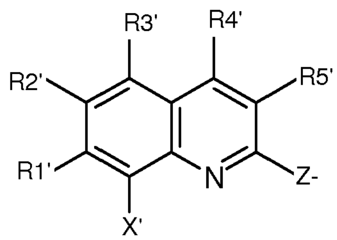

dans laquelle X' représente un groupe -OH, et
Z représente un groupe choisi parmi $-CH_2-$, $-CH_2-CH_2-$, $-(CH_2)_6-$, -CH=CH-, $-C(Me)_2-$, -thienyl-, $-CH_2-S-CH_2-$, -NH-, -NBu-, -NMe-, R1, R2, R3, R4, R5, R1', R2', R3', R4', R5', R6' sont égaux à H.

Plus préférentiellement, les composés selon la présente invention sont choisis parmi :

2,2'-Méthanediyl-bis(5-chloro-8-hydroxyquinoléine)
2,2'-(2,2-Propanediyl)-bis(5-chloro-8-hydroxyquinoléine)
2,2'-(Difluorométhanediyl)-bis(8-hydroxyquinoléine)
Bis(8-hydroxy-2-quinoléinyl)méthanone

2,2'-(1,2-Ethanediyl)-bis(5-chloro-8-hydroxyquinoléine)
2,2'-(1,2-Ethanediyl)-bis(5-chloro-7-iodo-8-hydroxyquinoléine)
2,2'-(1,4-Butanediyl)-bis[8-(méthyloxy)quinoléine]
2,2'-(1,4-Butanediyl)-bis(8-hydroxyquinoléine)
2,2',2"-(1,2,3-Propanetriyl)-tris[8-(méthyloxy)quinoléine]
2,2',2"-(1,2,3-Propanetriyl)-tris(8-hydroxyquinoléine)
Diméthyl bis{7-(méthyloxy)8-[phénylméthyl)oxy]-2-quinoléinyl}propanedioate
2,2'-(Méthanediyl)-bis(7-méthyloxy-8-hydroxy-2-quinoléinium) dichlorure
2,2'-(1,2-Ethanediyl)-bis[7-(méthyloxy)-8-quinoléinol]
8-Hydroxy-*N*-(8-hydroxy-2-quinoléinyl)-2-quinoléinecarboxamide
2-{[8-Hydroxy-2-quinoléinyl)amino]méthyl}-8-quinoléinol
2,2'-(Iminodiméthanediyl)di(*N*-boc-8-quinoléinamine)
2,2'-(Iminodiméthanediyl)di(8-quinoléinamine)
2,2',2"-(Nitrilotriméthanediyl)tri(*N*-boc-8-quinoléinamine)
2,2',2"-(Nitrilotriméthanediyl)tri(8-quinoléinamine)
2,2'-[(Butylimino)diméthanediyl]di(*N*-boc-8-quinoléinamine)
2,2'-[(Butylimino)diméthanediyl]di(8-quinoléinamine)
*N*-Butyl-2,2'-imino-bis(8-nitroquinoléine)
*N*-Butyl-2,2'-imino-bis(8-quinoléinamine)

ainsi que leurs stéréoisomères ou leur mélanges, leurs formes tautomères, leurs hydrates, solvates, leurs sels, formes libres et esters pharmaceutiquement acceptables.

De préférence, X représente OR et Y représente un groupe de formule (IY) dans laquelle X' représente OR et Z représente A où A représente -C(O)- ou Z représente Alk où Alk représente un groupe alkyle linéaire ou ramifié éventuellement substitué par un ou plusieurs atomes d'halogène ; ou
X représente OR et Y représente un groupe de la formule (IY) dans laquelle X' représente OR et Z représente A-A' où A représente -C(O)- et A' représente NR'; ou
X représente OR et Y représente un groupe de la formule (IY) dans laquelle X' représente OR et Z représente A-Alk où A représente NR' et Alk représente un groupe alkyle linéaire ou ramifié éventuellement substitué par un ou plusieurs atomes d'halogène ; ou
X représente NRR' et Y représente un groupe de la formule (IY) dans laquelle X' représente NRR' et Z représente Alk-NR"-Alk où Alk représente un groupe alkyle linéaire ou ramifié éventuellement substitué par un ou plusieurs atomes d'halogène ; ou
X représente NRR' et Y représente un groupe de la formule (IY) dans laquelle X' représente NRR' et Z représente NR" ; ou
X représente un groupe de formule (IX) dans laquelle Z représente -NR-, -NR-Alk-NR-, -O- , -NR-C(O)- ou -NR-S(O)$_2$- et Y représente H ;
k=1 ou 2
R=H ou COOR' ou alkyle éventuellement substitué, R'= H ou alkyl éventuellement substitué, R"= H, alkyle ou hétéroaryle éventuellement substitués, et
R1, R2, R3, R4, R5, R1', R2', R3', R4', R5', R6' identiques ou différents représente indépendamment un groupe ou atome choisi parmi H, OR, NRR', Hal, -CN, -$CF_3$, alkyle éventuellement substitué par un ou plusieurs substituants choisis parmi OR, NRR', $CF_3$, Hal, CN, $S(O)_pR$, COOR, OCOOR, CONRR', NRCOOR'
ainsi que leurs stéréoisomères ou leur mélanges, leurs formes tautomères, leurs hydrates, solvates, leurs sels, formes libres et esters pharmaceutiquement acceptables.

Ci-avant et ci-après, il est entendu dans la définition de m, m', m", n, n' et n" que si m=m'=1 ou m'=m"=1 ou m=m'=m"=1 ou m=m"=1 et m'=0, alors respectivement n=1 ou n'=1 ou n=n'=1 ou au moins des n ou n'=1.

Selon la présente invention, les radicaux Alkyle représentent des radicaux hydrocarbonés saturés, en chaîne droite ou ramifiée, de 1 à 20 atomes de carbone, de préférence de 1 à 5 atomes de carbone.

On peut notamment citer, lorsqu'ils sont linéaires, les radicaux méthyle, éthyle, propyle, butyle, pentyle, hexyle, octyle, nonyle, décyle, dodécyle, hexadécyle, et octadécyle.

On peut notamment citer, lorsqu'ils sont ramifiés ou substitués par un ou plusieurs radicaux alkyle, les radicaux isopropyle, tert-butyl, 2-éthylhexyle, 2-méthylbutyle, 2-méthylpentyle, 1-méthylpentyle et 3-méthylheptyle.

Les radicaux Alkoxy selon la présente invention sont des radicaux de formule -O-Alkyle, l'alkyle étant tel que défini précédemment.

Parmi les atomes d'Halogène, on cite plus particulièrement les atomes de fluor, de chlore, de brome et d'iode.

Les radicaux Alkényle représentent des radicaux hydrocarbonés, en chaîne droite ou linéaire, et comprennent une ou plusieurs insaturations éthyléniques. Parmi les radicaux Alkényle, on peut notamment citer les radicaux allyle ou vinyle.

Les radicaux Alkynyle représentent des radicaux hydrocarbonés, en chaîne droite ou linéaire, et comprennent une ou plusieurs insaturations acétyléniques. Parmi les radicaux Alkynyle, on peut notamment citer l'acétylène.

Le radical Cycloalkyle est un radical hydrocarboné mono-, bi- ou tricyclique saturé ou partiellement insaturé, non aromatique, de 3 à 11 atomes de carbone, tel que notamment le cyclopropyle, cyclopentyle, cyclohexyle ou adamantyle, ainsi que les cycles correspondants contenant une ou plusieurs insaturations.

Aryle désigne un système aromatique hydrocarboné, mono ou bicyclique de 4 à 11 atomes de carbone.

Parmi les radicaux Aryle, on peut notamment citer le radical phényle ou naphtyle, plus particulièrement substitué par au moins un atome d'halogène.

Parmi les radicaux -AlkylAryle, on peut notamment citer le radical benzyle ou phénéthyle.

Les radicaux Hétéroaryles désignent les systèmes aromatiques comprenant un ou plusieurs hétéroatomes choisis parmi l'azote, l'oxygène ou le soufre, mono ou bicyclique, de 4 à 11 atomes de carbone. Parmi les radicaux Hétéroaryles, on pourra citer le pyrazinyle, le thiényle, l'oxazolyle, le furazanyle, le pyrrolyle, le 1,2,4-thiadiazolyle, le naphthyridinyle, le pyridazinyle, le quinoxalinyle, le phtalazinyle, l'imidazo[1,2-a]pyridine, l'imidazo[2,1-b]thiazolyle, le cinnolinyle, le triazinyle, le benzofurazanyle, l'azaindolyle, le benzimidazolyle, le benzothiényle, le thiénopyridyle, le thiénopyrimidinyle, le pyrrolopyridyle, l'imidazopyridyle, le benzoazaindole, le 1,2,4-triazinyle, le benzothiazolyle, le furanyle, l'imidazolyle, l'indolyle, le triazolyle, le tétrazolyle, l'indolizinyle, l'isoxazolyle, l'isoquinoléinyle, l'isothiazolyle, l'oxadiazolyle, le pyrazinyle, le pyridazinyle, le pyrazolyle, le pyridyle, le pyrimidinyle, le purinyle, le quinazolinyle, le quinoléinyle, l'isoquinoléyle, le 1,3,4-thiadiazolyle, le thiazolyle, le triazinyle, l'isothiazolyle, le carbazolyle, ainsi que les groupes correspondants issus de leur fusion ou de la fusion avec le noyau phényle.

L'expression « sels pharmaceutiquement acceptables » fait référence aux sels d'addition acide relativement non toxiques, inorganiques et organiques, et les sels d'addition de base, des composés de la présente invention. Ces sels peuvent être préparés *in situ* pendant l'isolement final et la purification des composés. En particulier, les sels d'addition acide peuvent être préparés en faisant réagir séparément le composé purifié sous sa forme épurée avec un acide organique ou inorganique et en isolant le sel ainsi formé. Parmi les exemples de sels d'addition acide on trouve les sels bromhydrate, chlorhydrate, sulfate, bisulfate, phosphate, nitrate, acétate, oxalate, valérate, oléate, palmitate, stéarate, laurate, borate, benzoate, lactate, phosphate, tosylate, citrate, maléate, fumarate, succinate, tartrate, naphthylate, mésylate, glucoheptanate, lactobionate, sulfamates, malonates, salicylates, propionates, méthylènebis-betahydroxynaphtoates, acide gentisique, iséthionates, di-p-toluoyltartrates, méthanesulfonates, éthanesulfonates, benzènesulfonates, p-toluènesulfonates, cyclohexyl sulfamates et quinateslaurylsulfonate, et analogues. (Voir par exemple S.M. Berge et al. Pharmaceutical Salts, J. Pharm. Sci, 66 : p.1-19 (1977) qui est incorporé ici en référence). Les sels d'addition acide peuvent également être préparés en faisant réagir séparément le composé purifié sous sa forme acide avec une base organique ou inorganique et en isolant le sel ainsi formé. Les sels d'addition acide comprennent les sels aminés et métalliques. Les sels métalliques adaptés comprennent les sels de sodium, potassium, calcium, baryum, zinc, magnésium et aluminium. Les sels de sodium et de potassium sont préférés. Les sels d'addition inorganiques de base adaptés sont préparés à partir de bases métalliques qui comprennent hydrure de sodium, hydroxyde de sodium, hydroxyde de potassium, hydroxyde de calcium, hydroxyde d'aluminium, hydroxyde de lithium, hydroxyde de magnésium, hydroxyde de zinc. Les sels d'addition aminés de base adaptés sont préparés à partir d'amines qui ont une alcalinité suffisante pour former un sel stable, et de préférence comprennent les amines qui sont souvent utilisées en chimie médicinale en raison de leur faible toxicité et de leur acceptabilité pour l'usage médical : ammoniac, éthylènediamine, N-méthyl-glucamine, lysine, arginine, ornithine, choline, *N,N'*-dibenzyl-éthylenediamine, chloroprocaïne, diéthanolamine, procaïne, *N*-benzyl-phénéthylamine, diéthylamine, pipérazine, tris(hydroxyméthyl)-aminométhane, hydroxyde de tétraméthylammonium, triéthylamine, dibenzylamine, éphénamine, déhydroabiéthylamine, *N*-éthyl-pipéridine, benzylamine, tétraméthylammonium, tétraéthylammonium, méthylamine, diméthylamine, triméthylamine, éthylamine, acides aminés basiques, par exemple lysine et arginine, et dicyclohexylamine, et analogues.

L'invention se rapporte également aux stéréoisomères ou leurs mélanges, les formes tautomères, les hydrates, solvates, sels et esters pharmaceutiquement acceptables des composés de formule (I).

Les composés de l'invention de formule (I) définis tels que précédemment, possédant une fonction suffisamment acide ou une fonction suffisamment basique ou les deux, peuvent inclure les sels correspondants d'acide organique ou minéral ou de base organique ou minérale pharmaceutiquement acceptables.

Selon un autre objet, la présente invention concerne également le procédé de préparation des composés de formule (I).

Dans les modes de réalisation et les exemples qui vont suivre, le groupement R6 représente le groupement Y.

Selon un premier aspect, les composés de formule (I) sont préparés par couplage des composés de formule (II) et (II') :

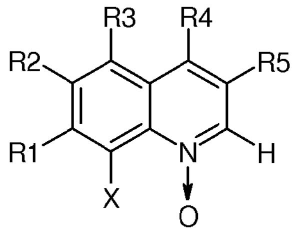

(II)

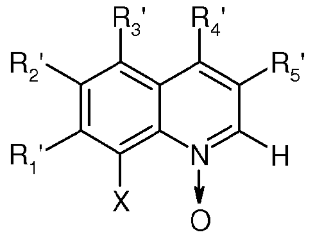

(II')

suivi d'une hydrolyse, et, éventuellement suivi de dérivatisation(s) du produit de formule (I) obtenu pour obtenir le produit de formule (I) souhaité.

Généralement, cette réaction est effectuée au moyen de diméthyle-malonate, en présence d'anhydride acétique. Ce procédé est illustré de façon représentative par le schéma 1.

La réaction d'hydrolyse est généralement effectuée au moyen d'un acide, tel que, par exemple l'acide chlorhydrique, ou tout autre acide convenable.

Selon un second aspect, les composés de formule (I) sont préparés par couplage des composés de formule (III) et (III') :

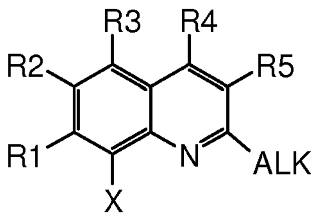

(III)

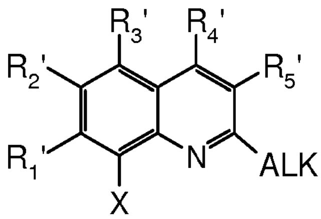

(III')

éventuellement suivi de dérivatisation(s) du produit de formule (I) obtenu pour obtenir le produit de formule (I) souhaité.

Généralement, cette réaction est effectuée au moyen d'une base telle que le lithium diisopropylamine (LDA) suivi par l'addition d'un composé de formule Hal-Z'-Hal dans laquelle Z' est choisi de façon à obtenir le groupe Z correspondant dans la formule générale (I).

Ce procédé est illustré de façon représentative par le schéma 2.

De préférence, lorsque k=2, on préfère opérer à partir de (III) et (III'), au moyen d'une base telle que le lithium diisopropylamine (LDA) suivi par l'addition de $CuCl_2$ et traitement par l'EDTA. Ce procédé est illustré de façon représentative par le schéma 5.

Selon un troisième aspect, les composés de formule (I) sont préparés par couplage des composés de formule (IV) et (IV') :

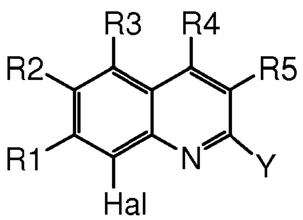

(IV)

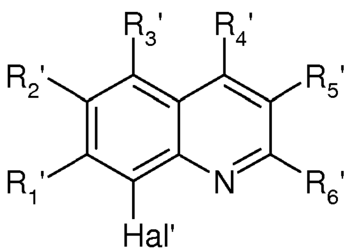

(IV')

dans lesquelles Hal et Hal' représentent des atomes d'halogène en présence d'un groupe de formule HZH, éventuellement suivi de dérivatisation(s) du produit de formule (I) obtenu pour obtenir le produit de formule (I) souhaité.

Généralement, cette réaction est effectuée en présence d'un catalyseur tel que tris(dibenzylidèneacétone) dipalladium(0) et rac-2,2'-bis(diphénylphosphino)-1,1',binaphthyl.

Ce procédé est illustré de façon représentative par le schéma 6.

Selon un quatrième aspect, les composés de formule (I) sont préparés par couplage des composés de formule (V) et (V') :

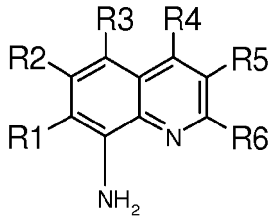

(V)

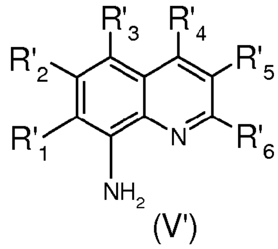

(V')

en présence d'un groupe de formule Hal-Z-Hal où Hal représente un atome d'halogène, éventuellement suivi de dérivatisation(s) du produit de formule (I) obtenu pour obtenir le produit de formule (I) souhaité.

Généralement, cette réaction est effectuée au moyen d'une base telle que le lithium diisopropylamine (LDA) suivi par l'addition d'un composé de formule Hal-Z-Hal dans laquelle Z est choisi de façon à obtenir le groupe Z correspondant dans la formule générale (I).

Ce procédé est illustré de façon représentative par le schéma 7.

Selon un cinquième aspect, les composés de formule (I) sont préparés par couplage des composés de formule (VI) et (VI') :

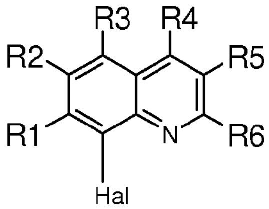

(VI)

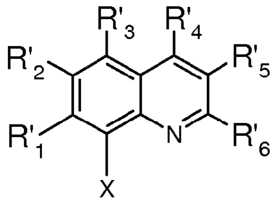

(VI')

dans lesquelles Hal est un atome d'halogène en présence de $CuCl_2$ et d'une base telle que $Cs_2CO_3$, éventuellement suivi de dérivatisation(s) du produit de formule (I) obtenu pour obtenir le produit de formule (I) souhaité.

Ce procédé est illustré de façon représentative par le schéma 8.

Selon un sixième aspect, les composés de formule (I) sont préparés par couplage des composés de formule (VII) et (VII') :

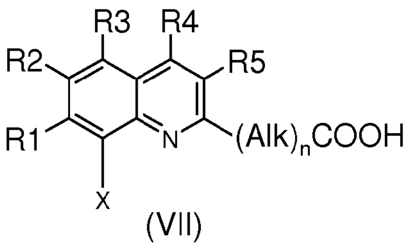

(VII)

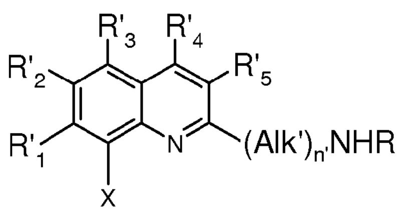

(VII')

éventuellement suivi de dérivatisation(s) du produit de formule (I) obtenu pour obtenir le produit de formule (I) souhaité.

Généralement, cette réaction est effectuée en présence d'agent d'activation des acides carboxyliques tels que le (benzotriazole-1-yloxy)tris-(diméthylamino)phosphonium hexafluorophosphate associé au hydroxybenzotriazole en présence d'une base telle que la triéthylamine.

Ce procédé est illustré de façon représentative par le schéma 11.

Selon un septième aspect, les composés de formule (I) sont préparés par couplage des composés de formule (VIII) et (VIII') :

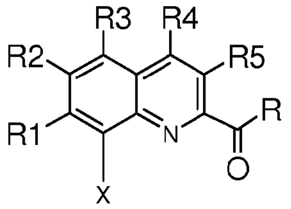

(VIII)

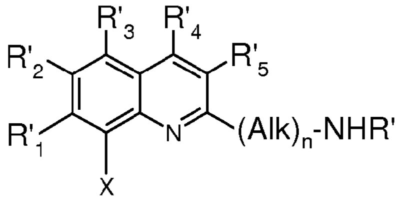

(VIII')

éventuellement suivi de dérivatisation(s) du produit de formule (I) obtenu pour obtenir le produit de formule (I) souhaité.

Généralement, cette réaction est effectuée en présence d'un agent de réduction des imines tel que $NaBHR_3$.

Ce procédé est illustré de façon représentative par le schéma 12.

Selon un huitième aspect, les composés de formule (I) sont préparés par couplage des composés de formule (VIIII) et (VIIII') :

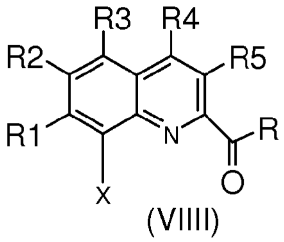

(VIIII)

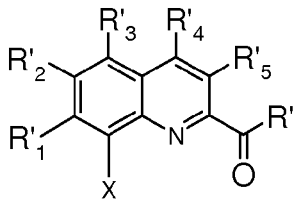

(VIIII')

en présence d'un groupe de formule $R''NH_2$, éventuellement suivi de dérivatisation(s) du produit de formule (I) obtenu pour obtenir le produit de formule (I) souhaité.

Généralement, cette réaction est effectuée au moyen d'un agent de réduction des imines tel que $NaBHR_3$.

Ce procédé est illustré de façon représentative par le schéma 13.

Selon un neuvième aspect, les composés de formule (I) sont préparés par couplage du composé de formule (VV) et deux composés de formule (VV'):

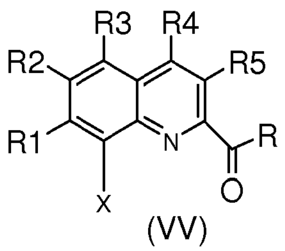

(VV)

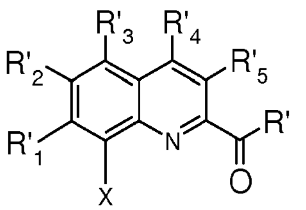

(VV')

en présence de $NH_3$, éventuellement suivi de dérivatisation(s)du produit de formule (I) obtenu pour obtenir le produit de formule (I) souhaité.

Généralement, cette réaction est effectuée au moyen d'un agent de réduction des imines tel que $NaBHR_3$.

Ce procédé est illustré de façon représentative par le schéma 14.

Selon un dixième aspect, les composés de formule (I) sont préparés par couplage des composés de formule (VVI) et (VVI') :

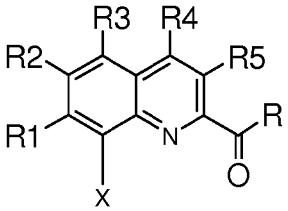

(VVI)

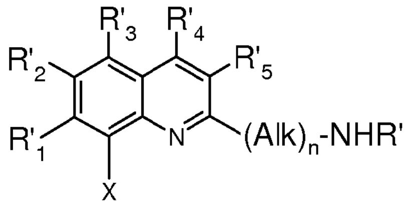

(VVI')

éventuellement suivi de dérivatisation(s) du produit de formule (I) obtenu pour obtenir le produit de formule (I) souhaité.

Généralement, cette réaction est effectuée en présence d'un agent de réduction des imines tel que $NaBHR_3$.

Ce procédé est illustré de façon représentative par le schéma 15.

Selon un onzième aspect, les composés de formule (I) sont préparés par couplage des composés de formule (VVII) et (VVII') :

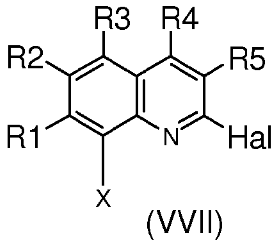

(VVII)

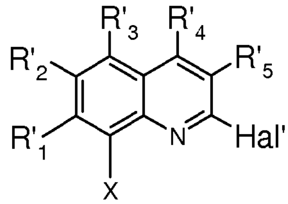

(VVII')

dans lesquelles Hal et Hal' sont des atomes d'halogène en présence d'un groupe de formule $RNH_2$, éventuellement suivi de dérivatisation(s) du produit de formule (I) obtenu pour obtenir le produit de formule (I) souhaité.

Généralement, cette réaction est effectuée par réaction de (VVII) avec $RNH_2$ suivi du couplage avec (VVII') en présence d'un catalyseur tel que letris(dibenzylidèneacétone)dipalladium(0) et le rac-2,2'-bis(diphénylphosphino)-1,1',binaphthyl.

Ce procédé est illustré de façon représentative par le schéma 16.

Selon un douzième aspect, les composés de formule (I) sont préparés par couplage des composés de formule (VVIII) et (VVIII') :

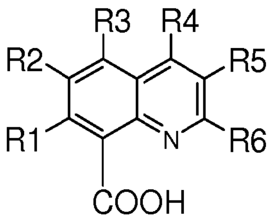

(VVIII)

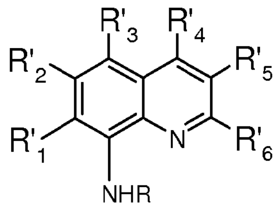

(VVIII')

éventuellement suivi de dérivatisation(s)du produit de formule (I) obtenu pour obtenir le produit de formule (I) souhaité.

Généralement, cette réaction est effectuée en présence d'agent d'activation des acides carboxyliques tel que le (benzotriazole-1-yloxy)-tris(diméthylamino)phosphonium hexafluorophosphate associé au hydroxybenzotriazole en présence d'une base telle que la triéthylamine.

Ce procédé est illustré de façon représentative par le schéma 17.

Par « dérivatisation », on entend toute réaction permettant de modifier ou introduire des groupes sur les molécules de départ. Il peut s'agir d'halogénation, d'alkylation, d'alkoxylation, d'addition, de substitution, de protection, déprotection, de réduction, etc. Ces réactions sont généralement connues en soi. Dans le procédé selon l'invention,

ces réactions peuvent être effectuées par application ou adaptation de ces méthodes.

Des exemples représentatifs de ces réactions de dérivatisation sont illustrés par les schémas 1, 3, 4, 9, 10, 13, 14, 15 et 16.

Ainsi, les composés de formule générale (I) peuvent être préparés par application ou adaptation de toute méthode connue en soi de et/ou à la portée de l'homme du métier, notamment celles décrites par Larock dans Comprehensive Organic Transformations, VCH Pub., 1989, ou par application ou adaptation des procédés décrits dans les exemples qui suivent.

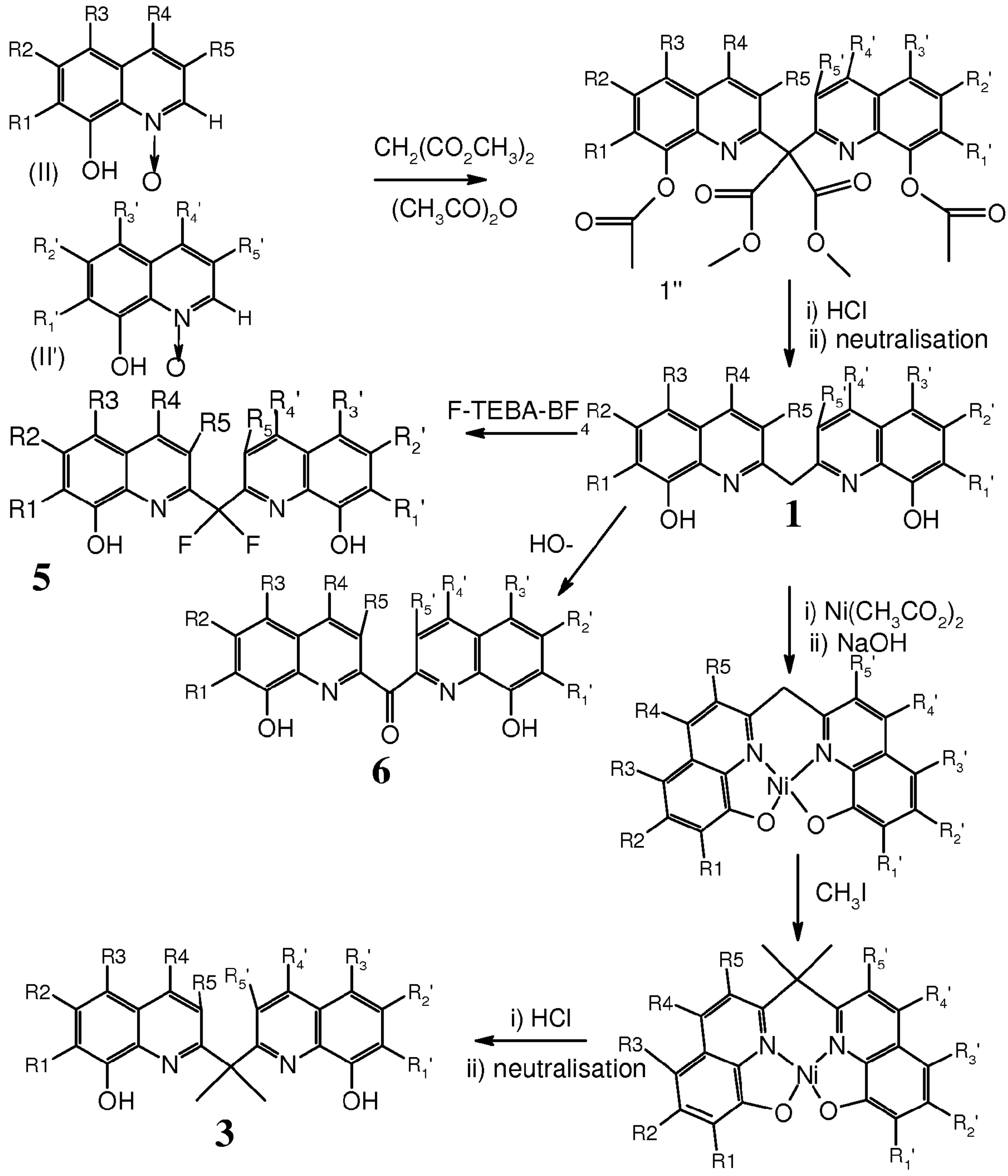


Schéma 1 : Synthèse de 1, 1" et de leurs dérivés : **3, 5, 6**, et analogues

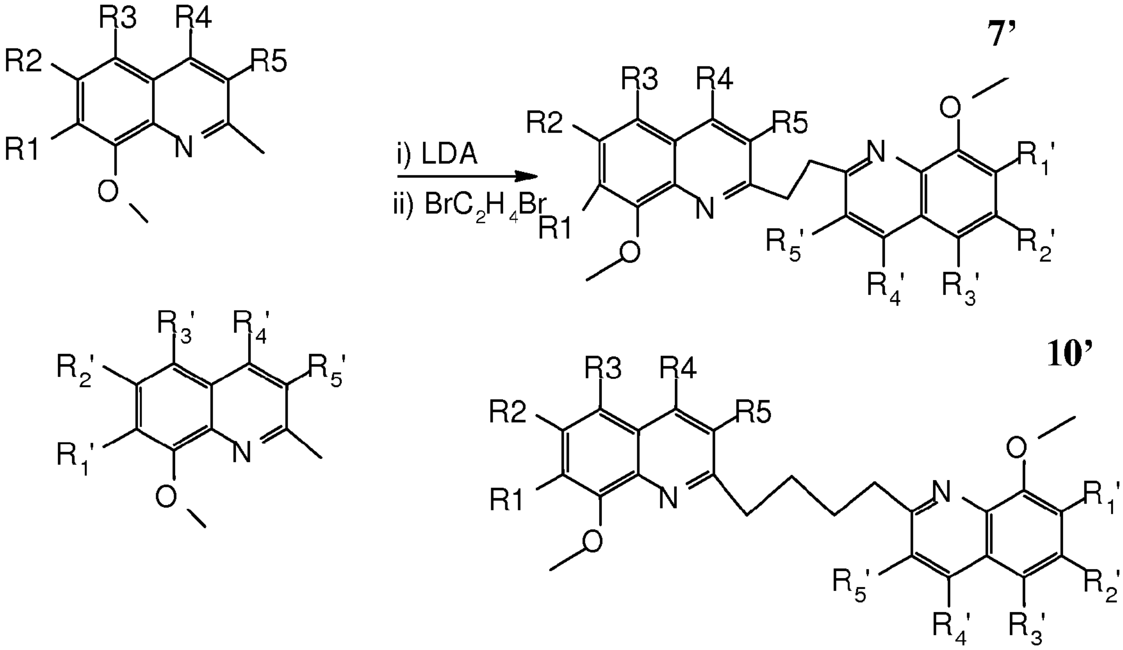


7'

10'

Schéma 2 : Synthèse de 2,2'-(1,2-éthanediyl)-bis[8-(méthyloxy)quinoléine] et de 2,2'-(1,4-butanediyl)-bis[8-(méthyloxy) quinoléine] **(7'), (10')** et leurs analogues

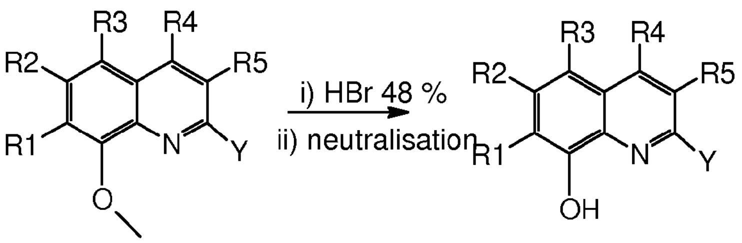


**7 10 11**

Schéma 3 : Hydrolyse des groupements protecteurs méthyloxy pour la synthèse de **7, 10, 11** et leurs analogues

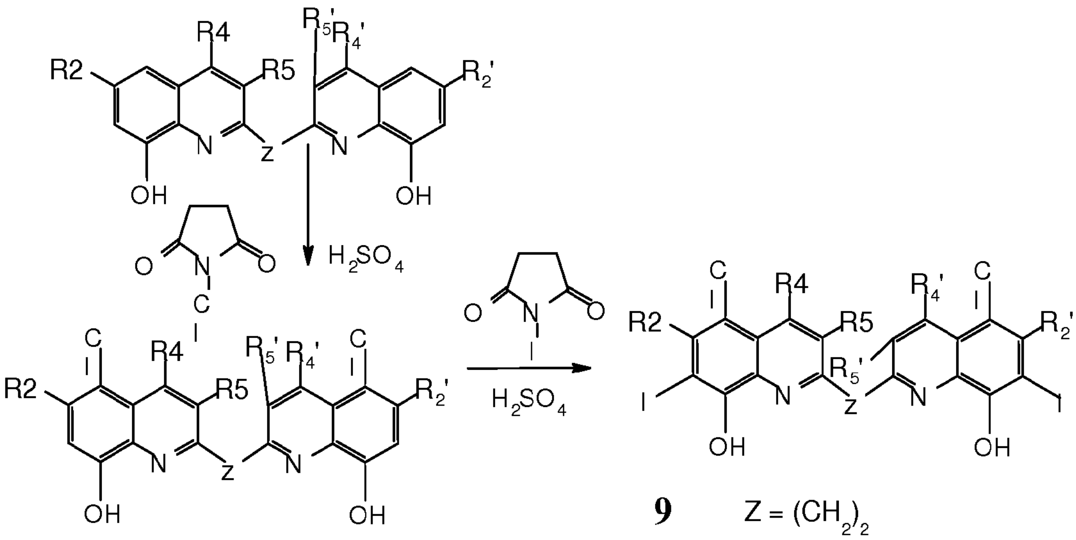


**9** Z = $(CH_2)_2$

**2** Z = $CH_2$

**4** Z = $(CH_3)_2C$

**8** Z = $(CH_2)_2$

Schéma 4 : Réaction d'halogénation pour la préparation de **2, 4, 8, 9** et leurs analogues

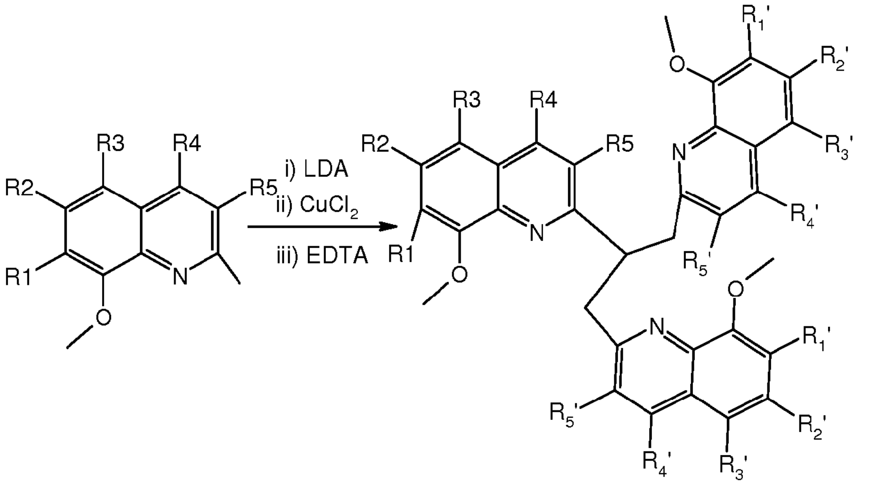


**11'**

Schéma 5 : Synthèse de 2,2',2"-(1,2,3-propanetriyl)-tris[8-(méthyloxy)quinoléine] **(11')** et leurs analogues.

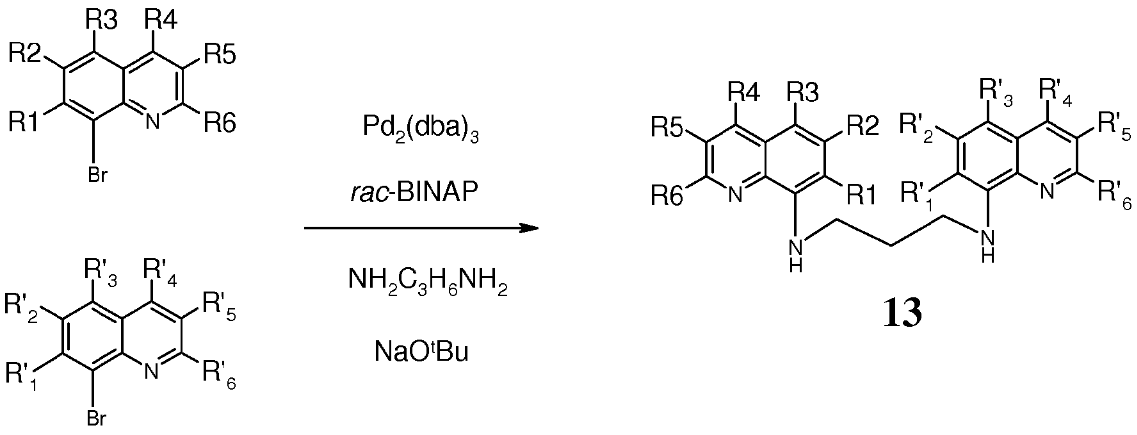


13

Schéma 6 : Synthèse de **13** et ses analogues.

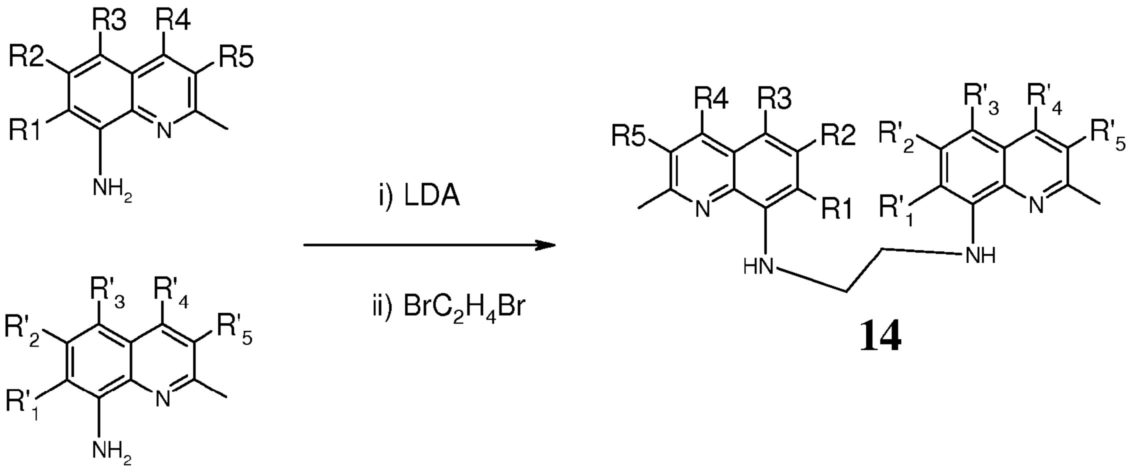


14

Schéma 7 : Synthèse de **14** et ses analogues.

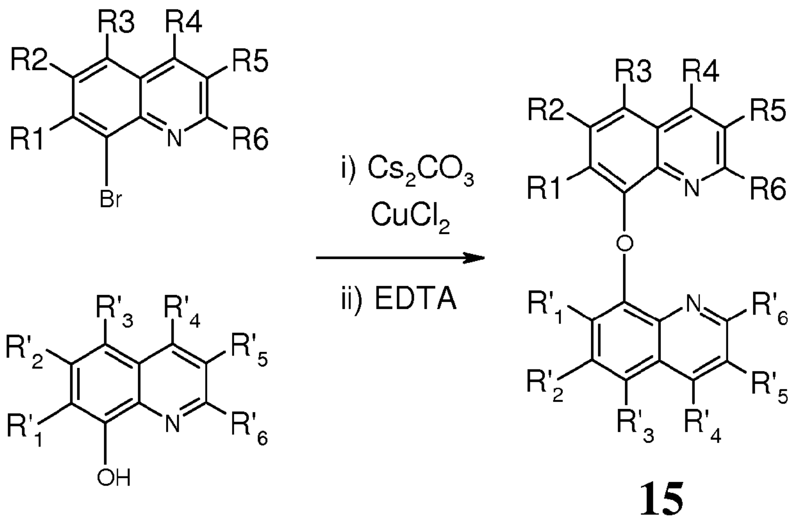


**15**

Schéma 8 : Synthèse de **15** et ses analogues.

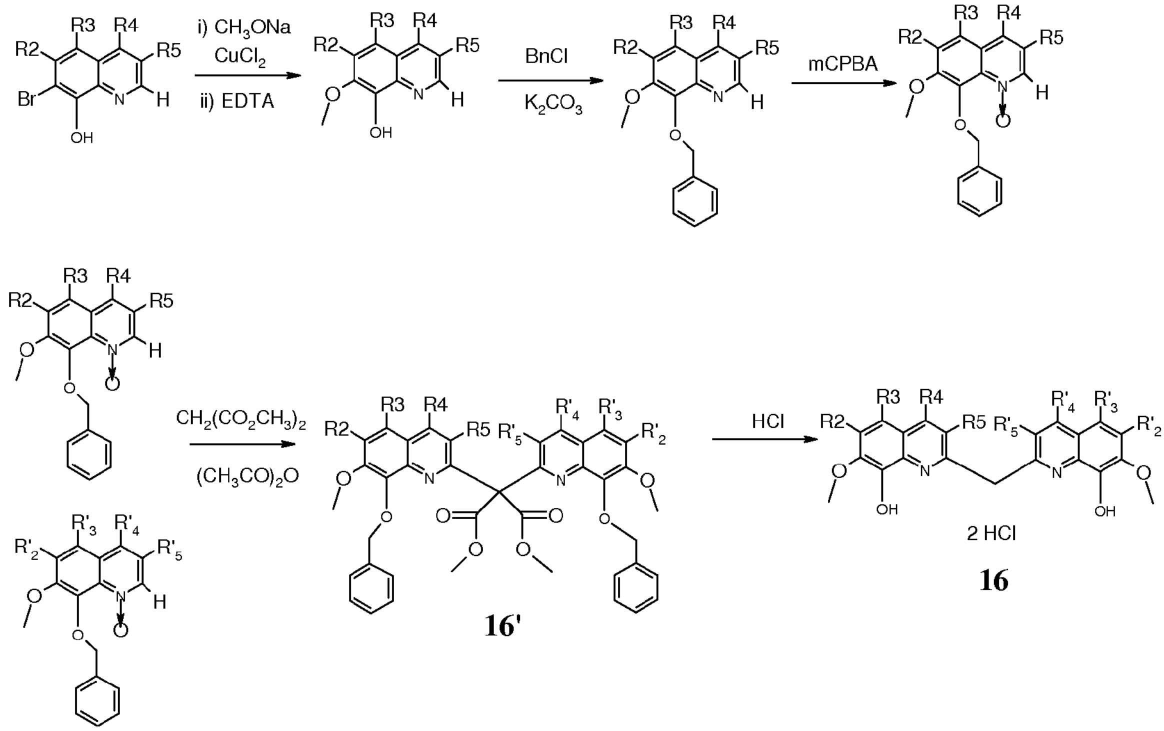


**16'**

**16**

Schéma 9 : Synthèse de **16', 16** et leurs analogues.

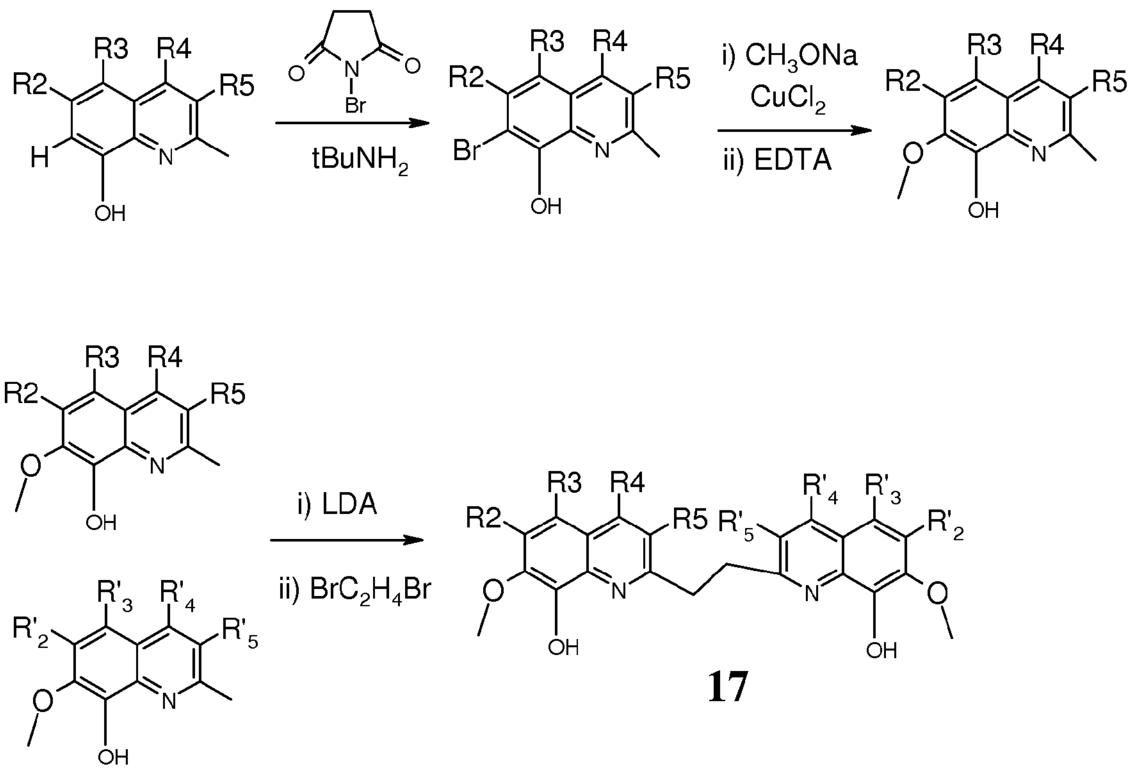


17

Schéma 10 : Synthèse de **17** et ses analogues.

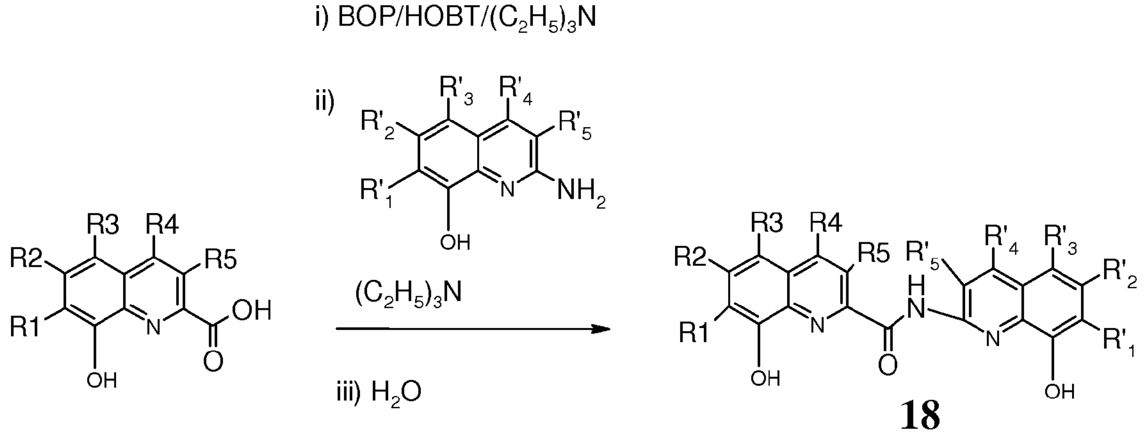


18

Schéma 11 : Synthèse de **18** et ses analogues.

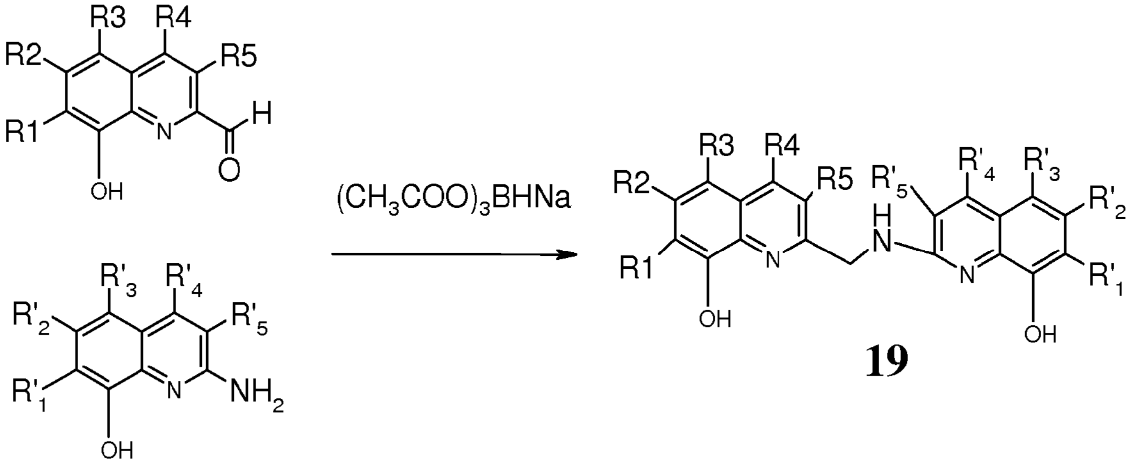


19

Schéma 12 : Synthèse de **19** et ses analogues.

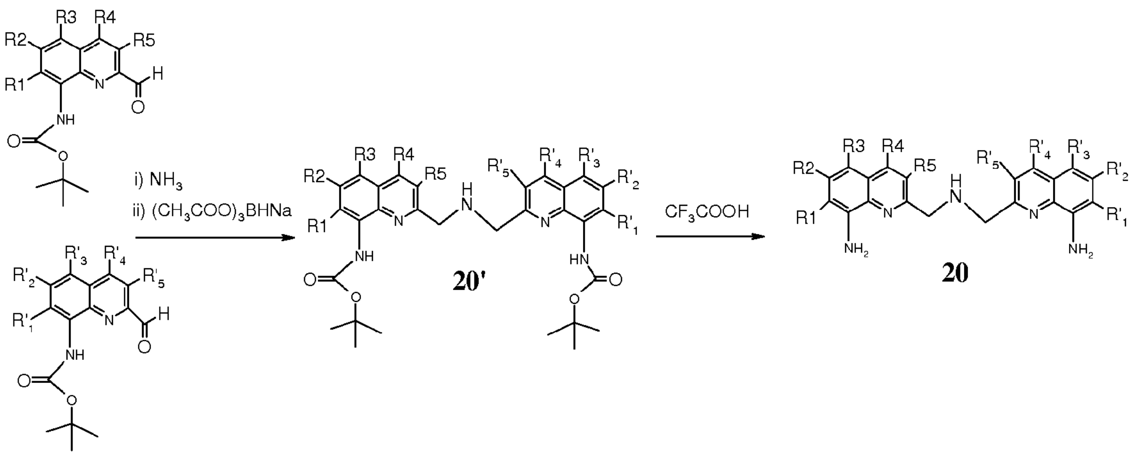


20'

20

Schéma 13 : Synthèse de **20', 20** et leurs analogues.

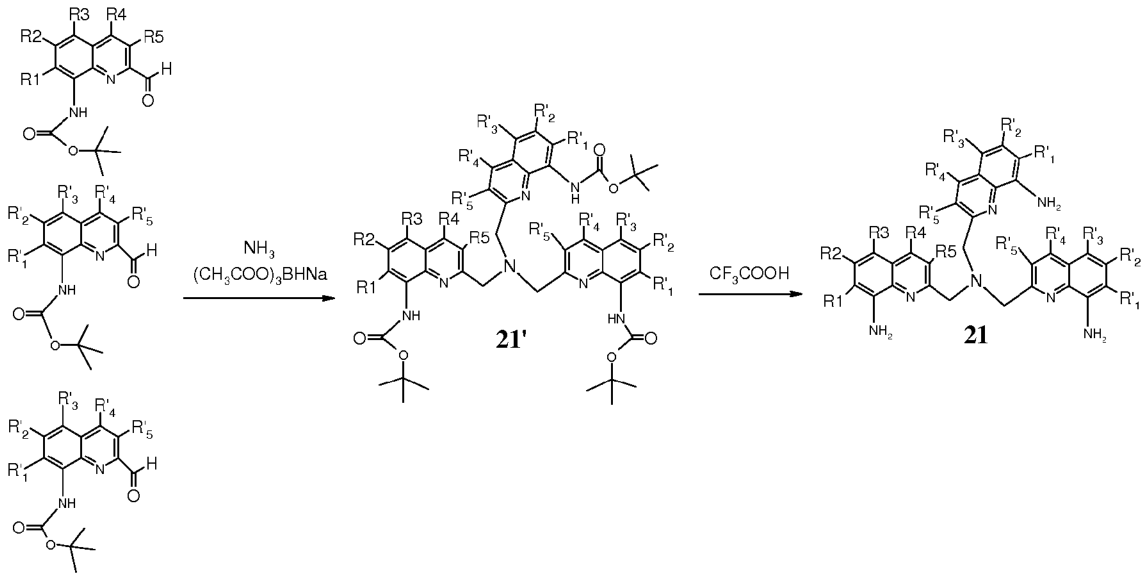


Schéma 14 : Synthèse de **21', 21** et leurs analogues.

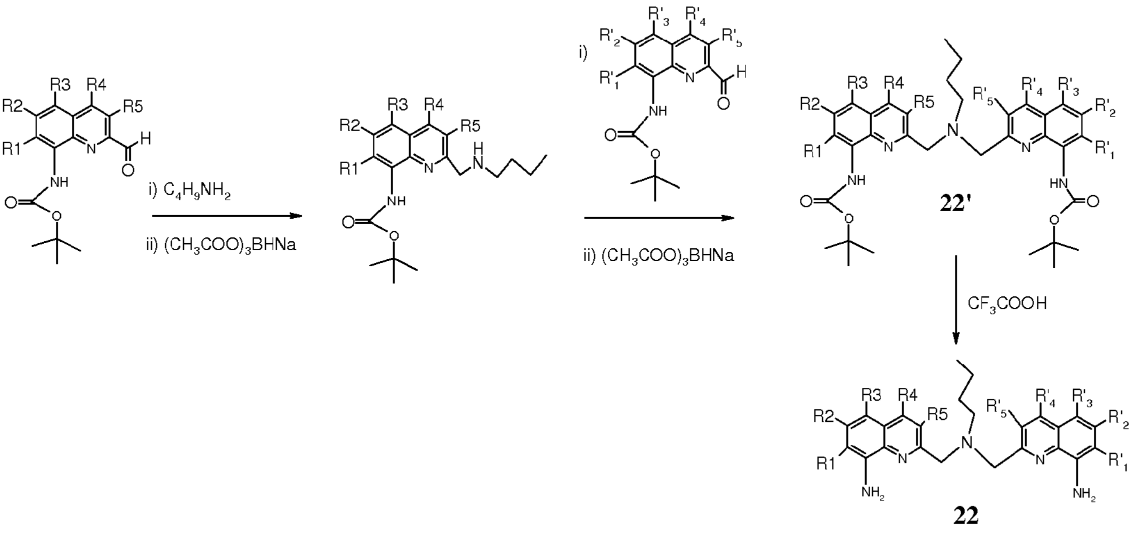


Schéma 15 : Synthèse de **22', 22** et leurs analogues.

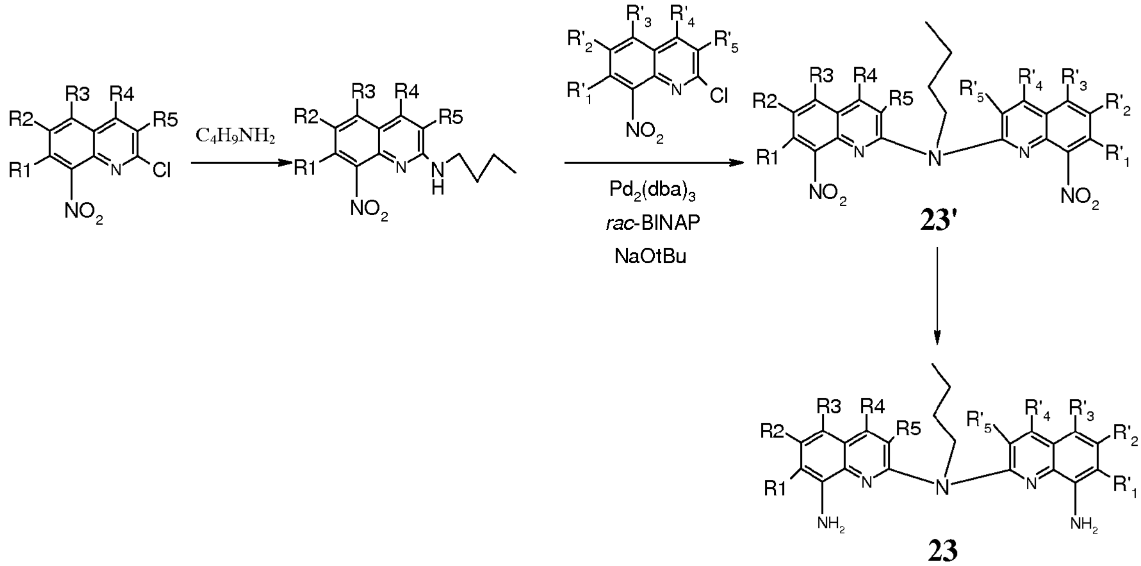


23'

23

Schéma 16 : Synthèse de **23', 23** et leurs analogues.

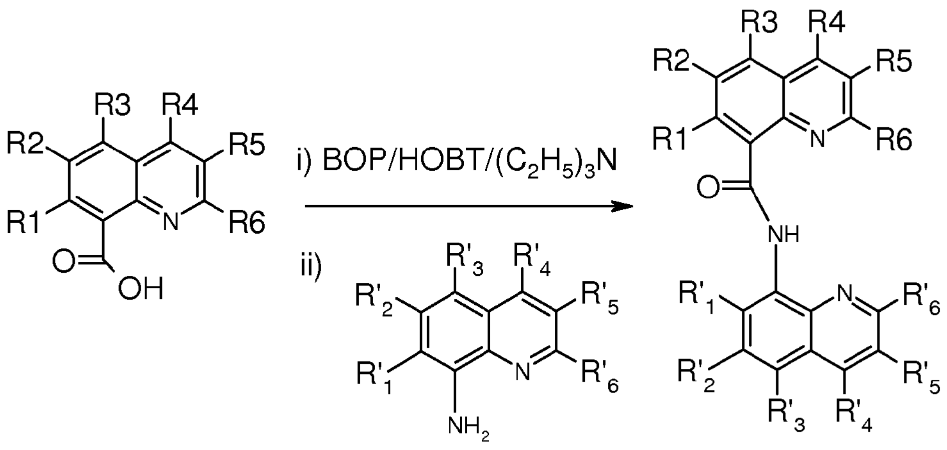


**24**

Schéma 17 : Synthèse de **24** et ses analogues.

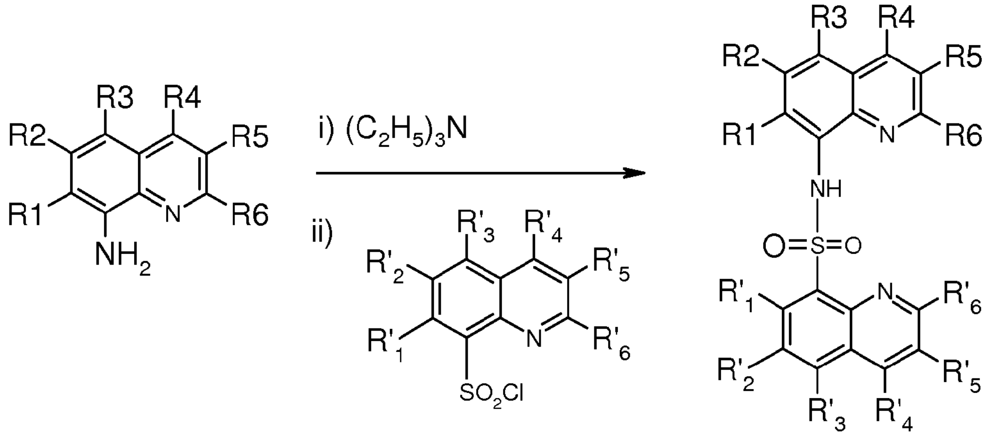


**25**

Schéma 18 : Synthèse de **25** et ses analogues.

Les intermédiaires réactionnels sont disponibles commercialement ou peuvent être préparés par l'homme du métier par application ou adaptation de méthodes connues en soi.

Le procédé selon l'invention peut également comprendre l'étape ultérieure d'isolation des produits de formule (I) obtenus.

Dans les réactions décrites ici, il peut être nécessaire de protéger les groupes fonctionnels réactifs, par exemples les groupes hydroxy, amino, imino, thio, carboxy, lorsqu'ils sont souhaités dans le produit final, pour éviter leur participation indésirable dans les réactions. Les groupes de protection traditionnels peuvent être utilisés conformément à la pratique standard, pour des exemples voir T.W. Greene et P.G.M. Wuts dans Protective Groups in Organic Chemistry, John Wiley and Sons, 1991 ; J.F.W. McOmie in Protective Groups in Organic Chemistry, Plenum Press, 1973.

Le composé ainsi préparé peut être récupéré à partir du mélange de la réaction par les moyens traditionnels. Par exemple, les composés peuvent être récupérés en distillant le solvant du mélange de la réaction ou si nécessaire après distillation du solvant du mélange de la solution, en versant le reste dans de l'eau suivi par une extraction avec un solvant organique immiscible dans l'eau, et en distillant le solvant de l'extrait. En outre, le produit peut, si on le souhaite, être encore purifié par diverses techniques, telles que la recristallisation, la reprécipitation ou les diverses techniques de chromatographie, notamment la chromatographie sur colonne ou la chromatographie en couche mince préparative.

Il sera apprécié que les composés utiles selon la présente invention peuvent contenir des centres asymétriques. Ces centres asymétriques peuvent être indépendamment en configuration R ou S. Il apparaîtra à l'homme du métier que certains composés utiles selon l'invention peuvent également présenter une isomérie géométrique. On doit comprendre que la présente invention comprend des isomères géométriques individuels et des stéréoisomères et des mélanges de ceux-ci, incluant des mélanges racémiques, de composés de formule (I) ci-dessus. Ce type d'isomères peuvent être séparés de leurs mélanges, par l'application ou l'adaptation de procédés connus, par exemple des techniques de chromatographie ou des techniques de recristallisation, ou ils sont préparés séparément à partir des isomères appropriés de leurs intermédiaires.

Aux fins de ce texte, il est entendu que les formes tautomériques sont comprises dans la citation d'un groupe donné, par exemple thio/mercapto ou oxo/hydroxy.

Les sels d'additions acides sont formés avec les composés utiles selon l'invention dans lesquels une fonction de base tels qu'un groupe amino, alkylamino ou dialkylamino, ou encore pyridine, phénol est présente. Les sels d'addition acide pharmaceutiquement acceptables, c'est-à-dire non toxiques, sont préférés. Les sels sélectionnés sont choisis de façon optimale pour être compatibles avec les véhicules pharmaceutiques habituels et adaptés pour l'administration orale ou parentérale. Les sels d'addition acide des composés utiles selon cette invention peuvent être préparés par réaction de la base libre avec l'acide approprié, par l'application ou l'adaptation de procédés connus. Par exemple, les sels d'addition acide des composés utiles selon cette invention peuvent être préparés soit en dissolvant la base libre dans de l'eau ou dans une solution aqueuse alcoolisée ou des solvants adaptés contenant l'acide approprié et en isolant le sel en évaporant la solution, ou en faisant réagir la base libre et l'acide dans un solvant organique, auquel cas le sel se sépare directement ou peut être obtenu par concentration de la solution. Parmi les acides adaptés pour l'usage dans la préparation de ces sels on trouve acide chlorhydrique, acide bromhydrique, acide phosphorique, acide sulfurique,

divers acides carboxyliques et sulfoniques organiques, tels que acide acétique, acide citrique, acide propionique, acide succinique, acide benzoïque, acide tartrique, acide fumarique, acide mandélique, acide ascorbique, acide malique, acide méthanesulfonique, acide toluène-sulfonique, acides gras, adipate, alginate, ascorbate, aspartate, benzène-sulfonate, benzoate, propionate de cyclopentane, digluconate, dodécylsulfate, bisulfate, butyrate, lactate, laurate, sulfate de lauryle, malate, hydroiodide, 2-hydroxyéthanesulfonate, glycéro-phosphate, picrate, pivalate, palmoate, pectinate, persulfate, 3-phényl-propionate, thiocyanate, 2-naphtalène-sulfonate, undécanoate, nicotinate, hémisulfate, heptanoate, hexanoate, camphorate, camphresulfonate et autres.

Les sels d'addition acide des composés utiles selon cette invention peuvent être régénérés à partir des sels par l'application ou l'adaptation de procédés connus. Par exemple, les composés parents utiles selon l'invention peuvent être régénérés à partir de leurs sels d'addition acide par traitement avec un alkali, par exemple une solution de bicarbonate de sodium aqueuse ou une solution d'ammoniac aqueuse.

Les composés utiles selon cette invention peuvent être régénérés à partir de leurs sels d'addition de base par l'application ou l'adaptation de procédés connus. Par exemple, les composés parents utiles selon l'invention peuvent être régénérés à partir de leurs sels d'addition de base par le traitement avec un acide, par exemple un acide chlorhydrique.

Les sels d'addition de base peuvent être formés lorsque le composé utile selon l'invention contient un groupe carboxyle, ou encore pyridine ou phénol, ou un bioisostère suffisamment acide. Les bases qui peuvent être utilisées pour préparer les sels d'addition de base comprennent de préférence celle qui produisent, lorsqu'elles sont associées à un acide libre, des sels pharmaceutiquement acceptables, c'est-à-dire des sels dont les cations ne sont pas toxiques pour le patient dans les doses pharmaceutiques des sels, de sorte que les effets inhibiteurs bénéfiques inhérents à la base libre ne soient pas annulés par les effets secondaires imputables aux cations. Les sels pharmaceutiquement acceptables, comprenant ceux dérivés des sels de métal alcalino-terreux, dans la portée de l'invention comprennent ceux dérivés des bases suivantes : hydrure de sodium, hydroxyde de sodium, hydroxyde de potassium, hydroxyde de calcium, hydroxyde d'aluminium, hydroxyde de lithium, hydroxyde de magnésium, hydroxyde de zinc, ammoniac, éthylènediamine, *N*-méthyl-glucamine, lysine, arginine, ornithine, choline, *N,N'*-dibenzyl-éthylènediamine, chloroprocaïne, diéthanolamine, procaïne, *N*-benzylphénéthyl-amine, diéthylamine, pipérazine, tris(hydroxy-méthyl)aminométhane, hydroxyde de tétraméthylammonium et analogues.

Les composés utiles selon la présente invention peuvent être facilement préparés, ou formés pendant le processus de l'invention, sous forme de solvates (par exemple hydrates). Les hydrates des composés utiles selon la présente invention peuvent être facilement préparés par la recristallisation d'un mélange de solvant aqueux/organique, en utilisant des solvants organiques tels que dioxane, tétrahydrofurane ou méthanol.

Les produits de base ou les réactifs utilisés sont disponibles commercialement et/ou peuvent être préparés par l'application ou l'adaptation de procédés connus, par exemple des procédés tels que décrits dans les Exemples de Référence ou leurs équivalents chimiques évidents.

Selon la présente invention, les composés de formule (I) présentent une activité biologique de chélateur de métaux et/ou de dissolution d'agrégats amyloïdes.

La présente invention a également pour objet les compositions pharmaceutiques comprenant un composé selon l'invention avec un véhicule ou excipient pharmaceutiquement acceptable.

De préférence, ladite composition contient une quantité efficace du composé selon l'invention.

Selon un autre objet, la présente invention concerne également l'utilisation de composés de formule générale (I) pour la préparation de compositions pharmaceutiques destinées à chélater les ions métalliques, plus préférentiellement ceux de zinc, cuivre, fer, aluminium ou manganèse, plus préférentiellement les ions du zinc de degré d'oxydation II, du cuivre de degré d'oxydation I, II, du fer de degré d'oxydation II, III, IV ou V.

L'invention concerne également l'utilisation de composés de formule générale (I) pour la préparation de compositions pharmaceutiques destinées à dissoudre les agrégats amyloïdes.

Selon un autre objet, la présente invention concerne également l'utilisation de composés de formule générale (I) pour la préparation de compositions pharmaceutiques destinées à prévenir et/ou traiter les maladies neurodégénératives. Plus particulièrement, les maladies neurodégénératives sont choisies parmi la maladie d'Alzheimer, la maladie de Parkinson, la maladie d'Huntington, la sclérose latérale amyotrophique ou la trisomie 21, les encéphalopathies spongiformes, telle que la maladie de Kreutzfeld-Jacob.

Selon un autre objet, la présente invention concerne également les méthodes de traitement thérapeutiques citées ci-dessus comprenant l'administration d'un composé selon l'invention à un patient qui en a besoin.

De préférence, ladite composition est administrée à un patient qui en a besoin.

Les compositions pharmaceutiques selon l'invention peuvent être présentées sous des formes destinées à l'administration par voie parentérale, orale, rectale, intraveineuse, permuqueuse ou percutanée.

Elles seront donc présentées sous forme de solutés ou de suspensions injectables ou flacons multi-doses, sous forme de comprimés nus ou enrobés, de dragées, de capsules, de gélules, de pilules, de cachets, de poudres, de suppositoires ou de capsules rectales, de solutions ou de suspensions, pour l'usage percutané dans un solvant polaire, pour l'usage permuqueux.

Les excipients qui conviennent pour de telles administrations sont les dérivés de la cellulose ou de la cellulose microcristalline, les carbonates alcalino-terreux, le phosphate de magnésium, les amidons, les amidons modifiés, le lactose pour les formes solides.

Pour l'usage rectal, le beurre de cacao ou les stéarates de polyéthylèneglycol sont les excipients préférés.

Pour l'usage parentéral, l'eau, les solutés aqueux, le sérum physiologique, les solutés isotoniques sont les véhicules les plus commodément utilisés.

La posologie peut varier dans les limites importantes (0,5 mg à 1000 mg) en fonction de l'indication thérapeutique et de la voie d'administration, ainsi que de l'âge et du poids du sujet.

<u>Légendes des figures</u> :

**Figure 1 :** Dérivés de quinoléine testés sur Aβ.

**Figure 2 :** Géométries *cis* et *trans* pour la complexation d'ions métalliques par les dérivés de 8-hydroxyquinoléine.

**Figure 3 :** Structure cristalline du [2,2'-(2,2-propanediyl)-bis[8-quinoléinolato]nickel(II); les atomes d'hydrogène ont été omis. Remarquer que le complexe est de configuration *cis* (Figure 2) et que les deux entités quinoléine du ligand chélatent le même atome métallique.

**Figure 4 :** Structure cristalline de bis{7-(méthyloxy)8-[phényl-méthyl)oxy]-2-quinoléinyl}propanedioate, les atomes d'hydrogène ont été omis.

**Figure 5 :** Structure cristalline de 2-chloro-8-nitroquinoléine.

**Figure 6:** Spectres UV-visible obtenus lors de la titration Cu(II) par le ligand **3**. Les spectres de titration sont obtenus avec 15 μM de ligand (L) dans le mélange tampon Tris·HCl 20 mM ; NaCl 150 mM (pH = 7,4)/$CH_3OH$ (1/1, v/v) et différents rapports de $CuCl_2$. La direction des flèches indique les variations observées sur les spectres lorsque le rapport Cu(II)/L augmente.

**Figure 7:** Spectres UV-visible de **3** ; Cu(II)-**3**; EDTA et (EDTA)Cu(II) dans le mélange tampon Tris·HCl 20 mM ; NaCl 150 mM (pH = 7,4)/$CH_3OH$ (1/1, v/v). La concentration des ligands et des complexes métalliques est de 15 μM. Remarquer qu'une absorbance à λ = 379 nm est seulement observée pour le complexe Cu(II)-**3**.

**Figure 8:** Réaction de compétition pour la complexation de $CuCl_2$ par le ligand **3** ($L_s$) et EDTA ($L_c$). Les spectres sont obtenus avec 15 μM de **3** et de $CuCl_2$ et différents rapports de EDTA dans le mélange 20 mM tampon Tris·HCl/150 mM NaCl (pH = 7,4)/$CH_3OH$ (1/1, v/v). La direction des flèches indique les variations d'absorbance observées quand le rapport $L_c/L_s$ augmente.

**Figure 9:** Variation du pourcentage de peptide $A\beta_{1-42}$ soluble en fonction de la stoechiométrie Aβ/Cu(II). Le peptide $A\beta_{1-42}$ (5 μM) est incubé durant deux heures à 37°C dans du tampon Tris·HCl 20 mM (pH = 7,4); NaCl 150 mM en présence de différentes stoechiométries de Cu(II) puis le mélange réactionnel est centrifugé. Le pourcentage de peptide dans le surnageant ($A\beta_{1-42}$ soluble) et dans le précipité ($A\beta_{1-42}$ agrégé) sont alors quantifiés à l'aide d'un kit Micro BCA Protein Assays. Valeur 1 et Valeur 2 sont issues de deux expériences indépendantes, quand le nombre de mesures est ≥ 3, la moyenne et l'écart type des valeurs obtenues sont présentés.

**Figure 10:** Variation du pourcentage de peptide $A\beta_{1-42}$ soluble en présence de différentes stoechiométries de $CuCl_2$ et de ligands. Le peptide $A\beta_{1-42}$ (5 μM) est incubé durant une heure à 37°C dans du tampon Tris·HCl 20 mM (pH = 7,4); NaCl 150 mM en présence de $CuCl_2$ 12,5 ou 20 μM puis le ligand [12,5 μM (sauf pour les monomères de quinoléine comme 8-hydroxyquinoléine, Clioquinol, 8-hydroxyquinaldine et 8-aminoquinoléine où cette valeur est de 25 μM) ou 200 μM] est ajouté. Après une heure supplémentaire d'incubation, le mélange réactionnel est centrifugé. Le pourcentage de peptide dans le surnageant ($A\beta_{1-42}$ soluble) et dans le précipité ($A\beta_{1-42}$ agrégé) sont alors quantifiés à l'aide d'un kit Micro BCA Protein Assays. Pour chaque composé, les valeurs de gauche (en gris foncé) et de droite (en gris clair) représentent les résultats obtenus pour respectivement les rapports 200 μM de ligand/20 μM $CuCl_2$ et 12,5 μM (dans le cas des dimères de quinoléine ou 25 μM dans le cas des monomères) de ligand/ 12,5 μM $CuCl_2$. Sont aussi indiqués les pourcentages de peptide $A\beta_{1-42}$ soluble en présence des différentes concentrations de $CuCl_2$ en l'absence de ligand

**Figure 11:** Variation de la production de $H_2O_2$ par les complexes de cuivre du peptide $A\beta_{1-42}$ en fonction de la stoechiométrie Cu(II)/Aβ. Les expériences sont réalisées durant 5 minutes dans du tampon phosphate de sodium (pH = 7,4) en présence de 0,4 μM $CuCl_2$, 10 μM ascorbate et d'air avec ou sans 0,4 μM de **3** ou de **7.** Le $H_2O_2$ libéré est quantifié à l'aide d'un kit Amplex-Red $H_2O_2$/HRP Assay.

Les exemples suivants illustrent l'invention, sans toutefois la limiter. Les produits de départ utilisés sont des produits connus ou préparés selon des modes opératoires connus.

Les abréviations suivantes ont été utilisées ci-avant ou ci-après :

Aβ: peptide β-amyloïde; BSA: Albumine de Sérum Bovin; biPy: 2,2'-bipyridine; BnCl : chlorure de benzyle; BOP: (benzotriazole-1-yloxy)tris(diméthylamino)phosphonium hexafluorophosphate; CTDA: acide *trans*-1,2-diaminocyclohexane-*N,N,N',N'*-tétraacétique; d: doublet; dd: doublet dédoublé ; DIC : détection par ionisation chimique ; Dien: diéthylènetriamine; DMF : diméthylformamide ; DMSO : diméthylsulfoxide ; DTPA: acide diéthylène-triaminepentaacétique; EDA: éthylènediamine; EDDA: acide éthylènediamine-*N,N'*-diacétique; EDTA: acide éthylènediaminetétraacétique; EGTA: acide éthylènebis(oxyéthylènenitrilo)-tétraacétique; eq: équivalent; Eq. : équation ; HOBT: 1-hydroxybenzotriazole monohydrate; HRP: péroxidase de raifort (Horse Radish Peroxidase); HIDA: acide *N*-(2-hydroéthyl)iminodiacétique; LDA: diisopropylamine de lithium; m : massif ; mCPBA: acide *m*-chloroperbenzoïque; NBS: *N*-bromosuccinimide; NTA: acide nitrilotriacétique; $Pd_2(dba)_2$ : tris(dibenzylidène-acétone)-dipalladium(0); PM : poids moléculaire ; *rac*-BINAP: rac-2,2'-bis(diphénylphosphino)-1,1',binaphthyl; s : singulet ; SM : spectrométrie de masse ; t : triplet ; Tetren: tétraéthylènepentamine; THF : tétrahydrofurane ; Trien: triéthylènetétramine ; t/min : tours par minute.

EXEMPLES

Description des exemples :

**Synthèse :**

***Remarques générales concernant la synthèse organique :***

8-méthyloxyquinaldine a été synthétisé suivant une méthode décrite dans la littérature. (C. Kitamura et al., J. Chem. Soc., Perkin Trans. 1 2000, 781-785). *N*-butyl-2,2'-imino-bis(8-nitroquinoléine) a été synthétisée selon les références: H. Jiang et al. Tetrahedron 2003, 59, 8365-8374; G. Xue et al., Tetrahedron 2001, 57, 7623-7628 et P. Belser et al. Tetrahedron 1996, 52, 2937-2944. 7-bromo-8-hydroxyquinoléine (préparée selon la méthode de Pearson: D.E. Pearson et al. J. Org. Chem. 1967, 32, 2358-2360; G. E. Collis et al. Acta Cryst. 2003, C59, o443-o444). 1,1-diméthyl(2-formyl-8-quinoléinyl)carbamate a été synthétisé selon G. Xue et al., Tetrahedron 2001, 57, 7623-7628. 2-Chloro-8-nitroquinoléine a été synthétisé selon M. C. Kimber et al., Aust. J. Chem. 2003, 56, 39-44.

L'acétonitrile a été séché sur tamis moléculaire 4 Å. Le tétrahydrofurane (THF) a été distillé sur de la benzophénone en présence de sodium. $CuCl_2$ sec a été obtenu par chauffage sous vide à 50°C. Le dichlorométhane a été séché sur allumine basique. Les autres réactifs et solvants utilisés proviennent de fournisseurs standard en produits chimiques et ont été utilisés sans purification ultérieure.

Les spectres RMN ont été enregistrés sur des appareils Bruker 200, 250 ou 500 MHz. Le spectromètre de masse en ionisation électrospray (IES-SM) était un appareil Perkin-Elmer SCIEX API 365, les échantillons ont été introduits dans la source électrospray avec une pompe à seringue Havard Apparatus. Les spectres UV-visible ont été enregistrés sur un spectrophotomètre à barrette de diodes Hewlett Packard 8452A ou un spectrophotomètre Perkin-Elmer Lamda 35.

Les chromatographies couche mince sont réalisées sur plaque de silice.

**Diméthyl-bis[8-(acétyloxy)-2-quinoléinyl]propanedioate (composé 1"):**

Le protocole a été optimisé à partir des travaux de : Y. Yamamoto et al., Bull. Chem. Soc. Jpn. 1978, 51, 3489-3495. Une suspension de 8-hydroxyquinoléine-*N*-oxyde (4,47 g; 27,7 mmol) et de diméthylmalonate (3,35 mL; 29,1 mmol) dans de l'anhydride acétique (11,2 mL) est agitée durant 27 jours à température ambiante protégée de l'humidité (grâce à un tube de $CaCl_2$) et de la lumière. La suspension orange obtenue est refroidie à 0°C et du méthanol (6,7 mL) est ajouté. Après 2 heures d'agitation à température ambiante, de l'eau (30 mL) est ajoutée en deux temps et le mélange est maintenu sous agitation 2 heures de plus. Un précipité est alors collecté et lavé avec un mélange acide acétique/eau (1/1, v/v, 30 mL) puis à l'eau avant d'être séché 15 heures à 110°C. Il est alors dissout à chaud dans du chloroforme (15 mL) et précipité par addition de méthanol (45 mL) pour donner du diméthyl-bis[8-(acétyloxy)-2-quinoléinyl]propanedioate sous la forme d'une poudre blanche (4,58 g; 9,12 mmol, rendement = 66 %). RMN-$^1$H (250 MHz, $CDCl_3$) δ, ppm: 8,06 (d, $^3J$ (H, H) = 9,0 Hz, 2 H); 7,87 (d, $^3J$ (H, H) = 9,0 Hz, 2 H); 7,64 (dd, $^3J$ (H, H) = 8,0 Hz, $^4J$ (H, H) = 1,5 Hz, 2 H); 7,49 (m, 2 H); 7,41 (dd, $^3J$ (H, H) = 7,5 Hz, $^4J$ (H, H) = 1,5 Hz, 2 H); 3,94 (s, 6 H); 2,53 (s, 6 H). SM (DIC, $NH_3$) m/z = 503 ($MH^+$). Analyse (%) pour $C_{27}H_{22}N_2O_8 \cdot 0,5H_2O$: calculé C 63,40; H 4,53; N 5,48; trouvé C 63,46; H 4,10; N 5,56.

**2,2'-Méthanediyl-bis(8-hydroxy-2-quinoléinium) dichlorure dihydrate (composé 1'):**

Le protocole a été optimisé à partir des travaux de : Y. Yamamoto et al., Bull. Chem. Soc. Jpn. 1978, 51,

3489-3495. Une suspension de diméthyl-bis[8-(acétyloxy)-2-quinoléinyl]propanedioate (4,00 g; 7,97 mmol) dans du HCl aqueux à 20 % (295 mL) est chauffée à reflux durant 5 heures 30 min puis agitée 4 heures à température ambiante pour donner un précipité jaune qui est récupéré et séché sous vide. Le produit est alors dissout à chaud dans du méthanol (6,1 mL) contenant de l'acide chlorhydrique concentré (610 $\mu$L) puis cristallisé par addition d'acide chlorhydrique concentré (6,1 mL) pour donner du 2,2'-méthane-diyl-bis(8-hydroxy-2-quinoléinium) dichlorure dihydrate sous la forme de cristaux jaunes (3,27 g ; 7, 96 mmol, rendement quantitatif). RMN-$^1$H (250 MHz, DMSO-$d_6$) $\delta$, ppm : 8,72 (d, $^3J$ (H, H) = 9,0 Hz, 2 H); 7,89 (d, $^3J$ (H, H) = 9,0 Hz, 2 H); 7,58 (m, 4 H); 7,39 (dd, $^3J$ (H, H)= 7,5 Hz, $^4J$ (H, H)= 2,0 Hz, 2 H); 5,14 (s, 2 H). RMN-$^{13}$C (100 MHz, DMSO-$d_6$) $\delta$, ppm: 156,5 (Cq); 151,3 (Cq); 142,7 (CH); 134,0 (Cq); 129,7(CH); 129,0 (Cq); 124,3 (CH); 118,9 (CH); 115,1 (CH); 42,2 ($CH_2$). SM (DIC, $NH_3$) m/z: 302 ($MH^+$). Analyse (%) pour $C_{19}H_{12}N_2O_2 \cdot 2HCl \cdot 2H_2O$: calculé C 55,49; H 4,90; N 6,81; trouvé C 55,95; H 4,79; N 6,66.

**2,2'-Méthanediyl-bis(8-hydroxyquinoléine)(composé 1):**

Du 2,2'-méthanediyl-bis(8-hydroxy-2-quinoléinium) dichlorure dihydrate (150 mg; 0,36 mmol), mis en suspension dans 20 mL de $CH_2Cl_2$, est lavé trois fois avec du tampon acétate de sodium (0,1 M; pH = 7,0) puis à l'eau. Le solvant de la phase organique est évaporé pour donner, après séchage sous vide, **1** sous la forme d'un solide orange (110 mg, 0,36 mmol, rendement quantitatif). RMN-$^1$H (250 MHz, $CDCl_3$) $\delta$, ppm: 8,20 (s large, 2 H); 8,08 (d, $^3J$ (H, H) = 8,5 Hz, 2 H); 7,43 (d, $^3J$ (H, H) = 8,5 Hz, 2 H); 7,42 (m, 2 H); 7,30 (dd, $^3J$ (H, H) = 8,0 Hz, $^4J$ (H, H) = 1,0 Hz, 2 H); 7,16 (dd, $^3J$ (H, H) = 7,5 Hz, $^4J$ (H, H) = 1,0 Hz, 2 H); 4,69 (s, 2 H). UV/vis [DMSO/Tris·HCl 20 mM pH = 7,4 ; NaCl 150 mM (8/2, v/v)]: $\lambda$ nm ($\varepsilon$ $M^{-1}$ $cm^{-1}$) = 256 (50 800), 312 (8 700), 454 (2 200), 480 (3500), 512 (2 700).

**2,2'-Méthanediyl-bis(5-chloro-8-hydroxyquinoléine) (composé 2) :**

Le protocole a été établi à partir des travaux de : H. Gershon, M. W. Mc Neil, J. Heterocycl. Chem. 1972, 9, 659-666. A une suspension de 2,2'-méthanediyl-bis(8-hydroxy-2-quinoléinium) dichlorure dihydrate (500 mg; 1,22 mmol) dans $H_2SO_4$ à 97 % (12 mL), refroidie sur bain de glace, est ajouté du *N*-chlorosuccinimide (324 mg; 2,43 mmol) par petites portions. Le mélange est alors agité 15 min à 0°C puis 4 heures à température ambiante. Il est ensuite versé sur de la glace pour donner une suspension rose qui est neutralisée avec une solution aqueuse de soude. Le mélange est alors centrifugé. Après avoir enlevé le surnageant, le précipité est suspendu dans de l'eau et extrait au dichlorométhane. La phase organique est lavée à l'eau puis le solvant est évaporé sous vide pour donner, après séchage sous vide, **2** sous la forme d'une poudre orange pâle (440 mg; 1,19 mmol; rendement = 98 %). RMN-$^1$H (250 MHz, $CDCl_3$) $\delta$, ppm: 8,45 (d, $^3J$ (H, H) = 8,5 Hz, 2H); 8,12 (s large, 2 H); 7,56 (d, $^3J$ (H, H) = 8,5 Hz, 2 H); 7,48 (d, $^3J$ (H, H) = 8,0 Hz, 2 H); 7,10 (d, $^3J$ (H, H) = 8,0 Hz, 2 H); 4,74 (s, 2 H). RMN-$^{13}$C (63 MHz, $CDCl_3$) $\delta$, ppm: 157,4 (Cq); 151,0 (Cq); 138,1 (Cq); 134,2 (CH); 127,2 (CH); 125,0 (Cq); 123,3 (CH); 120,4 (Cq); 110,3 (CH); 47,5 ($CH_2$). SM (DIC, $NH_3$) m/z: 371 ($MH^+$). Analyse (%) pour $C_{19}H_{12}Cl_2N_2O_2 \cdot 0,1Na_2SO_4$: calculé C 59,21; H 3,14; N 7,27; trouvé C 59,27; H 2,58; N 7,05. UV/vis [dioxane/Tris·HCl 20 mM pH = 7,4 ; NaCl 150 mM (1/1, v/v)]: $\lambda$ nm ($\varepsilon$ $M^{-1}$ $cm^{-1}$) = 258 (59 900), 320 (7 000), 460 (2 000), 489 (2 900), 518 (2 100).

**[2,2'-(2,2-Propanediyl)-bis[8-quinoléinolato]nickel(II) :**

La synthèse a été réalisée selon le protocole de la référence : Y. Yamamoto et al., Bull. Chem. Soc. Jpn. 1978, 51, 3489-3495. Des données de RMN plus précises que celles préalablement publiées sont ajoutées ci-après. RMN-$^1$H (250 MHz, $CDCl_3$) $\delta$, ppm: 8,15 (d, $^3J$ (H, H) = 9,0 Hz, 2 H); 7,45 (d large, $^3J$ (H, H) = 9,0 Hz, 2 H); 7,33 (m, 2 H); 6,91 (d large, $^3J$ (H, H) = 7,5 Hz, 2 H); 6,81 (d large, $^3J$ (H, H) = 6,5 Hz, 2 H); 1,93 (s, 6 H). RMN-$^{13}$C (63 MHz, $CDCl_3$) $\delta$, ppm: 166,4 (Cq); 159,1 (Cq); 144,3 (Cq); 138,7 (CH); 130,7 (CH); 127,7 (Cq); 119,3 (CH); 113,6 (CH); 110,4 (Cq); 50,7 (Cq); 32,4 ($CH_3$). SM(DIC, $NH_3$): *m/z* = 387 ($MH^+$), 404 ($MNH_4^+$). La structure de la molécule a été confirmée par analyse par diffraction de rayons X sur des monocristaux obtenus par cristallisation du produit dans du méthanol. La structure du complexe est présentée Figure 3. Les paramètres de l'analyse cristalline sont les suivants: système cristallin triclinique, P-1 ; a = 12,456(4) Å, b = 12,650(4) Å, c = 13,983(5) Å, $\alpha$= 112,260(5)°, $\beta$= 105,956(5)°, $\gamma$ = 90,115(6)°.

**2,2'-(2,2-Propanediyl)-bis(8-hydroxyquinoléine) (composé 3):**

Le protocole a été optimisé à partir des travaux de : Y. Yamamoto et al., Bull. Chem. Soc. Jpn. 1978, 51, 3489-3495. Des cristaux rouges de [2,2'-(2,2-propanediyl)-bis[8-quinoléinolato]nickel(II) (3,32 g; 8,60 mmol) sont mis en suspension dans 80 mL d'éthanol puis 16 mL d'acide chlorhydrique concentré sont ajoutés pour donner une solution verte à laquelle 320 mL d'eau bouillante sont ajoutés par portions pour former des cristaux jaunes. Après refroidissement, les cristaux sont collectés et lavés avec une solution de HCl aqueux 1 M puis séchés à l'air à température ambiante durant 15 heures avant d'être redissout à chaud dans 32 mL d'éthanol. Une solution aqueuse bouillante d'acétate de

sodium 18,4 mM est alors ajoutée par portions. Après refroidissement, un précipité est récupéré par centrifugation puis lavé à l'eau et extrait avec du dichlorométhane pour donner, après évaporation du solvant et séchage sous vide, **3** sous la forme d'un solide blanc (1,81 g). Le surnageant de précipitation de l'étape précédente, qui contient une quantité résiduelle du complexe de nickel, est neutralisé avec une solution aqueuse de soude 6 M puis 1 L de dichlorométhane est ajouté; le produit est démétallé par addition d'une solution aqueuse d'acide éthylènediaminetétraacétique (2 x 10 g dans 500 mL) sous agitation durant 1 heure. La phase organique est récupérée, lavée à l'eau puis concentrée sous vide. Le mélange est alors purifié par chromatographie sur gel de silice grâce à un gradient de 0 à 1 % de $CH_3OH$ dans du $CH_2Cl_2$ (v/v) pour donner 0,83 g supplémentaire de **3** sous la forme d'un solide blanc (masse totale = 2,64 g; 8,00 mmol, rendement = 93 %). RMN-$^1$H (250 MHz, $CDCl_3$) δ, ppm: 8,30 (s large, 2 H); 8,02 (d, $^3J$ (H, H) = 9,0 Hz, 2 H); 7,45 (m, 2 H); 7,30 (dd, $^3J$ (H, H) = 7,5 Hz, $^4J$ (H, H) = 1,0 Hz, 2 H); 7,22 (d, $^3J$ (H, H) = 9,0 Hz, 2 H); 7,21 (dd, $^3J$ (H, H) = 7,5 Hz, $^4J$ (H, H) = 1,0 Hz, 2 H); 2,0 (s, 6 H). RMN-$^{13}$C (63 MHz, $CDCl_3$) δ, ppm: 164,7 (Cq); 152,1 (Cq); 136,8 (Cq); 136,5 (CH); 127,5 (CH); 126,8 (Cq); 121,2 (CH); 117,5 (CH); 110,3 (CH); 49,3 (Cq); 28,0 ($CH_3$). SM (DIC, $NH_3$) m/z: 331 ($MH^+$). Analyse (%) pour $C_{21}H_{18}N_2O_2$: calculé C 76,34; H 5,49; N 8,48; trouvé C 75,80; H 5,30; N 8,38. UV/vis [$CH_3OH$/Tris·HCl 20 mM pH = 7,4 ; NaCl 150 mM (1/1, v/v)]: λ nm (ε $M^{-1}$ $cm^{-1}$) = 203 (77 700), 251 (84 600), 308 (6 900).

**2,2'-(2,2-Propanediyl)-bis(5-chloro-8-hydroxyquinoléine) (composé 4) :**

Le protocole a été établi à partir des travaux de : H. Gershon et al., J. Heterocycl. Chem. 1972, 9, 659-666. A une solution de **3** (50 mg; 0,15 mmol) dans du $H_2SO_4$ à 97 % (1,5 mL) et refroidie sur bain de glace est ajouté du *N*-chlorosuccinimide (40 mg; 0,30 mmol) par petites portions. Le mélange est agité durant 15 min à 0°C puis 3 heures à température ambiante. Il est ensuite versé sur de la glace pour donner une suspension jaune qui est neutralisée avec une solution aqueuse de soude 3 M. Le mélange est centrifugé, le surnageant est enlevé puis le précipité est suspendu dans de l'eau et extrait au dichlorométhane. La phase organique est lavée à l'eau puis le solvant est évaporé sous vide pour donner **4** sous la forme d'une poudre blanche (55 mg; 0,14 mmol, rendement = 93 %). RMN-$^1$H (250 MHz, $CDCl_3$) δ, ppm: 8,37 (d, $^3J$ (H, H) = 9,0 Hz, 2 H); 8,19 (s large, 2 H); 7,50 (d, $^3J$ (H, H) = 8,0 Hz, 2 H); 7,32 (d, $^3J$ (H, H) = 9,0 Hz, 2 H); 7,13 (d, $^3J$ (H, H) = 8,2 Hz, 2 H); 2,00 (s, 6 H). RMN-$^{13}$C (63 MHz, $CDCl_3$) δ, ppm: 165,2 (Cq); 151,1 (Cq); 137,3 (Cq); 134,0 (CH); 127,2 (CH); 124,7 (Cq); 121,9 (CH); 120,5 (Cq); 110,2 (CH); 49,4 (Cq); 27,9 ($CH_3$). SM (DIC, $NH_3$) m/z: 399 ($MH^+$). Analyse (%) pour $C_{21}H_{16}Cl_2N_2O_2$: calculé C 63,17; H 4,04; N 7,02; trouvé C 63,02; H 3,76; N 6,87. UV/vis [dioxane/Tris·HCl 20 mM pH = 7,4 ; NaCl 150 mM (1/1, v/v)]: λ nm (ε $M^{-1}$ $cm^{-1}$) = 258 (72 500), 315 (8 300).

**2,2'-(Difluorométhanediyl)-bis(8-hydroxyquinoléine) (composé 5) :**

A une suspension orange de 2,2'-méthanediyl-bis(8-hydroxy-2-quinoléine) (composé **1** 110 mg; 0,36 mmol) dans 30 mL d'acétonitrile sec est ajouté sous azote du 1-chlorométhyl-4-fluoro-1,4-diazoniabi-cyclo(2,2,2)octane de bis (tétrafluoroborate) (F-TEDA-$BF_4$, Selectfluor™, 258 mg; 0,73 mmol) par petites portions ce qui entraîne la solubilisation des produits sous la forme d'une solution jaune. Le mélange est agité 90 min à température ambiante. Après évaporation du solvant, le produit est solubilisé dans du dichlorométhane et lavé deux fois à l'eau. La phase organique est concentrée et séchée sous vide pour donner **5** sous la forme d'une poudre jaune (123 mg, 0,36 mmol, rendement quantitatif). RMN-$^1$H (250 MHz, $CDCl_3$) δ, ppm: 8,34 (d, $^3J$ (H, H) = 8,5 Hz, 2 H); 8,00 (d, $^3J$ (H, H) = 8,5 Hz, 2 H); 7,78 (s, 2 H); 7,52 (m, 2 H); 7,39 (dd, $^3J$ (H, H) = 8,0 Hz, $^4J$ (H, H) = 1,0 Hz, 2 H); 7,19 (dd, $^3J$ (H, H) = 7,5 Hz, $^4J$ (H, H) = 1,0 Hz, 2 H). RMN-$^{13}$C (126 MHz, $CDCl_3$) δ, ppm: 152,3 (t, $^2J$ (C, F) = 30.0 Hz, Cq); 152,3 (Cq); 137,6 (CH); 137,2 (Cq); 129,3 (CH); 128,5 (Cq); 118,7 (t, $^3J$(C, F) = 3.5 Hz, CH); 117,9 (CH); 117,0 (t, $^1J$ (C, F) = 245,6 Hz, Cq); 111,0 (CH). RMN-$^{19}$F (188 MHz, $CDCl_3$, référence : $CF_3CO_2H$) δ, ppm: - 22,2 (s). SM (DIC, $NH_3$) m/z : 339 ($MH^+$), 356 ($MNH_4^+$). Analyse (%) pour $C_{19}H_{12}F_2N_2O_2 \cdot 0,3\ H_2O$: calculé C 66,39 ; H 3,69 ; N 8,15; trouvé C 66,25 ; H 3,62 ; N 8,27. UV/vis [$CH_3OH$/Tris·HCl 20 mM pH = 7,4; NaCl 150 mM (1/1, v/v)]: λ nm (ε $M^{-1}$ $cm^{-1}$) = 241 (50 000), 251 (55 600), 314 (4 700).

**Bis(8-hydroxy-2-quinoléinyl)méthanone (composé 6) :**

Du 2,2'-méthanediyl-bis(8-hydroxy-2-quinoléinium) dichlorure dihydrate (60 mg; 0,16 mmol) est agité dans 8 mL d'un mélange THF/solution aqueuse saturée de $NaHCO_3$(1/1, v/v) durant 48 heures. 10 mL de $CH_2Cl_2$ et 10 mL d'eau sont alors ajoutés et le mélange réactionnel est transféré dans une ampoule à décanter. La phase organique est récupérée et la phase aqueuse est ensuite extraite avec du $CH_2Cl_2$ (2 x 10 mL). Les phases organiques sont réunies et lavées à l'eau (1 x 10 mL) puis le solvant est évaporé sous vide. Le produit est alors purifié par filtration sur gel de silice avec un mélange $CH_2Cl_2/CH_3OH$ (97/3, v/v) comme solvant. L'évaporation du solvant du filtrat donne **6** sous la forme d'une poudre rouge (42 mg; 0,13 mmol, rendement = 81 %). RMN-$^1$H (250 MHz, $CDCl_3$) δ, ppm: 8,36 (d, $^3J$ (H, H) = 8,5 Hz, 2 H); 8,19 (d, $^3J$ (H, H) = 8,5 Hz, 2 H); 8,04 (s large, 2 H); 7,59 (m, 2 H); 7,43 (dd, $^3J$ (H, H) = 8,5 Hz, $^4J$ (H, H) = 1,0 Hz, 2 H); 7,22 (dd, $^3J$ (H, H) = 7,5 Hz, $^4J$ (H, H) = 1,0 Hz, 2 H). RMN-$^{13}$C (126 MHz, $CDCl_3$) δ, ppm: 192,2 (Cq); 153,1 (Cq); 151,4 (Cq); 137,1 (Cq); 137,0 (CH); 130,4 (CH); 129,5 (Cq); 121,8 (CH); 117,9 (CH); 111,2 (CH). SM (DIC,

$NH_3$) m/z : 317 ($MH^+$). Analyse (%) pour $C_{19}H_{12}N_2O_3 \cdot 0,5\ H_2O$: calculé C 70,15 ; H 4,03 ; N 8,61 ; trouvé C 70,11 ; H 3,83 ; N 8,94. UV/vis [$CH_3OH$/Tris·HCl 20 mM pH = 7,4 ; NaCl 150 mM (1/1, v/v)]: λ nm (ε $M^{-1}$ $cm^{-1}$) = 254 (43 300), 273 (26 100), 310 (9 600, épaulement), 375 (2 300).

**2,2'-(1,2-Ethanediyl)-bis[8-(méthyloxy)quinoléine] (composé 7') :**

Le produit est déjà décrit dans : C. Kitamura et al., J. Chem. Soc., Perkin Trans. 1 2000, 781-785, mais le protocole est différent et a été établi grâce à la synthèse d'un autre produit par T. Garber et al., Inorg. Chem. 1990, 29, 2863-2868. Une solution sous argon de 8-méthoxyquinaldine (5,32 g; 30,75 mmol) dans 47 mL de THF sec est refroidie à -95°C à l'aide d'un mélange méthanol/azote liquide. Une solution 1,5 M de diisopropylamine de lithium THF dans du cyclohexane (20,5 mL; 30,7 mmol) et de THF sec (63 mL) est ajoutée sur 2 heures. La solution est agitée 2 heures de plus à -95°C puis du 1,2-dibromoéthane (5,30 mL; 61,5 mmol) est ajouté. Le mélange est ramené à température ambiante et agité durant 15 heures. De l'eau (32 mL) est alors ajoutée entraînant la formation d'un précipité blanc qui est récupéré par filtration. Le filtrat est concentré sous vide et chromatographié sur une colonne de gel de silice élué avec un gradient de 0 à 90 % d'acétate d'éthyle dans $CHCl_3$ (v/v). Le produit obtenu par chromatographie et le précipité sont réunis et cristallisés à chaud dans du méthanol pour donner du 2,2'-(1,2-éthanediyl)-bis[8-(méthyloxy)quinoléine] sous la forme d'un solide blanc (2,04 g; 5,93 mmol, rendement = 38 %). RMN-$^1H$ (250 MHz, $CDCl_3$) δ, ppm: 8,04 (d, $^3J$ (H, H) = 8;5 Hz, 2 H); 7,40 (d, $^3J$ (H, H) = 8,5 Hz, 2 H); 7,39 (m, 2 H); 7,35 (dd, $^3J$ (H, H) = 8,0 Hz, $^4J$ (H, H) = 1,5 Hz, 2 H); 7,06 (dd, $^3J$ (H, H) = 7,5 Hz, $^4J$ (H, H) = 1,5 Hz, 2 H); 4,10 (s, 6 H); 3,61 (s, 4 H). SM (CDI, $NH_3$): *m/z* (%) = 345 ($MH^+$).

**2,2'-(1,4-Butanediyl)-bis[8-(méthyloxy)quinoléine] (composé 10'):**

Il est obtenu comme principal sous-produit dans le surnageant méthanolique de cristallisation du 2,2'-(1,2-éthanediyl)-bis[8-(méthyloxy)-quinoléine]. Une seconde cristallisation dans le méthanol permet d'obtenir une solution méthanolique pure du produit désiré. Le solvant est alors évaporé pour donner, après séchage sous vide, du 2,2'-(1,4-butanediyl)-bis[8-(méthyloxy)-quinoléine] sous la forme d'une poudre blanche (0,68 g; 1,84 mmol, rendement = 6 %). RMN-$^1H$ (250 MHz, $CDCl_3$) δ, ppm: 8,01 (d, $^3J$ (H, H) = 8,5 Hz, 2 H); 7,40 (d, $^3J$ (H, H) = 8,5 Hz, 2 H); 7,39 (m, 2 H); 7,35 (dd, $^3J$ (H, H) = 8,0 Hz, $^4J$ (H, H) = 1,5 Hz, 2 H); 7,06 (dd, $^3J$ (H, H) = 7,5 Hz, $^4J$ (H, H) = 1,5 Hz, 2 H); 4,01 (s, 6H); 3,10 (m, 4 H); 1,95 (m, 4 H). SM (CDI, $NH_3$): *m/z*= 373 ($MH^+$).

**2,2'-(1,2-Ethanediyl)-bis(8-hydroxyquinoléine) (composé 7):**

Le produit est déjà décrit dans : C. Kitamura et al., J. Chem. Soc., Perkin Trans. 1 2000, 781-785, à partir duquel cette étape de synthèse a été très légèrement modifiée. Il est aussi décrit mais obtenu par une autre voie de synthèse dans : M. Albrecht et al., Synthesis 1999, 10, 1819-1829. Une solution de 2,2'-(1,2-éthanediyl)-bis[8-(méthyloxy) quinoléine] (2,83 g; 8,23 mmol) dans de l'acide bromhydrique à 48 % (150 mL) est chauffée à reflux durant 24 heures. Après refroidissement à température ambiante, le mélange est neutralisé avec une solution aqueuse de soude 3 M entraînant la formation d'un précipité vert. Le produit est extrait au dichlorométhane puis lavé à l'eau et à la saumure. La phase organique est séchée sur $Na_2SO_4$ et le solvant est évaporé sous vide pour donner **7** sous la forme d'une poudre vert pâle (2,53 g; 8,00 mmol, rendement = 97 %). RMN -$^1H$ (250 MHz, $CDCl_3$) δ, ppm: 9,35 (s large, 2 H); 8,20 (d, $^3J$ (H, H) = 8,5 Hz, 2 H); 7,53 (d, $^3J$ (H, H) = 8,5 Hz, 2 H); 7,37 (m, 2 H); 7,32 (dd, $^3J$ (H, H) = 6,5 Hz, $^4J$ (H, H) = 2,0 Hz, 2 H); 7,05 (dd, $^3J$ (H, H) = 6,5 Hz, $^4J$ (H, H) = 2,0 Hz, 2 H); 3.57 (s, 4H). SM (DIC, $NH_3$) m/z: 317 ($MH^+$). Analyse (%) pour $C_{20}H_{16}N_2O_{20}$•0,1 NaBr: calculé C 73,53; H 4,93; N 8,58; trouvé C 73,81; H 4,73; N 8,50. UV/vis [$CH_3OH$/Tris·HCl 20 mM pH = 7,4 ; NaCl 150 mM (1/1, v/v)]: λ, nm (ε $M^{-1}$ $cm^{-1}$) = 204 (71 800), 248 (74 200), 305 (5 200).

**2,2'-(1,4-Butanediyl)-bis(8-hydroxyquinoléine) (composé 10):**

Le protocole a été établi à partir des travaux de C.Kitamura et al., J. Chem. Soc., Perkin Trans 1 2000, 781-785 sur le produit précédent. Du 2,2'-(1,4-butanediyl)-bis[8-(méthyloxy)quinoléine] (50 mg, 0,13 mmol) est chauffé à reflux dans de l'acide bromhydrique à 48 % (2,7 mL) durant 24 heures. Après refroidissement à 4°C à l'aide d'un bain de glace, le mélange réactionnel est basifié jusqu'à pH = 8 avec une solution aqueuse de soude 3 M (5 mL, 15 mmol) et une solution aqueuse saturée de $NaHCO_3$ (2 mL). Le volume est amené à 15 mL avec de l'eau et la phase aqueuse est extraite avec du $CH_2Cl_2$ (3x10 mL). Les phases organiques sont réunies, lavées avec de l'eau (10 mL), puis le solvant est évaporé sous vide. Le produit est alors purifié par chromatographie flash sur gel de silice élué avec un mélange $CH_2Cl_2/CH_3OH/CH_3COOH$ (96/3/1, v/v/v). Les dernières fractions sont réunies, le solvant est évaporé et elles sont redissoutes dans un mélange de 10 mL de $CH_2Cl_2$ et de tampon acétate de sodium (10 mL; 0,1 M, pH = 7,0). La phase organique est récupérée et donne après évaporation du solvant **10** sous la forme d'une poudre blanche (34 mg; 0,10 mmol, rendement = 77 %). RMN-$^1H$ (250 MHz, $CDCl_3$) δ, ppm: 8,04 (d, $^3J$ (H, H) = 8,5 Hz, 2 H); 7,38 (m, 2 H); 7,31-7,25

(m, 4 H); 7,15 (dd, $^{3}J$ (H, H) = 7,5 Hz, $^{4}J$ (H, H) = 1,0 Hz, 2 H); 3,02 (m, 4 H); 1,93 (m, 4 H). SM (DIC, $NH_3$) m/z: 345 ($MH^+$). Analyse (%) pour $C_{22}H_{20}N_2O_2 \cdot 0{,}25\ H_2O$: calculé C 75,73; H 5,92; N 8,35; trouvé C 75,39; H 5,69; N 8,63. UV/vis [$CH_3OH$/Tris·HCl 20 mM pH = 7,4, NaCl 150 mM (1/1, v/v)]: λ nm (ε $M^{-1}$ $cm^{-1}$) = 202 (81 300), 244 (90 800), 303 (6 300).

**2,2'-(1,2-Ethanediyl)-bis(5-chloro-8-hydroxyquinoléine) (composé 8) :**

Le protocole a été établi à partir des travaux de : H. Gershon et al., J. Heterocycl. Chem. 1972, 9, 659-666. A une solution de **7** (300 mg; 0,95 mmol) dans du $H_2SO_4$ à 97 % (9,5 mL) et refroidie sur bain de glace est ajouté du *N*-chlorosuccinimide (253 mg; 1,90 mmol) par petites portions. Le mélange est agité 15 min à 0°C puis 3 heures à température ambiante. Il est ensuite versé sur de la glace pour donner une suspension jaune qui est neutralisée avec une solution aqueuse de soude 3 M. Un précipité vert est alors filtré et séché sous vide avant d'être dissout dans 200 mL de diméthylformamide et précipité deux fois à 4°C avec 200 mL d'eau et filtré pour donner après séchage **8** sous la forme d'un solide vert pâle (258 mg; 0,67 mmol, rendement = 71 %). RMN-$^{1}$H (250 MHz, DMSO-$d_6$) δ, ppm: 9,73 (s large, 2 H); 8,37 (d, $^{3}J$ (H, H) = 8,5 Hz, 2 H); 7,70 (d, $^{3}J$ (H, H) = 8,5 Hz, 2 H); 7,52 (d, $^{3}J$ (H, H) = 8,0 Hz, 2 H); 7,06 (d, $^{3}J$ (H, H) = 8,0 Hz, 2 H); 3,64 (s, 4H). RMN-$^{13}$C (100 MHz, DMSO-$d_6$) δ, ppm: 161,5 (Cq); 153,0 (Cq); 139,2 (Cq); 133,5 (CH); 127,4 (CH); 125,4 (Cq); 124,4 (CH); 119,6 (Cq); 112,1 (CH); 37,1 ($CH_2$). L'observation en RMN de points de corrélation $^{1}$H/$^{13}$C entre le C5 et le H4 a permis d'attribuer la position de l'halogène. SM (DIC, $NH_3$) m/z: 385 ($MH^+$). Analyse (%) pour $C_{20}H_{14}Cl_2N_2O_2 \cdot 0{,}1\ Na_2SO_4$: calculé C 60,14; H 3,53; N 7,01; trouvé C 59,92; H 3,07; N 6,65. UV/vis [dioxane/Tris·HCl 20 mM pH = 7,4 ; NaCl 150 mM (1/1, v/v)]: λ nm (ε $M^{-1}$ $cm^{-1}$) = 254 (75 000), 313 (7 200).

**2,2'-(1,2-Ethanediyl)-bis(5-chloro-7-iodo-8-hydroxyquinoléine) (composé 9) :**

Le protocole a été établi à partir des travaux de : H. Gershon et al., J. Heterocycl. Chem. 1972, 9, 659-666. A une solution de **8** (300 mg; 0,78 mmol) dans 12 mL de méthanol, sous $N_2$, est ajouté 250 μL de $H_2SO_4$ à 97 % pour donner une suspension jaune qui est refroidie sur bain de glace. Du *N*-iodosuccinimide (418 mg; 1,86 mmol) est alors ajouté lentement et le mélange est agité 15 min à 0°C puis 5 jours à température ambiante. Il est ensuite versé sur de la glace et décoloré par addition de $Na_2S_2O_5$ (450 mg). La suspension est neutralisée avec de l'ammoniaque et centrifugée. Le surnageant est enlevé, le précipité est dissout dans du dichlorométhane et lavé avec du tampon Tris·HCl (0,1 M; pH = 7,0). Le solvant de la phase organique est évaporé sous vide pour donner **9** sous la forme d'une poudre jaune (350 mg; 0,55 mmol; rendement = 70 %). RMN-$^{1}$H (250 MHz, DMSO-$d_6$) δ, ppm: 8,36 (d, $^{3}J$ (H, H) = 8,5 Hz, 2 H); 7,90 (s, 2 H); 7,70 (d, $^{3}J$ (H, H) = 8,7 Hz, 2 H); 3,68 (s, 4 H). RMN-$^{13}$C (63 MHz, DMSO-$d_6$) δ, ppm: 162,1 (Cq); 153,1 (Cq); 137,3 (Cq); 134,4 (CH); 133,5 (CH); 124,6 (Cq); 124,5 (CH); 120,2 (Cq); 78,4 (Cq); 37,1 ($CH_2$). SM (DIC, $NH_3$) m/z: 637 ($MH^+$). Analyse (%) pour $C_{20}H_{14}Cl_2I_2N_2O_2$: calculé C 37,71; H 1,90; N 4,40; trouvé C 38,15; H 2,04; N 4,15. UV/vis [DMSO/Tris·HCl 20 mM pH = 7,4 ; NaCl 150 mM (8/2, v/v)]: λ, nm (ε $M^{-1}$ $cm^{-1}$) = 262 (69 500), 319 (7 900).

**2,2',2''-(1,2,3-Propanetriyl)-tris[8-(méthyloxy)quinoléine] (composé 11'):**

A une solution de 8-méthyloxyquinaldine (1,41 g; 8,14 mmol) dans du THF sec (20 mL), sous argon et refroidie sur bain de glace, est ajoutée une solution 1,5 M de diisopropylamine de lithium·THF dans du cyclohexane (20 mL; 30,52 mmol) sur une période d'une minute. La solution est agitée 1 heure à 4°C puis du $CuCl_2$ sec (1,32 g; 9,81 mmol) est ajouté et le mélange est agité 36 heures à température ambiante sous argon. De l'eau (100 mL) est alors ajoutée et le produit est extrait avec du chloroforme, lavé avec de la saumure et le solvant est évaporé. Le produit est alors chromatographié sur une colonne de gel de silice élué avec un gradient de 20 à 100 % d'acétate d'éthyle dans du $CHCl_3$ (v/v). Le produit résultant est dissout dans du dichlorométhane et lavé avec une solution aqueuse d'EDTA pour enlever des traces d'ion cuivrique puis de nouveau chromatographié selon les conditions précédentes pour donner du 2,2', 2''-(1,2,3-propanetriyl)-tris[8-(méthyloxy)quinoléine] sous la forme d'une poudre blanche (150 mg; 0,29 mmol, rendement = 11 %). RMN-$^{1}$H (250 MHz, $CDCl_3$) δ, ppm: 7,81 (d, $^{3}J$ (H, H) = 8,5 Hz, 1 H); 7,75 (d, $^{3}J$ (H, H) = 8,5 Hz, 2 H); 7,37-7,18 (m, 9 H), 7.00 (dd, $^{3}J$ (H, H) = 7,5 Hz, $^{4}J$ (H, H) = 1,5 Hz, 1 H); 6,95 (dd, $^{3}J$ (H, H) = 7,5 Hz, $^{4}J$ (H, H) = 1,5 Hz, 1 H); 4.49 (système ABX, $^{3}J_{AX}$ (H, H) = 8,0 Hz, $^{3}J_{BX}$ (H, H) = 7,0 Hz, 1 H); 4,05 (s, 3 H); 4,01 (s, 6 H); 3,78 (système ABX, $^{2}J_{AB}$ (H, H) = 14,0 Hz, $^{3}J_{Ax}$ (H, H) = 8,0 Hz, 2 H); 3,62 (système ABX, $^{2}J_{AB}$ (H, H) = 14,0 Hz, $^{3}J_{ex}$ (H, H) = 7,0 Hz, 2 H). SM (CDI, $NH_3$): *m*/*z* = 516 ($MH^+$).

**2,2',2''-(1,2,3-Propanetriyl)-tris(8-hydroxyquinoléine) (composé 11):**

Du 2,2',2''-(1,2,3-propanetriyl)-tris[8-(méthyloxy)quinoléine] (30 mg; 0,058 mmol) est chauffé à reflux dans de l'acide bromhydrique à 48 % (1,2 mL) durant 36 heures. L'acide est évaporé sous vide et le résidu est dissout dans un mélange de 10 mL de $CH_2Cl_2$ et de 10 mL de tampon acétate de sodium (0,1 M; pH = 7,0). La phase organique est collectée et la phase aqueuse est ensuite extraite avec du $CH_2Cl_2$ (2 x 10 mL). Les phases organiques sont réunies,

lavées à l'eau (10 mL), puis le solvant est évaporé sous vide pour donner **11** sous la forme d'une poudre orange (20 mg; 0,042 mmol; rendement = 73 %). RMN-$^1$H (250 MHz, $CDCl_3$) δ, ppm: 7,94 (d, $^3J$ (H, H) = 8,0 Hz, 1 H); 7,91 (d, $^3J$ (H, H) = 8,0 Hz, 2 H); 7,42-7,10 (m, 12 H) ; 4,47 (système ABX, $^3J_{AX}$ (H, H) = 8,0 Hz, $^3J_{BX}$ (H, H) = 6,5 Hz, 1 H); 3,71 (système ABX, $^2J_{AB}$ (H, H) = 14,5 Hz, $^3J_{AX}$ (H, H) = 8,0 Hz, 2 H); 3,57 (système ABX, $^2J_{AB}$ (H, H) = 14,5 Hz, $^3J_{BX}$ (H, H) = 6,5 Hz, 2 H). SM (CDI, $NH_3$): m/z = 474 ($MH^+$). Analyse (%) pour $C_{30}H_{23}N_3O_3 \cdot 0{,}5\ H_2O$: calculé C 74,67; H 5,01; N 8,71; trouvé C 74,54; H 5,09; N 9,19. UV/vis [$CH_3OH$/Tris-HCl 20 mM pH = 7,4 ; NaCl 150 mM (1/1, v/v)]: λ nm (ε $M^{-1}$ $cm^{-1}$) = 204 (106 000), 248 (113 000), 302 (9 200).

**Bis(8-quinoléinyl)amine (composé 12):**

Il a été préparé selon J. C. Peters et al., Inorg. Chem. 2001, 40, 5083-5091.
En bref : une suspension sous argon de tris(dibenzylidène-acétone)-dipalladium(0) (4,4 mg, 5 μmol) et de *rac*-2,2'-bis (diphénylphosphino)-1,1',binaphthyl (6,0 mg, 10 μmol) dans 750 μL de toluène est agitée durant 5 min à température ambiante puis du 8-bromoquinoléine (50 mg, 0,24 mmol), du 8-aminoquinoléine (35 mg, 0,24 mmol) et 1,75 mL de toluène sont ajoutés à la suspension. L'ajout de *tertio*-butanolate de sodium (28 mg ; 0,29 mmol) entraîne la formation d'une solution rouge qui est agitée 3 jours à 110°C. Après refroidissement, la solution est filtrée sur silice et extraite au dichlorométhane. La phase organique est concentrée pour donner un solide rouge qui est purifié par chromatographie sur gel de silice élué à l'aide d'un mélange toluène/acétate d'éthyle (4/1, v/v) pour donner **12** sous la forme d'un solide orange (44,0 mg, 0,16 mmol, rendement = 67 %). RMN-$^1$H (250 MHz, $CDCl_3$) δ, ppm: 10,62 (s large, 1 H) ; 8,96 (dd, $^3J$ (H, H) = 4,0 Hz, $^4J$ (H, H) = 1,5 Hz, 2 H) ; 8,16 (dd, $^3J$ (H, H) = 8,5 Hz, $^4J$ (H, H) = 1,5 Hz, 2 H) ; 7,90 (dd, $^3J$ (H, H) = 7,5 Hz, $^4J$ (H, H) = 1,0 Hz, 2 H) ; 7,53 (m, 2 H) ; 7,46 (m, 2 H) ; 7,34 (dd, $^3J$ (H, H) = 6, 5 Hz, $^4J$ (H, H) = 1,0 Hz, 2 H). SM (CDI, $NH_3$): m/z= 272 ($MH^+$). UV/vis [DMSO/Tris·HCl 20 mM pH = 7,4 ; NaCl 150 mM (8/2, v/v)]: λ nm (ε $M^{-1}$ $cm^1$) = 268 (43 700), 341 (5 100), 399 (16 900).

***N,N'*-di-8-quinoléinyl-1,3-propanediamine (composé 13) :**

Une suspension sous argon de tris(dibenzylidèneacétone)dipalladium(0) (55 mg, 0,06 mmol) et de *rac*-2,2'-bis(diphénylphosphino)-1,1',binaphthyl (75 mg, 0,12 mmol) dans 6 mL de toluène est agitée durant 5 min à température ambiante puis du 8-bromoquinoléine (500 mg, 314 μL, 2,40 mmol), du 1,3-diaminopropane (89 mg, 100 μL, 1,20 mmol) et 6 mL de toluène sont ajoutés à la suspension. L'ajout de tertio-butanolate de sodium (323 mg ; 3,36 mmol) entraîne la formation d'une solution rouge qui est agitée 3 jours à 100°C. Après refroidissement, la solution est filtrée sur célite et extraite au dichlorométhane. La phase organique est concentrée pour donner un solide violet qui est purifié par chromatographie sur gel de silice élué à l'aide d'un gradient de 0 à 5 % de $CH_3OH$ dans du $CH_2Cl_2$ (v/v). Après évaporation du solvant, le produit est repris dans du dichlorométhane et précipité à 4°C par ajout de 4 équivalents de HCl (sous forme d'une solution 1 M dans du diéthyléther). Le précipité est récupéré, redissout dans de l'eau, la solution est alcalinisée jusqu'à le pH = 10 à l'aide d'ammoniaque et le produit est extrait au dichlorométhane pour donner, après évaporation du solvant et séchage sous vide, **13** sous la forme d'une poudre marron (355 mg, 1,08 mmol, rendement = 90 %). RMN-$^1$H (250 MHz, $CDCl_3$) δ, ppm: 8,70 (dd, $^3J$ (H, H) = 4,0 Hz, $^4J$ (H, H) = 1,5 Hz, 2 H) ; 8,06 (dd, $^3J$ (H, H) = 8,0 Hz, $^4J$ (H, H) = 1,5 Hz, 2 H) ; 7,37 (m, 4 H) ; 7,05 (dd, $^3J$ (H, H) = 8, 0 Hz, $^4J$ (H, H) = 0,5 Hz, 2 H) ; 6,71 (dd, $^3J$ (H, H) = 8, 0 Hz, $^4J$ (H, H) = 0,5 Hz, 2 H ); 6,24 (t large, $^3J$ (H, H) = 5, 0 Hz, 2 H) ; 3, 55 (m, 4H) ; 2,27 (quintuplet, $^3J$ (H, H) = 7, 0 Hz, 2 H). RMN-$^{13}$C (126 MHz, $CDCl_3$) δ, ppm: 146,8 (CH); 144,8 (Cq); 138,3 (Cq); 136,0 (CH); 128,7 (Cq); 127,8 (CH); 121,4 (CH); 113,8 (CH); 104,7 (CH); 41,3 ($CH_2$); 29,0 ($CH_2$). SM (CDI, $NH_3$): *m/z*=329 ($MH^+$).

***N,N'*-bis(2-méthyl-8-quinolényl)-1,2-éthanediamine (composé 14):**

Une solution sous argon de 8-aminoquinaldine (0,50 g; 3,16 mmol) dans 5 ml de THF sec est refroidie à -90°C à l'aide d'un mélange méthanol/azote liquide. Une solution 1,5 M de diisopropylamine de lithium-THF dans du cyclohexane (4,2 mL; 6,33 mmol) dans 7,5 mL de THF est ajouté sur 30 minutes. La solution est agitée 90 minutes de plus à -95°C puis du 1,2-dibromoéthane (0,55 mL; 6,33 mmol) est ajouté goutte à goutte. Le mélange est ramené à température ambiante et agité durant 15 heures. De l'eau (6 mL) est alors ajoutée et le mélange est agité 90 minutes à température ambiante. Le THF est évaporé sous pression réduite et la phase aqueuse est extraite avec $CH_2Cl_2$ (2 x 75 mL). La phase organique est lavée avec 75 mL d'une solution aqueuse saturée de $NaHCO_3$ puis avec 75 mL d'$H_2O$ et le solvant est évaporé sous pression réduite. Le brut de réaction est alors chromatographié sur une colonne de gel de silice, élué avec un gradient de 0 à 100 % d'acétate d'éthyle dans $CH_2Cl_2$ (v/v). Les fractions contenant le produit sont réunies et le solvant est évaporé. Le produit est redissous dans 1 mL de $CH_2Cl_2$ et précipité par l'ajout de 6 mL d'hexane. Il est alors filtré puis rincé à l'hexane pour donner après séchage sous vide **14** sous la forme d'une poudre blanche (34 mg; 0,10 mmol, rendement = 6 %). RMN-$^1$H (250 MHz, $CDCl_3$) δ, ppm: 7,95 (d, $^3J$ (H, H) = 8,5 Hz, 2 H); 7,33 (m, 2 H); 7,24 (d, $^3J$ (H, H) = 8,5 Hz, 2 H); 7,03 (d, $^3J$ (H, H) = 8,0 Hz, 2 H); 6,78 (d, $^3J$ (H, H) = 7,5 Hz, 2 H); 6,45 (s large, 2 H); 3, 75

(m, 4 H), 2,68 (s, 6 H). RMN-$^{13}C$ (126 MHz, $CDCl_3$) δ, ppm: 155,7; 144,1; 137,5; 136,2; 126,8; 126,7; 122,2; 114,0; 105,0; 42,8; 25,0. SM (DIC, $NH_3$): *m/z* = 343 ($MH^+$). Analyse (%) pour $C_{22}H_{22}N_4 \cdot 0,3CH_2Cl_2$: calculé C 72,80; H 6,19; N 15,23; trouvé C 72,41; H 6,02; N 14,87. UV/vis [$CH_3OH$/Trisλ·HCl 20 mM pH = 7,4; NaCl 150 mM (1/1, v/v)]: λ nm (ε $M^{-1}$ $cm^{-1}$) = 254 (44 200), 341 (6 000).

**8,8'-Oxydiquinoléine (composé 15):**

Le produit est déjà décrit dans : V. M. Dziomko et al., Yakugaku Zasshi 1951, 71, 452-455; mais par une voie de synthèse différente. A une solution sous argon de 8-hydroxyquinoléine (71 mg; 0,49 mmol) dans 2,0 mL de DMF sec sont ajoutés du carbonate de césium (156 mg; 0,48 mmol), 8-bromoquinoléine (32 μL; 50 mg; 0,24 mmol) et $CuCl_2 \cdot 2H_2O$ (4,1 mg ; 0,024 mmol). Le mélange est chauffé à reflux pendant 72 heures. Après refroidissement, 5 mL de $CH_2Cl_2$ et 5 mL d'une solution aqueuse 0,12 M de sel di-sodique d'EDTA sont ajoutés. La phase organique est récupérée puis la phase aqueuse est extraite avec $CH_2Cl_2$ (2 x 5 mL). Les phases organiques sont réunies et lavées une fois avec 5 mL d'EDTA 0,12 M dans $H_2O$ puis à l'eau. Le volume est réduit sous pression réduite puis le brut réactionnel est purifié par chromatographie sur gel de silice, élué avec un gradient de 50 à 100% d'acétate d'éthyle dans du toluène (v/v) puis avec 100 % de $CH_3OH$ et le solvant est évaporé pour récupérer **15,** à partir de la phase méthanolique, sous la forme d'une poudre blanche (13 mg; 0,046 mmol, rendement = 19 %). RMN-$^1H$ (250 MHz, $CDCl_3$) δ, ppm: 8,95 (dd, $^3J$ (H, H) = 4,0 Hz, $^4J$ (H, H) = 1,5 Hz, 2 H); 8,21 (dd, $^3J$ (H, H) = 8,5 Hz, $^4J$ (H, H) = 1,5 Hz, 2 H); 7,61 (dd, $^3J$ (H, H) = 8,0 Hz, $^4J$ (H, H) = 1,0 Hz, 2 H); 7,46 (dd, $^3J$ (H, H) = 4,0 Hz et 8,5 Hz, 2 H); 7,42 (dd, $^3J$ (H, H) = 7,5 Hz et 8,0 Hz, 2 H); 7,14 (dd, $^3J$ (H, H) = 7,5 Hz, $^4J$ (H, H) = 1,0 Hz, 2 H). SM (DIC, $NH_3$): m/z = 273 ($MH^+$). UV/vis [$CH_3OH$/Tris-HCl 20 mM pH = 7,4; NaCl 150 mM (1/1, v/v)]: λ nm (ε $M^{-1}$ $cm^{-1}$) = 232 (53 300), 240 (42 900), 2994 (9 400), 327 (5 800, épaulement).

**7-Méthyloxy-8-hydroxyquinoléine :**

Il a été préparé à l'aide des protocoles de D. Planchenault et al. Tetrahedron 1995, 51, 5823-5830; D. Nobel, J. Chem. Soc., Chem. Commun. 1993, 419-420 et M. Numazawa et al. J. Chem. Soc., Chem. Commun. 1983, 533-534. A une solution de 7-bromo-8-hydroxyquinoléine (2,00 g; 8,93 mmol), dans 125 mL de DMF, est ajoutée une solution de $CH_3ONa$ (30 % en masse) dans du $CH_3OH$ (17 mL, 89,30 mmol). Le mélange est agité pendant 10 min sous argon. $CuCl_2$-2 $H_2O$ (0,46 g; 2,68 mmol) est ajouté et le mélange réactionnel est chauffé à reflux pendant 20 heures. Après refroidissement à température ambiante, de l'eau (100 mL) et de l' EDTA disodium dihydrate (7,83 g; 26,80 mmol) sont ajoutés et le mélange est agité pendant 1 heure. La solution est acidifiée à pH = 4-5 avec $CH_3COOH$ (3 mL) et elle est ensuite doucement basifiée avec une solution aqueuse saturée de $NaHCO_3$ La phase aqueuse est extraite au $CH_2Cl_2$ (3 x 100 mL). Les phases organiques combinées sont séchées sur $Na_2SO_4$ anhydre et concentrées sous pression réduite. Le solide obtenu est purifié par chromatographie sur gel de silice, élué à l'aide d'un gradient de $CH_2Cl_2$/$CH_3OH$/ $CH_3COOH$ (94/4/2, v/v) à $CH_2Cl_2$/$CH_3OH$ (90/10, v/v). Les fractions contenant le produit sont combinées et lavées avec une solution aqueuse saturée de $NaHCO_3$ (3 x 100 mL). La phase organique est séchée sur $Na_2SO_4$ anhydre et concentrée sous pression réduite pour donner 7-méthyloxy-8-hydroxyquinoléine sous la forme d'une poudre blanche (0,65 g; 3,68 mmol, rendement =40 %). RMN-$^1H$ (250 MHz, $CDCl_3$) δ, ppm: 8,77 (dd, $^3J$ (H, H) = 4,0 Hz, $^4J$ (H, H) = 1,5 Hz, 1 H); 8,11 (dd, $^3J$ (H, H) = 8,5 Hz, $^4J$ (H, H) = 1,5 Hz, 1 H); 7,36 (m, 2 H), 7,31 (dd, $^3J$ (H, H) = 8,5 Hz, $^3J$ (H, H) = 4,0 Hz, 1 H), 4,06 (s, 3H). RMN-$^{13}C$ (63 MHz, $CDCl_3$) δ, ppm: 148,6 (CH); 144,0 (Cq); 139,7 (Cq); 138,6 (Cq); 136,0 (CH); 123,5 (Cq); 119,7 (CH); 117,7 (CH); 116,4 (CH); 57,3 ($CH_3$). SM (DIC, $NH_3$): m/z = 176 ($MH^+$). Analyse (%) pour $C_{10}H_9NO_2 \cdot 0,1\ H_2O$: calculé C 67,86; H 5,24; N 7,91; trouvé C 67,82; H 5,11; N 7,95.

**7-(Méthyloxy)-8-[(phénylméthyl)oxy]quinoléine:**

7-Méthyloxy-8-hydroxyquinoléine (1,10 g; 6,29 mmol) et du $K_2CO_3$ (1,30 g; 9,43 mmol) dans 30 mL d'acétonitrile sec sont agités 10 min sous argon. Du chlorure de benzyle (0,87 mL, 7,54 mmol) est alors ajouté et le milieu réactionnel est chauffé au reflux pendant la nuit. Après retour à température ambiante, le précipité est filtré, lavé avec du $CH_2Cl_2$ et le filtrat est concentré sous pression réduite. L'huile obtenue est redissoute dans du $CH_2Cl_2$ (50 mL) et lavée successivement avec une solution aqueuse de NaOH 2 N (4 x 50 mL) et de l'eau (1 x 50 mL). La phase organique est séchée sur $Na_2SO_4$ anhydre et le solvant est évaporé sous pression réduite pour donner 7-(méthyloxy)-8-[(phénylméthyl) oxy]quinoléine sous la forme d'une huile marron (1,28 g; 4,84 mmol, rendement =77 %). RMN-$^1H$ (250 MHz, $CDCl_3$) δ, ppm: 8,94 (dd, $^3J$ (H, H) = 4,0 Hz, $^4J$ (H, H) = 1,5 Hz, 1 H); 8,06 (dd, $^3J$ (H, H) = 8,5 Hz, $^4J$ (H, H) = 1,5 Hz, 1 H); 7,59-7,51 (m, 3 H); 7,36-7,24 (m, 5 H); 5,40 (s, 2 H); 3,92 (s, 3 H). RMN-$^{13}C$ (63 MHz, $CDCl_3$) δ, ppm: 151,3 (Cq); 149,5 (CH); 142,9 (Cq); 141,2 (Cq); 137,4 (Cq); 135,3 (CH); 127,9 (CH); 127,4 (CH); 127,1 (CH); 123,6 (Cq); 122,9 (CH); 118,5 (CH); 114,8 (CH); 75,2 ($CH_2$); 56,1 ($CH_3$). SM (DIC, $NH_3$): m/z = 266 ($MH^+$). Analyse (%) pour $C_{17}H_{15}NO_2 \cdot 0,05\ CHCl_3$: calculé C 75,49; H 5,59; N 5,16; trouvé C 75,74; H 5,40; N 5,33.

**7-(Méthyloxy)-8-[(phénylméthyl)oxy]quinoléine-N-oxyde:**

A du $CH_2Cl_2$ sec (11 mL) à 4°C sous argon sont successivement ajoutés 7-(méthyloxy)-8-[(phénylméthyl)oxy] quinoléine (0,30 g; 1,13 mmol) et de l'acide m-chloroperbenzoïque à 77 % en masse (0,38 g; 1,69 mmol). Après 48 heures d'agitation à température ambiante, du $CH_2Cl_2$ (40 mL) est ajouté. Le milieu réactionnel est lavé avec une solution aqueuse saturée de $NaHCO_3$ (2 x 50 mL) et le solvant est évaporé sous pression réduite. L'huile résultante est purifiée par chromatographie sur gel de silice, élué avec un mélange $CH_2Cl_2/CH_3OH$ (95/5, v/v) pour donner 7-(méthyloxy)-8-[(phénylméthyl)oxy]quinoléine-*N*-oxyde sous la forme d'une huile jaune (0,18 g; 0,64 mmol, rendement = 58 %). RMN-$^1$H (250 MHz, $CDCl_3$) $\delta$, ppm: 8,43 (dd, $^3J$ (H, H) = 6,0 Hz, $^4J$ (H, H) = 1,0 Hz, 1 H); 7,68-7,58 (m, 4 H); 7,41-7,30 (m, 4 H); 7,10 (dd, $^3J$ (H, H) = 8,5 Hz, $^3J$ (H, H) = 6,0 Hz, 1 H); 5,23 (s, 2 H); 3,96 (s, 3 H). RMN-$^{13}$C (63 MHz, $CDCl_3$) $\delta$, ppm: 138,4; 128,9; 128,7; 128,2; 127,8; 127,6; 125,7; 124,9; 123,4; 119,5; 118,8; 116,8; 107,7; 75,2; 57,1. SM (DIC, $NH_3$): m/z = 282 ($MH^+$). Analyse (%) pour $C_{17}H_{15}NO_3$: calculé C 72,58; H 5,37; N 4,98; trouvé C 72,74; H 4,91; N 4,87.

**Diméthyl bis{7-(méthyloxy)8-[phénylméthyl)oxy]-2-quinoléinyl}propanedioate (composé 16'):**

Le protocole a été établi à partir de la synthèse de **1"**. 7-(Méthyloxy)-8-[(phénylméthyl)oxy]quinoléine-N-oxyde (0,72 g; 2,56 mmol) est dissous dans $CH_2Cl_2$ sec (7 mL). Du diméthylmalonate (0,307 mL, 2,69 mmol) et de l'anhydride acétique (1,18 mL) sont alors ajoutés à cette solution et le mélange est agité durant 24 jours à température ambiante à l'abri de la lumière et protégé de l'humidité (grâce à un tube de $CaCl_2$). Du $CH_3OH$ (20 mL) est additionné et, après 15 heures à -20°C, un précipité est éliminé par centrifugation. Le solvant du surnageant est évaporé et le brut réactionnel est redissous dans du $CH_3OH$ (0,5 mL) et précipité par addition d'eau (3 mL). Le surnageant est éliminé et le précipité est repris dans 40 mL de $CH_2Cl_2$ et lavé à l'eau (2 x 20 mL). Après évaporation du solvant, le produit est purifié par chromatographie préparative sur couche mince, élué avec un mélange $CH_2Cl_2/CH_3OH$ (99/1, v/v). Les impuretés résiduelles sont éliminées par cristallisation dans du $CH_3OH$ pour donner diméthyl bis{7-(méthyloxy)8-[phénylméthyl)oxy]-2-quinoléinyl}propanedioate sous la forme de cristaux blancs (12 mg; 0,02 mmol, rendement = 1,5 %). RMN-$^1$H (250 MHz, $CDCl_3$) $\delta$, ppm: 7,93 (d, $^3J$ (H, H) = 8,5 Hz, 2 H); 7,63 (d, $^3J$ (H, H) = 8,5 Hz, 2 H); 7,59 (m, 4H); 7,47 (d, $^3J$ (H, H) = 9,0 Hz, 2 H); 7,40-7,28 (m, 8 H); 5,41 (s, 4H); 3,95 (s, 6 H); 3,84 (s, 6 H). RMN-$^{13}$C (63 MHz, $CDCl_3$) $\delta$, ppm: 169,3 (Cq); 157,5 (Cq); 152,2 (Cq); 142,1 (Cq); 142,1 (Cq); 138,2 (Cq); 135,9 (CH); 128,4 (CH); 128,2 (CH); 127,7 (CH); 123,2 (Cq); 123,0 (CH); 121,1 (CH); 115,9 (CH); 75,8 ($CH_2$); 75,2 (Cq); 57,1 ($CH_3$); 53,0 ($CH_3$). SM (DIC, $NH_3$): m/z = 659 ($MH^+$). La structure de la molécule a été confirmée par analyse par diffraction de rayons X sur des monocristaux obtenus par cristallisation du produit dans du méthanol. La structure du complexe est présentée à la figure 4. Les paramètres de l'analyse cristalline sont les suivants: système cristallin monoclinique ; P 1 21 1 ; a = 10,.2593 (9) Å, b = 10,.8311 (10) Å, c = 15,2192(14) Å, $\alpha$ = 90°, $\beta$ = 97,622(5)°, $\gamma$ = 90°.

**2,2'-(Méthanediyl)-bis(7-méthyloxy-8-hydroxy-2-quinoléinium) dichlorure (composé 16):**

Le protocole a été établi à partir de la synthèse de **1'**. Diméthyl bis{7-(méthyloxy)8-[phénylméthyl)oxy]-2-quinoléinyl}propanedioate (40 mg; 0,06 mmol) est chauffé au reflux dans du HCl aqueux à 37 % (3 mL) pendant 1 heure 30 min. Après refroidissement, le solvant est évaporé sous pression réduite. Le produit obtenu est solubilisé dans du $CH_3OH$ et versé sur 3 mL de diéthyléther. Après centrifugation, le précipité obtenu est lavé avec 3 mL de diéthyléther puis séché sous vide pour donner **16** sous la forme d'une poudre jaune (28 mg; 0,06 mmol, rendement = 98 %). RMN-$^1$H (250 MHz, $CD_3OD$) $\delta$, ppm: 8,97 (d, $^3J$ (H, H) = 7,5 Hz, 2 H), 7,86 (s, 4 H); 7,68 (d, $^3J$ (H, H) = 8,0 Hz, 2 H); 4,16 (s, 6 H). SM (DCI, $NH_3$): m/z = 363 ($MH^+$). UV/vis [$CH_3OH$/Tris·HCl 20 mM pH = 7,4; NaCl 150 mM (1/1, v/v)]: $\lambda$, nm ($\varepsilon$ $M^{-1}$ $cm^{-1}$) = 203 (47 800), 246 (34 100, épaulement), 257 (49 800), 334 (3 500).

**7-Bromo-2-méthyl-8-hydroxyquinoléine:**

Le protocole a été établi à partir des travaux de: D. E. Pearson et al. J. Org. Chem. 1967, 2358-2360. De la *tert*-butylamine (3,23 mL, 30,78 mmol) est agitée sous argon dans 100 mL de toluène pendant 2 heures à température ambiante en présence de tamis moléculaire 4 Å activé (10 g). Après refroidissement à -70°C, du *N*-bromosuccinimide (5,48 g; 30,78 mmol) et 2-méthyl-8-hydroxyquinoléine (5,00 g; 30,78 mmol) sont ajoutés successivement. Le mélange est lentement ramené à température ambiante (environ 4 heures). Le milieu réactionnel est filtré et le tamis moléculaire est lavé avec du diéthyléther (20 mL). Le filtrat est lavé à l'eau (3 x 50 mL), séché sur $Na_2SO_4$ anhydre et la phase organique est concentrée pour donner un solide qui est porté à reflux dans 100 mL d'hexane pendant 1 heure. Après 24 heures à température ambiante, le précipité est filtré et séché sous vide pour donner 7-bromo-2-méthyl-8-hydroxyquinoléine sous la forme d'une poudre blanche (4,85 g; 20,47 mmol, rendement = 67 %). RMN-$^1$H (250 MHz, $CDCl_3$) $\delta$, ppm: 8,00 (d, $^3J$ (H, H) = 8,5 Hz, 1 H); 7,52 (d, $^3J$ (H, H) = 9,0 Hz, 1 H); 7,31 (d, $^3J$ (H, H) = 8,5 Hz, 1 H); 7,16 (d, $^3J$ (H, H) = 9,0 Hz, 1 H); 2,72 (s, 3 H). RMN-$^{13}$C (63 MHz, $CDCl_3$) $\delta$, ppm: 157,9; 149,2; 137,7; 136,2; 130,1; 125,4; 122,9;

118,2; 103,9; 24,8. SM (DIC, $NH_3$): m/z = 238 ($MH^+$). Analyse (%) pour $C_{10}H_8BrNO$: calculé C 50,45; H 3,39; N 5,88; trouvé C 50,02; H 3,37; N 6,12.

**2-Méthyl-7-(méthyloxy)-8-quinoléinol:**

Le protocole a été établi grâce aux travaux de: D. Planchenault et al. Tetrahedron 1995, 51, 5823-5830. A une solution de 7-bromo-2-méthyl-8-hydroxyquinoléine (1,00 g; 4,22 mmol) dans du DMF (60 mL) est ajoutée une solution de $CH_3ONa$ (30 % en masse) dans $CH_3OH$ (8,04 mL; 42,2 mmol). Le mélange est agité pendant 10 min sous argon. $CuCl_2{\cdot}2H_2O$ (0,22 g; 1,27 mmol) est ajouté et le mélange réactionnel est chauffé à reflux pendant 30 heures. Après refroidissement, de l'eau (50 mL) et de l' EDTA disodium dihydrate (10 g; 27 mmol) sont ajoutés et le milieu est agité pendant 1 heure. La solution est légèrement acidifiée avec de l'acide acétique (3 mL, pH = 4-5) et ensuite doucement re-basifiée avec une solution aqueuse saturée de $NaHCO_3$ (3 mL). La phase aqueuse est extraite au $CH_2Cl_2$ (3 x 100 mL). Les phases organiques combinées sont séchées sur $Na_2SO_4$ anhydre et concentrées sous pression réduite. Le solide obtenu est purifié par chromatographie sur gel de silice, élué à l'aide d'un gradient de $CH_2Cl_2/CH_3OH/CH_3COOH$ (94/4/2, v/v) à $CH_2Cl_2/CH_3OH/$ $CH_3COOH$ (94/5/1, v/v) puis à $CH_2Cl_2/CH_{3O}H$ (94/6, v/v). Les fractions contenant le produit sont combinées et lavées avec une solution aqueuse saturée de $NaHCO_3$ (3 x 100 mL). La phase organique est séchée sur $Na_2SO_4$ anhydre et concentrée sous pression réduite pour donner 2-méthyl-7-(méthyloxy)-8-quinoléinol sous la forme d'une poudre blanche (480 mg; 2,54 mmol, rendement = 60 %). RMN-$^1$H (250 MHz, $CDCl_3$) $\delta$, ppm: 7,95 (d, $^3J$ (H, H) = 8,5 Hz, 1 H); 7,26 (s, 2 H); 7,15 (d, $^3J$ (H, H) = 8,5 Hz, 1 H); 4,03 (s, 3H); 2,69 (s, 3H). RMN-$^{13}$C (63 MHz, $CDCl_3$) $\delta$, ppm: 157,6 (Cq); 143,9 (Cq); 139,3 (Cq); 138,0 (Cq); 136,0 (CH); 121,7(Cq); 120,6 (CH); 117,3 (CH); 115,2 (CH); 57,2 ($CH_3$); 25,1 ($CH_3$). SM (DIC, $NH_3$): m/z = 190 ($MH^+$). Analyse (%) pour $C_{11}$ $H_{11}NO_2$: calculé C 69,83; H 5,86; N 7,40; trouvé C 69,40; H 5,85; N 7,47.

**2,2'-(1,2-Ethanediyl)-bis[7-(méthyloxy)-8-quinoléinol] (composé 17):**

Le protocole a été établi à partir de la synthèse de 7'. 2-Méthyl-7-(méthyloxy)-8-quinoléinol (0,50 g; 2,64 mmol) est agitée sous argon dans 10 mL de THF distillé pendant 30 min à température ambiante, en présence de tamis moléculaire 4 Å activé (2,0 g). Après refroidissement à -90°C, une solution de LDA-THF 1,5 M dans le cyclohexane (3,70 mL, 5,56 mmol) est ajoutée goutte à goutte sur une période de 10 min. Le mélange est ensuite ramené lentement à - 50°C, puis refroidi à nouveau à -90°C. Du 1,2-dibromoéthane (0,50 mL; 5,82 mmol) est ajouté et le mélange est ramené à température ambiante en présence du bain froid (4 heures). De l'eau (5 mL) et $CH_3COOH$ (0,50 mL, pH = 4-5) sont ajoutés et le milieu est agité pendant 1 heure. Le tamis moléculaire est éliminé par filtration et est lavé successivement avec une solution aqueuse saturée de $NaHCO_3$ (10 mL), de l'eau (10 mL) et du diéthyléther (40 mL). Le filtrat biphasique est transféré dans une ampoule à décanter et la phase organique collectée. La phase aqueuse est extraite au diéthyléther (3 x 50 mL) et les phases organiques combinées sont séchées sur $Na_2SO_4$ anhydre et concentrées sous pression réduite. Le solide résultant est dissous à chaud dans du $CH_3OH$ (10 mL) et le précipité blanc formé est collecté après 3 jours, donnant 17 pur après séchage sous vide (306 mg; 0,81 mmol, rendement = 62 %). RMN-$^1$H (250 MHz, $CDCl_3$) $\delta$, ppm: 8,00 (d, $^3J$ (H, H) = 8,5 Hz, 2 H); 7,28 (s, 4 H); 7,24 (d, $^3J$ (H, H) = 8,5 Hz, 2 H); 4,04 (s, 6 H); 3,57 (s, 4 H). RMN-$^{13}$C (63 MHz, $CDCl_3$) $\delta$, ppm: 160,1 (Cq); 144,0 (Cq); 139,3 (Cq); 137,9 (Cq); 136,3 (CH); 121,9(Cq); 120,4 (CH); 117,5 (CH); 115,5 (CH); 57,2 ($CH_3$); 37,1 ($CH_2$). SM (DIC, $NH_3$): m/z = 377 ($MH^+$). Analyse (%) pour $C_{22}H_{20}N_2O_4{\cdot}0,2\ H_2O$:calculé C 69,53; H 5,41 ; N 7,37; trouvé C 69,40; H 5,30; N 7,47. UV/vis [$CH_3OH$/Tris·HCl 20 mM pH = 7,4; NaCl 150 mM (1/1, v/v)]: $\lambda$, nm ($\varepsilon$ $M^{-1}$ $cm^{-1}$) = 204 (83 000), 208 (53 200, épaulement), 252 (97 200), 338 (6 600).

**8-Hydroxy-N-(8-hydroxy-2-quinoléinyl)-2-quinoléinecarboxamide (composé 18):**

A une suspension d'acide 8-hydroxyquinoléine-2-carboxylique (50 mg; 0, 26 mmol) dans 5 mL de $CH_2Cl_2$ sec sont ajoutés du 1-hydroxybenzotriazole monohydrate (71 mg; 0,53 mmol), du (benzotriazole-1-yloxy)tris-(diméthylamino) phosphonium hexafluorophosphate (177 mg; 0,40 mmol) et de la triéthylamine (0,037 mL; 0,26 mmol). Après 30 minutes d'agitation à température ambiante, du 2-amino-8-hydroxyquinoléine (85 mg; 0, 53 mmol) et de la triéthylamine (0,037 mL; 0,26 mmol puis, après 5 minutes d'agitation, 0,117 mL; 0,794 mmol) sont ajoutés. Après 18 heures d'agitation à température ambiante 10 mL d'$H_2O$ et 5 mL de $CH_2Cl_2$ sont ajoutés et la phase organique est récupérée par décantation. Le volume est réduit par évaporation sous pression réduite puis le produit est purifié par chromatographie sur gel de silice, élué avec un mélange $CH_2Cl_2/CH_3OH$ (99,5/0,5, v/v) et le solvant est évaporé sous pression réduite. Le produit est dissous dans 30 mL de $CH_2Cl_2$ et 30 mL d'une solution aqueuse saturée de $NaHCO_3$ sont ajoutés. Le produit est extrait 3 fois au $CH_2Cl_2$ puis les phases organiques sont réunies et le solvant est évaporé sous pression réduite pour donner **18** sous la forme d'une poudre blanche (28 mg; 0,085 mmol, rendement = 32 %). RMN-$^1$H (250 MHz, DMSO-d6) $\delta$, ppm: 11,89 (s, 1 H); 10,89 (s, 1 H); 9,55 (s, 1 H); 8,60 (d, $^3J$ (H, H) = 8,5 Hz, 1 H); 8,41 (s, 2 H); 8,32 (d, $^3J$ (H, H) = 8,5 Hz, 1 H); 7,63 (dd, $^3J$ (H, H) = 7,0 et 8,0 Hz, 1 H); 7,53 (dd, $^3J$ (H, H) = 8,0 Hz, $^4J$ (H, H) = 1,0 Hz, 1 H); 7,42

(dd, $^3J$ (H, H) = 8,0 Hz, $^4J$ (H, H) = 2,0 Hz, 1 H); 7,37 (dd, $^3J$ (H, H) = 7,0 et 8,0 Hz, 1 H); 7,23 (dd, $^3J$ (H, H) = 7,0 Hz, $^4J$ (H, H) = 1,0 Hz, 1 H); 7,13 (dd, $^3J$ (H, H) = 7,0 Hz, $^4J$ (H, H) = 2,0 Hz, 1 H). SM (DIC, $NH_3$): m/z = 332 ($MH^+$). UV/vis [DMSO/Tris·HCl 20 mM pH = 7,4; NaCl 150 mM (8/2, v/v)]: λ nm (ε $M^{-1}$ $cm^{-1}$) = 259 (49 100), 332 (11 200, épaulement).

**2-{[8-Hydroxy-2-quinoléinyl)amino]méthyl}-8-quinoléinol (composé 19):**

Une solution de 2-amino-8-hydroxyquinoléine (100 mg; 0,62 mmol) et de 8-hydroxyquinoléine-2-carboxaldéhyde (130 mg; 0,75 mmol) dans 8 mL de 1,2-dichloroéthane est agitée durant 1 heure à température ambiante puis $(CH_3COO)_3BHNa$ (291 mg; 1,29 mmol) est ajouté et l'agitation est poursuivie durant 90 minutes à température ambiante. 50 mL de $CH_2Cl_2$ et 50 mL d'une solution aqueuse saturée de $NaHCO_3$ sont alors ajoutés. La phase organique est récupérée puis la phase aqueuse est extraite avec $CH_2Cl_2$ (2 x 120 mL). Les phases organiques sont réunies et séchées sur $Na_2SO_4$ anhydre puis le solvant est évaporé. Le produit est dissous dans 75 mL de $CH_2Cl_2$ et précipité par addition d'un volume (75 mL) d'hexane. Le surnageant est récupéré par filtration et le solvant est évaporé. Le résidu d'évaporation est purifié par chromatographie sur gel de silice, élué avec un mélange $CH_2Cl_2/CH_3OH$ (0,5 à 1 % de $CH_3OH$; v/v) pour donner après évaporation du solvant **19** sous la forme d'une poudre beige (32 mg; 0,10 mmol, rendement = 16 %). RMN-$^1$H (200 MHz, DMSO-$d_6$) δ, ppm: 9,79 (s, 1 H); 8,55 (s, 1 H); 8,29 (d, $^3J$ (H, H) = 8,5 Hz, 1 H); 8,01 (t, $^3J$ (H, H) = 4,5 Hz, 1 H); 7,93 (d, $^3J$ (H, H) = 9,0 Hz, 1 H); 7,57 (d, $^3J$ (H, H) = 8,5 Hz, 1 H); 7,39 (m, 2 H); 7,08 (m, 4 H); 6, 91 (dd, $^3J$ (H, H) = 8,0 Hz, $^4J$ (H, H) = 1,5 Hz, 1 H); 5,08 (d, $^3J$ (H, H) = 4,5 Hz, 2 H). SM (FAB, MBA): m/z = 318 ($MH^+$).

**2,2'-(Iminodiméthanediyl)di(*N*-boc-8-quinoléinamine) (composé 20'):**

A une solution de 1,1-diméthyl(2-formyl-8-quinoléinyl)carbamate (300 mg; 1,10 mmol) dans 15 mL de 1,2-dichloroéthane, 0,25 mL d'une solution 7M de $NH_3$ (1,75 mmol) dans $CH_3OH$ est ajouté et le mélange est agité 30 minutes à température ambiante. $(CH_3COO)_3BHNa$ (432 mg; 2,04 mmol) est ajouté et l'agitation est poursuivie durant 15 heures à température ambiante puis le solvant est évaporé sous pression réduite. Le produit est purifié par chromatographie sur gel de silice, élué à l'acétate d'éthyle pour donner après évaporation du solvant 2,2'-(iminodiméthanediyl) di(*N*-boc-8-quinoléinamine) sous la forme d'une poudre jaune pâle (150 mg; 0,28 mmol, rendement = 52 %). RMN-$^1$H (200 MHz, $CDCl_3$) δ, ppm: 8,98 (s large, 2 H); 8,41 (d large, $^3J$ (H, H) = 7,0 Hz, 2 H); 8,10 (d, $^3J$ (H, H) = 8,5 Hz, 2 H); 7,53 (d, $^3J$ (H, H) = 8,5 Hz, 2 H); 7,48 (dd, $^3J$ (H, H) = 8,0 et 7,5 Hz, 2 H); 7,40 (dd, $^3J$ (H, H) = 8,0 Hz, $^4J$ (H, H) = 1,5 Hz, 2 H); 4,25 (s, 4 H); 2,21 (s large, 1 H); 1,53 (s, 18H). RMN-$^{13}$C (50 MHz, $CDCl_3$) δ, ppm: 157,7 (Cq); 152,9 (Cq); 137,4 (Cq); 136,8 (CH); 134,8 (Cq); 127,1 (Cq); 126,9 (CH); 120,9 (CH); 120,0 (CH); 114,7 (CH); 80,4 (Cq); 54,9 ($CH_2$); 28,3 ($CH_3$). SM (DIC, $NH_3$): m/z = 530 ($MH^+$). Analyse (%) pour $C_{30}H_{35}N_5O_4$·0,25 $C_4H_sO_2$: calculé C 67,16; H 6,77; N 12,71; trouvé C 67,59; H 7,10; N 12,23.

**2,2'-(Iminodiméthanediyl)di(8-quinoléinamine) (composé 20):**

Une solution de 2,2'-(iminodiméthanediyl)di(*N*-boc-8-quinoléinamine) (100 mg; 0,19 mmol) dans 2,5 mL de $CH_2Cl_2$ et 2,5 mL d'acide trifluoroacétique est agitée durant 45 minutes à température ambiante puis les solvants sont évaporés sous pression réduite. Du diéthyléther (10 mL) est ajouté et le solvant est de nouveau évaporé. Le produit est repris dans 20 mL de $CH_2Cl_2$ et lavé avec une solution aqueuse saturée de $NaHCO_3$ (2 x 40 mL). La phase organique est séchée sur $Na_2SO_4$ anhydre puis le solvant est évaporé sous pression réduite pour donner **20** sous la forme d'une poudre beige (62 mg; 0,19 mmol, rendement quantitatif). RMN-$^1$H (250 MHz, $CDCl_3$) δ, ppm: 8,01 (d, $^3J$ (H, H) = 8,5 Hz, 2 H); 7,40 (d, $^3J$ (H, H) = 8,5 Hz, 2 H); 7,29 (dd, $^3J$ (H, H) = 8,0 et 8.0 Hz, 2 H); 7,13 (dd, $^3J$ (H, H) = 8,0, $^4J$ (H, H) = 1,0 Hz, 2 H); 6,91 (d large, $^3J$ (H, H) = 8,0 Hz, 2 H); 5,01 (s large, 4 H); 4,22 (s, 4 H); 2,60 (s large, 1 H). RMN-$^{13}$C (50 MHz, $CDCl_3$) δ, ppm: 157,0 (Cq); 143,6 (Cq); 137,6 (Cq); 136,4 (CH); 127,7 (Cq); 126,8 (CH); 120,8 (CH); 115,9 (CH); 110,2 (CH); 54,9 ($CH_2$). SM (DIC, $NH_3$): m/z = 330 ($MH^+$).

**2,2',2"-(Nitriiotriméthanediyl)tri(*N*-boc-8-quinoléinamine) (composé 21'):**

A une solution de 1,1-diméthyl(2-formyl-8-quinolinyl)carbamate (50 mg; 0,18 mmol) dans 2,5 mL de 1,2-dichloroéthane, 30 μL d'une solution 7 M de $NH_3$ (0,21 mmol) dans $CH_3OH$ sont ajoutés et le mélange est agité 5 minutes à température ambiante. $(CH_3COO)_3BHNa$ (72 mg; 0,34 mmol) est ajouté et l'agitation est poursuivie durant 15 heures à température ambiante. 10 mL de $CH_2Cl_2$ et 10 mL d'une solution aqueuse saturée de $NaHCO_3$ sont alors ajoutés, la phase organique est récupérée puis la phase aqueuse est extraite avec $CH_2Cl_2$ (2x10 mL). Les phases organiques sont réunies et séchées sur $Na_2SO_4$ anhydre puis le solvant est évaporé sous pression réduite. Le produit est purifié par chromatographie sur gel de silice, élué au $CH_2Cl_2$ pour donner après évaporation du solvant 2,2',2"-(nitrilotriméthanediyl) tri(*N*-boc-8-quinoléinamine) sous la forme d'une poudre jaune pâle (25 mg; 0,032 mmol, rendement = 52 %). RMN-$^1$H (250 MHz, $CDCl_3$) δ, ppm: 9,03 (s, 3H); 8,39 (d large, $^3J$ (H, H) = 7,5 Hz, 3 H); 8,11 (d, $^3J$ (H, H) = 8,5 Hz, 3 H); 7,75

(d, $^3J$ (H, H) = 8,5 Hz, 3 H); 7,47 (dd, $^3J$ (H, H) = 8,0 et 7,5 Hz, 3 H); 7,38 (dd, $^3J$ (H, H) = 8,0 Hz, $^4J$ (H, H) = 1,5 Hz, 3 H); 4,10 (s, 6 H); 1,59 (s, 27 H). RMN-$^{13}$C (63 MHz, $CDCl_3$) δ, ppm: 157,8 (Cq); 152,9 (Cq); 137,3 (Cq); 136,7 (CH); 134,9 (Cq); 127,1 (Cq); 127,0 (CH); 121,4 (CH); 119,9 (CH); 114,6 (CH); 80,5 (Cq); 61,0 ($CH_2$); 28,4 ($CH_3$). SM (DIC, $NH_3$): m/z = 786 ($MH^+$).

**2,2',2"-(Nitrilotriméthanediyl)tri(8-quinoléinamine) (composé 21):**

Une solution de 2,2',2"-(nitrilotriméthanediyl)tri(*N*-boc-8-quinoléinamine) (10 mg; 0,013 mmol) dans 0,5 mL de $CH_2Cl_2$ et 0,5 mL d'acide trifluoroacétique est agitée durant 45 minutes à température ambiante puis les solvants sont évaporés sous pression réduite. Du diéthyléther (5 mL) est ajouté et le solvant est de nouveau évaporé sous pression réduite. Le produit est repris dans 15 mL de $CH_2Cl_2$ et lavé avec une solution aqueuse saturée de $NaHCO_3$ (2x10 mL). La phase organique est séchée sur $Na_2SO_4$ anhydre puis le solvant est évaporé sous pression réduite pour donner **21** sous la forme d'une poudre beige (6 mg; 0,013 mmol, rendement quantitatif). RMN-$^1$H (250 MHz, $CDCl_3$) δ, ppm: 8,05 (d, $^3J$ (H, H) = 8,5 Hz, 3 H); 7,67 (d large, $^3J$ (H, H) = 8,5 Hz, 3 H); 7,29 (dd, $^3J$ (H, H) = 8,0 et 7,0 Hz, 3 H); 7,12 (dd, $^3J$ (H, H) = 8,5 Hz, $^4J$ (H, H) = 1,0 Hz, 3 H); 6,90 (d large, $^3J$ (H, H) = 7,0 Hz, 3 H); 4,99 (s large, 6 H); 4,08 (s large, 6 H). SM (DIC, $NH_3$): m/z = 486 ($MH^+$).

**[2-(Butylamino)méthyl]-*N*-boc-8-quinoléinamine:**

Le protocole est inspiré des travaux de G. Xue et al., Tetrahedron 2001, 57, 7623-7628, sur d'autres produits. A une solution sous argon de 1,1-diméthyl(2-formyl-8-quinolinyl)carbamate (50 mg; 0,18 mmol) dans 2,5 mL de 1,2-dichloroéthane est ajouté de la 1-butylamine (40 μL; 0,40 mmol) et le mélange est agité 30 minutes à température ambiante puis $(CH_3COO)_3BHNa$ (72 mg; 0,34 mmol) est ajouté et l'agitation est poursuivie durant 15 heures à température ambiante. 10 mL de $CH_2Cl_2$ et 10 mL d'une solution aqueuse saturée de $NaHCO_3$ sont alors ajoutés, la phase organique est récupérée puis la phase aqueuse est extraite avec $CH_2Cl_2$ (2x10 mL). Les phases organiques sont réunies et séchées sur $Na_2SO_4$ anhydre puis le solvant est évaporé sous pression réduite pour donner [2-(butylamino)méthyl]-N-boc-8-quinoléinamine sous la forme d'une huile orange (39 mg; 0,12 mmol, rendement = 65 %). RMN-$^1$H (250 MHz, $CDCl_3$) δ, ppm: 9,00 (s large, 1 H); 8,39 (d large, $^3J$ (H, H) = 7,0 Hz, 1 H); 8,08 (d, $^3J$ (H, H) = 8,5 Hz, 1 H); 7,46 (dd, $^3J$ (H, H) = 8,5 et 7,0 Hz, 1 H); 7,41 (dd, $^3J$ (H, H) = 8,5 Hz, $^4J$ (H, H) = 2,0 Hz, 1 H); 7,39 (dd, $^3J$ *(H,* H) = 8,5 Hz, $^4J$ (H, H) = 1,5 Hz, 1 H); 4,10 (s, 1 H); 2,73 (t, $^3J$ (H, H) = 7,5 Hz, 2 H); 2,13 (s large, 1 H); 1,59 (s, 9H); 1,56 (m, 2 H); 1,40 (m, 2 H); 0,94 (t, $^3J$ (H, H) =7,0 Hz, 3H).

**2,2'-[(Butylimino)diméthanediyl]di(*N*-boc-8-quinoléinamine) (composé 22'):**

Une solution de [2-(butylamino)méthyl]-*N*-boc-8-quinoléinamine (39 mg; 0,12 mmol) et de 1,1-diméthyl(2-formyl-8-quinolinyl)carbamate (36 mg; 0,13 mmol) dans 2,5 mL de 1,2-dichloroéthane est agitée durant 30 minutes à température ambiante puis $(CH_3COO)_3BHNa$ (35 mg; 0,16 mmol) est ajouté et l'agitation est poursuivie durant 20 heures à température ambiante. 10 mL de $CH_2Cl_2$ et 10 mL d'une solution aqueuse saturée de $NaHCO_3$ sont alors ajoutés, la phase organique est récupérée puis la phase aqueuse est extraite avec $CH_2Cl_2$ (2x10 mL). Les phases organiques sont réunies et séchées sur $Na_2SO_4$ anhydre puis le solvant est évaporé sous pression réduite pour donner 2,2'-[(butylimino) diméthanediyl]di(N-boc-8-quinoléinamine) sous la forme d'une poudre jaune pâle qui caractérisée par RMN-$^1$H puis introduite dans la réaction suivante sans purification supplémentaire. RMN-$^1$H (250 MHz, $CDCl_3$) δ, ppm: 9,02 (s large, 2 H); 8,38 (d large, $^3J$ (H, H) = 7,5 Hz, 2 H); 8,09 (d, $^3J$ (H, H) = 8,5 Hz, 2 H); 7,72 (d, $^3J$ (H, H) = 8,5 Hz, 2 H); 7,46 (dd, $^3J$ (H, H) = 8,0 et 7,5 Hz, 2 H); 7,38 (dd, $^3J$ (H, H) = 8,0 Hz, $^4J$ (H, H) = 1,5 Hz, 2 H); 3,99 (s, 4 H); 2,62 (t, $^3J$ (H, H) = 7,0 Hz, 2 H); 1,62 (m, 2 H); 1,59 (s, 18 H); 1,34 (m, 2 H); 0,87 (t, $^3J$ (H, H) = 7,5 Hz, 3 H).

**2,2'-[(Butylimino)diméthanediyl]di(8-quinoléinamine) (composé 22):**

2,2'-[(Butylimino)diméthanediyl]di(*N*-boc-8-quinoléinamine) obtenu à l'étape précédente est dissous dans 0,5 mL de $CH_2Cl_2$ et 0,5 mL d'acide trifluoroacétique et agité durant 45 minutes à température ambiante puis les solvants sont évaporés sous pression réduite. Le résidu est dissous dans 5 mL de $CH_2Cl_2$ et lavé avec une solution aqueuse saturée de $NaHCO_3$ (2 x 5 mL) avant d'être séché sur $Na_2SO_4$ anhydre puis le solvant est évaporé sous pression réduite. Le produit est purifié par chromatographie sur gel de silice, élué avec un mélange hexane/acétate d'éthyle (8/2 à 1/1 ; v/v) pour donner après évaporation du solvant **22** sous la forme d'une huile orange (20 mg; 0,05 mmol, rendement = 42 % sur 2 étapes). RMN-$^1$H (250 MHz, $CDCl_3$) δ, ppm: 8,02 (d, $^3J$ (H, H) = 8,5 Hz, 2 H); 7,65 (d, $^3J$ (H, H) = 8,5 Hz, 2 H); 7,28 (dd, $^3J$ (H, H) = 8,5 et 7,5 Hz, 2 H); 7,12 (dd, $^3J$ (H, H) = 8,5 Hz, $^4J$ (H, H) = 1,0 Hz, 2 H); 6,90 (dd, $^3J$ (H, H) = 7,5 Hz, $^4J$ (H, H) = 1,0 Hz, 2 H); 4,98 (s large, 4 H); 3,97 (s, 4 H); 2,60 (t, $^3J$ (H, H) = 7,0 Hz, 2 H); 1,60 (tt, $^3J$ (H, H) = 7,5 et 7,0 Hz, 2 H); 1,32 (qt, $^3J$ (H, H) = 8,0 et 7,5 Hz, 2 H); 0,85 (t, $^3J$ (H, H) = 8,0 Hz, 3H). RMN-$^{13}$C (63 MHz, $CDCl_3$)

δ, ppm: 143,7 (Cq); 137,4 (Cq); 136,2 (CH); 127,7 (Cq); 126,8 (CH); 126,7 (Cq); 121,3 (CH); 115,8 (CH); 110,0 (CH); 60,9 ($CH_2$); 54,3 ($CH_2$); 29,4 ($CH_2$); 20,5 ($CH_2$); 13,9 ($CH_3$). SM (DIC, $NH_3$): m/z = 386 ($MH^+$).

**2-Chloro-8-nitroquinoléine :**

La synthèse a été réalisée selon le protocole de: M. C. Kimber et al., *Aust. J. Chem* **2003**, *56*, 39-44. Des données de RMN plus précises que celles préalablement publiées sont ajoutées ci-après. RMN-$^1$H (250 MHz, $CDCl_3$) d, ppm: 8,21 (d, $^3J$ (H, H) = 8,5 Hz, 1 H); 8,10 (dd, $^3J$ (H, H) = 7,5 Hz, $^4J$ (H, H) = 1,5 Hz, 1 H); 8,05 (dd, $^3J$ (H, H) = 8,0 Hz, $^4J$ (H, H) = 1,5 Hz, 1 H); 7,65 (m, 1 H); 7,55 (d, $^3J$ (H, H) = 8,5 Hz, 1 H). RMN-$^{13}$C (63 MHz, $CDCl_3$) δ, ppm: 153,5 (Cq); 147,1 (Cq); 138,9 (Cq); 138,8 (CH); 131,9 (CH); 127,6 (Cq); 125,8 (CH); 125,0 (CH); 124,5 (CH). SM (DIC, $NH_3$): m/z = 209 ($MH^+$), 226 ($MNH_4^+$), 243 ($MN_2H_7^+$). Analyse (%) pour $C_9H_5N_2O_2Cl$: calculé C 51,82; H 2,42; N 13,43; trouvé C 51,76; H 2,37; N 13,24. La structure de la molécule a été confirmée par analyse par diffraction de rayons X sur des monocristaux obtenus par cristallisation du produit dans du chloroforme deutéré. La structure est présentée Figure 5. Les paramètres de l'analyse cristalline sont les suivants: système cristallin orthorhombique ; P c a 21; a = 18,090 (4) Å, b = 3,7781 (7) Å, c = 12,581 (2) Å, $\alpha$ = 90°, $\beta$ = 90, $\gamma$ = 90°.

***N*-Butyl-8-nitro-2-quinoléinamine :**

2-Chloro-8-nitroquinoléine (0,20 g; 0,96 mmol) est mis en suspension dans 9,5 mL de 1-butylamine et le milieu est chauffé au reflux (78°C) pendant 15 heures. La solution jaune obtenue est ensuite concentrée sous vide. Le brut réactionnel est repris dans un volume minimum de $CH_3OH$ et versé sur 3 mL de diéthyéther. Après centrifugation, des cristaux de chlorure de butylammonium sont éliminés et le surnageant est concentré sous pression réduite pour donner *N*-butyl-8-nitro-2-quinoléinamine sous la forme d'une huile jaune (0,21 g; 0,86 mmol, rendement = 90%). RMN-$^1$H (250 MHz, $CDCl_3$) δ, ppm: 7,83 (dd, $^3J$ (H, H) = 7,5 Hz, $^4J$ (H, H) = 1,5 Hz, 1 H); 7,77 (d, $^3J$ (H, H) = 9,0 Hz, 1 H); 7,69 (dd, $^3J$ (H, H) = 8,0 Hz, $^4J$ (H, H) = 1,5 Hz, 1 H); 7,14 (m, 1 H), 6,67 (d, $^3J$ (H, H) = 9,0 Hz, 1 H); 5,07 (s large, 1 H); 3,47 (m, 2 H); 1,60 (m, 2 H); 1,39 (m, 2 H); 0,93 (t, $^3J$ (H, H) = 7,5 Hz, 3 H). RMN-$^{13}$C (63 MHz, $CDCl_3$) δ, ppm: 157,8 (Cq); 145,6 (Cq); 140,2 (Cq); 136,7 (CH); 131,5 (CH); 124,7 (CH); 124,1 (CH); 119,7 (CH); 113,3 (Cq); 41,3 ($CH_2$); 31,5 ($CH_2$); 20,2 ($CH_2$); 13,8 ($CH_3$). SM (DIC, $NH_3$): m/z = 246 ($MH^+$). Analyse (%) pour $C_{13}H_{15}N_3O_2$: calculé C 63,66; H 6,16; N 17,13; trouvé C 63,33; H 6,21; N 16,64.

***N*-Butyl-2,2'-imino-bis(8-nitroquinoléine) (composé 23'):**

A une suspension violette de *N*-butyl-8-nitro-2-quinoléinamine (0,17 g; 0,69 mmol), de tris(dibenzylidène-acétone)-dipalladium(0) (13 mg; 0,014 mmol) et de rac-2,2'-bis(diphénylphosphino)-1,1'-binaphthyl (17 mg; 0,027 mmol) dans 5 mL de toluène sous argon est ajouté 2-chloro-8-nitroquinoléine (122 mg; 0,59 mmol) et NaO*t*Bu (77,5 mg; 0,81 mmol). Le milieu est chauffé au reflux pendant 3 heures, puis du 2-chloro-8-nitroquinoléine (22 mg; 0,11 mmol) est à nouveau ajouté. Le chauffage est poursuivi pendant 2 heures 30 min puis 10 mL d'une solution saturée de chlorure d'ammonium sont ajoutés. Le produit est extrait avec 3 x 30 mL de $CH_2Cl_2$ et le solvant est évaporé sous pression réduite. Le produit est purifié par chromatographie sur gel de silice, élué avec $CH_2Cl_2$/hexane (80/20, v/v) pour donner *N*-butyl-2,2'-imino-bis(8-nitroquinoléine) sous la forme d'une poudre jaune (0,11 g; 0,26 mmol, rendement = 38 %). RMN-$^1$H (250 MHz, $CDCl_3$) δ, ppm: 8,14 (d, $^3J$ (H, H) = 9,0 Hz, 2 H); 7,99 (dd, $^3J$ (H, H) = 7,5 Hz, $^4J$ (H, H) = 1,5 Hz, 2 H); 7,93 (dd, $^3J$ (H, H) = 8,0 Hz, $^4J$ (H, H) = 1,5 Hz, 2 H); 7,73 (d, $^3J$ (H, H) = 9,0 Hz ,2 H); 7,43 (m, 2 H); 4,45 (t, $^3J$ (H, H) = 7,5 Hz, 2 H); 1,83 (m, 2 H); 1,47 (m, 2 H); 0,98 (t, $^3J$ (H, H) = 7,5 Hz, 3 H). RMN-$^{13}$C (63 MHz, $CDCl_3$) δ, ppm: 156,2 (Cq); 146,4 (Cq); 138,7 (Cq); 137,0 (CH); 131,5 (CH); 126,2 (Cq); 124,2 (CH); 122,8 (CH); 117,3 (CH); 48,9 ($CH_2$); 30,4 ($CH_2$); 20,3 ($CH_2$); 13,9 ($CH_3$). SM (DIC, $NH_3$): m/z = 418 ($MH^+$). Analyse (%) pour $C_{22}H_{19}N_5O_4$: calculé C 63,30; H 4,59; N 16,78; trouvé C 63,18; H 4,49; N 16,39. UV/vis [$CH_3OH$/Tris·HCl 20 mM pH = 7,4; NaCl 150 mM (1/1, v/v)]: $\lambda$ nm ($\varepsilon$ $M^{-1}$ $cm^{-1}$) = 210 (91 400), 226 (53 500, épaulement), 270 (42 100), 294 (26 700, épaulement), 383 (28 400).

**N-Butyl-2,2'-imino-bis(8-quinoléinamine) (composé 23)**

A une solution de *N*-butyl-2,2'-imino-bis(8-nitroquinoléine) (230 mg; 0,55 mmol) dans 35 mL d'acétate d'éthyle est ajouté du palladium sur charbon à 10% en masse (50 mg). Le mélange est placé sous atmosphère de dihydrogène (1 bar) et agité pendant 4 heures à température ambiante. Après élimination du palladium par filtration sur célite, la phase organique est concentrée sous pression réduite. Le brut réactionnel est repris dans le minimum de $CH_2Cl_2$ et précipité par 4 volumes d'hexane. Après filtration, le surnageant est concentré sous pression réduite et le produit est séché sous vide pour donner **23** sous la forme d'une poudre brune (197 mg; 0,55 mmol, rendement quantitatif). RMN-$^1$H (250 MHz, $CDCl_3$) δ, ppm: 7,90 (d, $^3J$ (H, H) = 9,0 Hz, 2 H); 7,32 (d, $^3J$ (H, H) = 9,0 Hz, 2 H); 7,20 (m, 2 H); 7,10 (dd, $^3J$ (H, H) = 8,0 Hz, $^4J$ (H, H) = 1,0 Hz, 2 H); 6,93 (dd, $^3J$ (H, H) = 7,5 Hz, $^4J$ (H, H) = 1,0 Hz, 2 H); 4,60 (s, 4H); 4,48 (t, $^3J$

(H, H) = 7,5 Hz, 2 H); 1,88 (m, 2 H); 1,47 (m, 2 H); 0,97 (t, $^{3}J$ (H, H) = 7,5 Hz, 3 H). RMN-$^{13}$C (63 MHz, $CDCl_3$) δ, ppm: 154,1 (Cq); 142,1 (Cq); 137,2 (CH); 137,0 (Cq); 125,3 (Cq); 125,0 (CH); 116,3 (CH); 116,1 (CH); 111,3 (CH); 48,9 ($CH_2$); 30,6 ($CH_2$); 20,7 ($CH_2$); 14,1 ($CH_3$). SM (DIC, $NH_3$): m/z = 358 ($MH^+$). UV/vis [$CH_3OH$/Tris·HCl 20 mM pH = 7,4; NaCl 150 mM (1/1, v/v)]: λ nm (ε $M^{-1}$ $cm^{-1}$) = 211 (53400, épaulement), 302 (40 300), 367 (20 000), 382 (14 600, épaulement).

**N-8-Quinoléinyl-8-quinoléinecarboxamide (composé 24):**

A une solution d'acide quinoléine-8-carboxylique (50 mg; 0,29 mmol) dissous dans 5 mL de $CH_2Cl_2$ sec sont ajoutés du (benzotriazole-1-yloxy)tris(diméthylamino)phosphonium hexafluorophosphate (192 mg; 0,43 mmol), du 1-hydroxybenzotriazole monohydrate (79 mg; 0,59 mmol), et de la triéthylamine (0,200 mL; 1,44 mmol). Après 30 minutes d'agitation à température ambiante, du 8-aminoquinoléine (84 mg; 0,58 mmol) est ajouté et le mélange est agité 4 heures 30 minutes à température ambiante. De l'eau (10 mL) est ajoutée et le produit est extrait au $CH_2Cl_2$ (3 x 10 mL). Le volume est réduit et le produit est purifié par chromatographie sur gel de silice, élué avec $CH_2Cl_2$/$CH_3OH$ (0 à 2 %, v/v) puis le solvant est évaporé sous pression réduite et les fractions riches en produits sont repurifiées par chromatographie sur gel de silice, élué avec $CH_2Cl_2$/$CH_3OH$ (99,5/0,5; v/v). **24** est obtenu après évaporation du solvant sous la forme d'une poudre blanche (83 mg; 0,28 mmol, rendement = 96 %). RMN-$^{1}$H (250 MHz, $CDCl_3$) δ, ppm: 15,13 (s, 1 H); 9,25 (dd, $^{3}J$ (H, H) = 4,0 Hz, $^{4}J$ (H, H) = 2,0 Hz, 1 H); 9,19 (dd, $^{3}J$ (H, H) = 7,5 Hz, $^{4}J$ (H, H) = 1,5 Hz, 1 H); 9,00 (m, 2 H); 8,32 (dd, $^{3}J$ (H, H) = 8,0 Hz, $^{4}J$ (H, H) = 2,0 Hz, 1 H); 8,19 (dd, $^{3}J$ (H, H) = 8,0 Hz, $^{4}J$ (H, H) = 1,5 Hz, 1 H); 8,01 (dd, $^{3}J$ (H, H) = 8,0 Hz, $^{4}J$ (H, H) = 1,5 Hz, 1 H); 7,74 (dd, $^{3}J$ (H, H) = 7,5 et 8,0 Hz, 1 H); 7,60 (m, 3 H); 7,48 (dd, $^{3}J$ (H, H) = 4,5 et 8,0 Hz, 1 H). RMN-$^{13}$C (63 MHz, $CDCl_3$) δ, ppm: 164,28; 149,48; 148,57; 145,60; 140,12; 137,59; 136,65; 136,18; 134,02; 132,10; 129,85; 128,41; 128,28; 127,50; 126,63; 121,75; 121,34; 121,12; 118,18. SM (DIC, $NH_3$): m/z = 300 ($MH^+$). UV/vis [DMSO/Tris·HCl 20 mM pH = 7,4; NaCl 150 mM (8/2, v/v)]: λ nm (ε $M^{-1}$ $cm^{-1}$) = 261 (13 300, épaulement), 274 (14 800), 294 (12 200).

**N-8-Quinoléinyl-8-quinoléinesulfonamide** (composé 25):

Le produit est déjà décrit dans : V. M. Dziomko et al., *Azotsoderzhashchie Geterotsikly* **1967,** 281-284 ; mais par une voie de synthèse différente. A une solution sous argon de 8-aminoquinoléine (200 mg; 1,39 mmol) et de triéthylamine (0,30 mL; 2,15 mmol) dans 10 mL de $CHCl_3$ sont ajoutés quelques grains de tamis moléculaire activé 4 Ået le mélange est agité doucement durant 1 heure. 8-Quinoléinesulfonyl chlorure (350 mg; 1,54 mmol) est ajouté et le mélange est chauffé à 65°C pendant 15 heures. Après refroidissement, 50 mL de $CHCl_3$ sont ajoutés et le mélange est lavé à l'eau (2 x 50 mL) puis séché sur $Na_2SO_4$ anhydre et le solvant est évaporé sous pression réduite. Le solide est lavé avec 5 mL de $CH_2Cl_2$ pour donner après séchage **25** sous la forme d'une poudre beige (108 mg; 0,32 mmol, rendement = 23 %). RMN-$^{1}$H (250 MHz, DMSO-$d_6$) δ, ppm: 10,44 (s, 1 H); 9,11 (dd, $^{3}J$ (H, H) = 4,5 Hz, $^{4}J$ (H, H) = 1,5 Hz, 1 H); 8,83 (dd, $^{3}J$ (H, H) = 4,0 Hz, $^{4}J$ (H, H) = 1,5 Hz, 1 H); 8,47 (dd, $^{3}J$ (H, H) = 7,5 Hz, $^{4}J$ (H, H) = 1,5 Hz, 1 H); 8,40 (dd, $^{3}J$ (H, H) = 8,5 Hz, $^{4}J$ (H, H) = 1,5 Hz, 1 H); 8,22 (dd, $^{3}J$ (H, H) = 8,0 Hz, $^{4}J$ (H, H) = 1,5 Hz, 1 H); 8,20 (dd, $^{3}J$ (H, H) = 8,0 Hz, $^{4}J$ (H, H) = 1,5 Hz, 1 H); 7,80 (dd, $^{3}J$ (H, H) = 7,5 Hz, $^{4}J$ (H, H) = 1,5 Hz, 1 H); 7,70 (dd, $^{3}J$ (H, H) = 8,0 et 7,5 Hz, 1 H); 7,62 (dd, $^{3}J$ (H, H) = 8,5 et 4,5 Hz, 1 H); 7,51 ((dd, $^{3}J$ (H, H) = 8,5 et 4,0 Hz, 1 H); 7,50 (dd, $^{3}J$ (H, H) = 8,5 Hz, $^{4}J$ (H, H) = 1,0 Hz, 1 H); 7,42 (dd, $^{3}J$ (H, H) = 8,0 et 7,5 Hz, 1 H). RMN-$^{13}$C (63 MHz, DMSO-$d_6$) δ, ppm: 151,5 (CH); 148,9 (CH); 142,1 (Cq); 137,6 (Cq); 136,9 (CH); 136,3 (CH); 134,4 (CH); 134,2 (Cq); 133,7 (Cq); 131,7 (CH); 128,2 (Cq); 127,8 (Cq); 126,6 (CH); 125,5 (CH); 122,6 (CH); 122,3 (CH); 122,1 (CH); 113,8 (CH). SM (DIC, $NH_3$): m/z=336 ($MH^+$). Analyse (%) pour $C_{18}H_{13}N_3O_2S \cdot 0,2\ H_2O$: calculé C 63,78; H 3,98; N 12,40; trouvé C 63,53; H 3,70; N 12,23.

**RESULTATS :**

**Capacité des composés à chélater les métaux:**

***Détermination de la stoechiométrie métal/ligand :***

Les spectres d'absorption UV-visible et les titrations spectrophotométriques UV-visible ont été réalisées en présence de tampon Tris·HCl 20 mM pH = 7,4 ; NaCl 150 mM puisque c'est le solvant utilisé durant les essais de resolubilisation de $A\beta_{1-42}$. Un solvant organique ($CH_3OH$, dioxane ou DMSO) a été ajouté afin d'avoir une bonne solubilité des ligands et des complexes métalliques aux concentrations utilisées dans ces expériences. Les proportions exactes du mélange solvant organique/tampon sont précisées au niveau de chaque caractérisation UV-visible des différents ligands ou complexes métalliques.

A une solution 15 μM de ligand sont ajoutés des aliquots de solutions concentrées de $CuCl_2$ ou de $ZnCl_2$ (M) afin de n'induire que des variations négligeables de volume. Après chaque addition, des changements des spectres

d'absorption sont immédiatement observés et sont stables entre deux additions ce qui est en accord avec un processus rapide de complexation. Les valeurs exactes d'absorption concernant chaque ligand et ses complexes de cuivre(II) ou de zinc(II) sont détaillées par la suite dans la partie expérimentale (dans la partie synthèse pour les ligands et ci-dessous pour les complexes métalliques).

La stoechiométrie des différents ligands (L) pour Cu(II) ou Zn(II) a ainsi été déterminée spectrophotométriquement par titration de l'ion métallique. Un exemple typique, obtenu dans le cas de la titration de $CuCl_2$ par le ligand 3 est présenté sur la Figure 6. Dans cet exemple, pour un rapport M/L augmentant de 0 à 1, la transition $\pi \rightarrow \pi^*$, centrée à 251 nm pour le ligand libre, est déplacée vers une énergie plus basse avec une apparition concomitante d'une bande d'absorption dans la région visible du spectre ($\lambda_{max}$ à 383 nm) qui est probablement due à une transition MLCT. Pour des rapports M/L supérieurs, aucun changement supplémentaire dans les spectres d'absorption UV-visible n'est observé. Ces résultats sont en accord avec une capacité du ligand 3 à former un seul type de complexe de Cu(II) avec une stoechiométrie M/L (1/1).

Des résultats analogues on été obtenus avec d'autres ligands en présence de Cu(II) ou de Zn(II), ils sont repris dans le Tableau 3.

***Préparation des complexes métalliques pour la caractérisation en spectrométrie de masse :***

Les ligands, dissouts dans du méthanol ou du dioxane, ont été métallés en présence d'un équivalent d'ion métallique durant une heure à température ambiante puis le solvant est évaporé. Du $Cu(AcO)_2$ ou du $Zn(AcO)_2$ ont été employés pour les ligands **1** à **11** et du $CuCl_2$ ou du $ZnCl_2$ pour les autres ligands. Des analyses de contrôle, réalisées après redissolution des complexes dans le mélange tampon/solvant organique adéquat, ont montré que les spectres UV-visible des différents complexes ainsi obtenus sont les mêmes que ceux obtenus durant les expériences de titration pour une stoechiométrie identique de ligand et d'ion métallique.

**Cu(II)-1:** SM (DIC, $NH_3$) m/z: 364 (LCu(II) - 1 H), 381 (LCu(II)$NH_4$ - 2 H). UV/vis [dioxane/Tris·HCl 20 mM pH = 7,4 ; NaCl 150 mM (1/1, v/v)]: $\lambda$ nm ($\varepsilon$ $M^{-1}$ $cm^{-1}$) = 256 (41 900), 272 (32 200, épaulement), 304 (8 800), 338 (5 100), 376 (3 100).

**Cu(II)-2:** SM (DIC, $NH_3$) m/z: 434 (LCu(II) - 1 H), 451 (LCu(II)$NH_4$ - 2 H). Analyse (%) pour $C_{19}H_{10}N_2O_2Cl_2Cu$: calculé C 52,73; H 2,33; N 6,47; trouvé C 52,52; H 1,64; N 6,48. UV/vis [dioxane/Tris·HCl 20 mM pH = 7,4 ; NaCl 150 mM (1/1, v/v)]: $\lambda$ nm ($\varepsilon$ $M^{-1}$ $cm^{-1}$) = 256 (38 300), 278 (35 900), 310 (10 300), 342 (7 300), 398 (4 000).

**Cu(II)-3 :** SM (DIC, $NH_3$) m/z: 392 (LCu(II) - 1 H), 409 (LCu(II)$NH_4$ - 2 H). Analyse (%) pour $C_{21}H_{16}N_2O_2Cu \cdot 0,3$ $C_2H_4O_2$: calculé C 63,33; H 4,16; N 6,84; trouvé C 63,30; H 3,50; N 6,82. UV/vis [$CH_3OH$/Tris·HCl 20 mM pH = 7,4 ; NaCl 150 mM (1/1, v/v)]: $\lambda$ nm ($\varepsilon$ $M^{-1}$ $cm^{-1}$) = 202 (70 800), 254 (58 900), 268 (42 400, épaulement), 383 (4 300).

**Cu(II)-4 :** SM (DIC, $NH_3$) m/z: 462 (LCu(II) - 1 H), 479 (LCu(II)$NH_4$ - 2 H). Analyse (%) pour $C_{21}H_{14}N_2O_2Cl_2Cu$: calculé C 54,74; H 3,06; N 6,08; trouvé C 54,50; H 2,84; N 5,92. UV/vis [dioxane/Tris·HCl 20 mM pH = 7,4 ; NaCl 150 mM (1/1, v/v)]: $\lambda$ nm ($\varepsilon$ $M^{-1}$ $cm^{-1}$) = 261 (59 500), 274 (46 900, épaulement), 347 (4 700), 409 (5 800).

**Cu(II)-5:** SM (DIC, $NH_3$) m/z: 400 (LCu(II) - 1 H), 417 (LCu(II)$NH_4$ - 2 H). UV/vis [$CH_3OH$/Tris·HCl 20 mM pH = 7,4 ; NaCl 150 mM (1/1, v/v)]: $\lambda$ nm ($\varepsilon$ $M^{-1}$ $cm^{-1}$) = 205 (64 000), 255 (47 600), 276 (34 400), 415 (2 900).

**Cu(II)-6 :** SM (DIC, $NH_3$) m/z: 378 (LCu(II) - 1 H), 395 (LCu(II)$NH_4$ - 2 H). UV/vis [$CH_3OH$/Tris·HCl 20 mM pH = 7,4 ; NaCl 150 mM (1/1, v/v)]: $\lambda$ nm ($\varepsilon$ $M^{-1}$ $cm^{-1}$) = 245 (41 100), 306 (27 900), 348 (12 100, épaulement), 485 (2 500).

**Cu(II)-7 :** SM (DIC, $NH_3$) m/z: 378 (LCu(II) - 1 H)395 (LCu(II)$NH_4$ - 2 H). Analyse (%) pour $C_{20}H_{14}N_2O_2Cu \cdot 0,3$ $H_2O$: calculé C 62,67; H 3,84; N 7,31; trouvé C 62,68; H 2,91; N 7,21. UV/vis [$CH_3OH$/Tris·HCl 20 mM pH = 7,4 ; NaCl 150 mM (1/1, v/v)]: $\lambda$ nm ($\varepsilon$ $M^{-1}$ $cm^{-1}$) = 202 (57 700), 259 (68 000), 268 (46 200, épaulement), 392 (4 900).

**Cu(II)-8 :** SM (DIC, $NH_3$) m/z: 448 (LCu(II) - 1 H), 465 (LCu(II)$NH_4$ - 2 H). Analyse (%) pour $C_{20}H_{12}N_2O_2Cl_2Cu \cdot 0,3$ $H_2O$: calculé C 53,12; H 2,81; N 6,20; trouvé C 53,09; H 2,37; N 6,08. UV/vis [dioxane/Tris·HCl 20 mM pH = 7,4 ; NaCl 150 mM (1/1, v/v)]: $\lambda$ nm ($\varepsilon$ $M^{-1}$ $cm^{-1}$) = 264 (64 700), 276 (46 700), 413 (5 300).

**Cu(II)-9 :** SM (DIC, $NH_3$) m/z: 700 (LCu(II) - 1 H), 717 (LCu(II)$NH_4$ - 2 H). Anal. pour $C_{20}H_{10}N_2O_2Cl_2I_2Cu \cdot 2$ $(C_2H_4O_2)$: calculé C 35,30; H 1,97; N 3,43; trouvé C 35,46; H 1,20; N 3,83. UV/vis [DMSO/Tris·HCl 20 mM pH = 7,4 ; NaCl 150 mM (8/2, v/v)]: $\lambda$ nm ($\varepsilon$ $M^{-1}$ $cm^{-1}$) = (56 000), 340 (55 000), 392 (6 200), 420 (5 000).

**Cu(II)-10:** SM (DIC, $NH_3$) m/z: 406 (LCu(II) - 1 H), 423 (LCu(II)$NH_4$ - 2 H). UV/vis [$CH_3OH$/Tris·HCl 20 mM pH = 7,4 ; NaCl 150 mM (1/1, v/v)]: $\lambda$ nm ($\varepsilon$ $M^{-1}$ $cm^{-1}$) = 204 (56 000), 260 (71 800), 374 (4 800).

**Cu(II)-12-Cl :** SM (DIC, $NH_3$) m/z: 369 (LCu(II)Cl). UV/vis [DMSO/Tris·HCl 20 mM pH = 7,4 ; NaCl 150 mM (8/2, v/v)]: $\lambda$ nm ($\varepsilon$ $M^{-1}$ $cm^{-1}$) = 287 (40 700), 368 (3 300), 486 (14 100).

**Cu(II)-13:** SM (électrospray, > 0) m/z: 390 (LCu(II) - 1 H). UV/vis [$CH_3OH$/Tris·HCl 20 mM pH = 7,4; NaCl 150 mM (1/1, v/v)]: $\lambda$ nm ($\varepsilon$ $M^{-1}$ $cm^{-1}$) = 230 (55 700); 303 (11 800), 315 (10 400), 360 (1 100, épaulement).

**Cu(II)-14 :** SM (électrospray, > 0) m/z: 405 [LCu(II)].

**Cu(II)-16 :** SM (électrospray, > 0) m/z: 423 (LCu(II) - 2 H). UV/vis [$CH_3OH$/Tris·HCl 20 mM pH = 7,4; NaCl 150 mM (1/1, v/v)]: $\lambda$ nm ($\varepsilon$ $M^{-1}$ $cm^{-1}$) = 254 (34 400), 278 (35 900), 316 (9 700, épaulement), 380 (3 100).

**Cu(II)-17 :** SM (électrospray, > 0) m/z: 438 (LCu(II) - 1 H). UV/vis [$CH_3OH$/Tris·HCl 20 mM pH = 7,4; NaCl 150

mM (1/1, v/v)]: λ nm (ε $M^{-1}$ $cm^{-1}$) = 212 (50 300), 263 (65 300), 275 (47 300, épaulement), 319 (4 500), 421 (5 900).

**Cu(II)-23 :** SM (électrospray, > 0) m/z: 419 (LCu(II) - 1 H). UV/vis [$CH_3OH$/Tris·HCl 20 mM pH = 7,4; NaCl 150 mM (1/1, v/v)]: λ nm (ε $M^{-1}$ $cm^{-1}$) = 211 (65 400); 235 (33 800, épaulement), 275 (37 000), 302 (5 900, épaulement), 329 (17 900), 352 (19 000), 366 (20 900).

**Cu(II)-24 :** SM (électrospray, > 0) m/z: 361 (LCu(II) - 1 H).

**Zn(II)-1 :** SM (DIC, $NH_3$) m/z: 365 (LZn(II) - 1 H), 382 (LZn(II)$NH_4$ - 2 H).

**Zn(II)-2 :** SM (DIC, $NH_3$) m/z: 433 (LZn(II) - 1 H), 454 (LZn(II)$NH_4$ - 2 H).

**Zn(II)-3:** SM (DIC, $NH_3$) m/z: 393 (LZn(II) - 1 H), 410 (LZn(II)$NH_4$ - 2 H). UV/vis [$CH_3OH$/Tris·HCl 20 mM pH = 7,4 ; NaCl 150 mM (1/1, v/v)]: λ nm (ε $M^{-1}$ $cm^{-1}$) = 202 (78 700), 258 (74 100), 268 (46 700), 378 (4 600).

**Zn(II)-4 :** SM (DIC, $NH_3$) m/z: 463 (LZn(II) - 1 H), 482 (LZn(II)$NH_4$ - 2 H). UV/vis [dioxane/Tris·HCl 20 mM pH = 7,4 ; NaCl 150 mM (1/1, v/v)]: λ nm (ε $M^{-1}$ $cm^{-1}$) = 265 (69 200), 275 (45 700), 346 (5 800), 405 (6 100).

**Zn(II)-5 :** SM (DIC, $NH_3$) m/z: 401 (LZn(II) - 1 H),. UV/vis [$CH_3OH$/Tris·HCl 20 mM pH = 7,4 ; NaCl 150 mM (1/1, v/v)]: λ nm (ε $M^{-1}$ $cm^{-1}$) = 205 (43 300), 258 (50 500), 274 (30 900), 404 (3 000).

**Zn(II)-6:** SM (DIC, $NH_3$) m/z: 379 (LZn(II) - 1 H), 396 (LZn(II)$NH_4$ - 2 H). UV/vis [$CH_3OH$/Tris·HCl 20 mM pH = 7,4 ; NaCl 150 mM (1/1, v/v)]: λ nm (ε $M^{-1}$ $cm^{-1}$) = 238 (31 100), 298 (26 200), 346 (10 500, épaulement), 473 (3 000).

**Zn(II)-7 :** SM (DIC, $NH_3$) m/z: 379 (LZn(II) - 1 H), 396 (LZn(II)$NH_4$ - 2 H). UV/vis [$CH_3OH$/Tris·HCl 20 mM pH = 7,4 ; NaCl 150 mM (1/1, v/v)]: λ nm (ε $M^{-1}$ $cm^{-1}$) = 202 (55 500), 258 (63 600), 268 (43 400, épaulement), 374 (4 000).

**Zn(II)-8:** SM (DIC, $NH_3$) m/z: 449 (LZn(II) - 1 H), 466 (LZn(II)$NH_4$ - 2 H). UV/vis [dioxane/Tris·HCl 20 mM pH = 7,4 ; NaCl 150 mM (1/1, v/v)]: λ nm (ε $M^{-1}$ $cm^{-1}$) = 264 (57 800), 272 (43 600, épaulement), 390 (5 400).

**Zn(II)-9 :** SM (ES-SM, < 0) m/z: 735 (LZn(II)HCl). UV/vis [DMSO/Tris·HCl 20 mM pH = 7,4 ; NaCl 150 mM (8/2, v/v)]: λ nm (ε $M^{-1}$ $cm^{-1}$) = 276 (54 200), 340 (5 800), 352 (7 700), 397 (5 100).

**Zn(II)-10 :** SM (DIC, $NH_3$) m/z: 407 (LZn(II) - 1 H), 424 (LZn(II)$NH_4$ - 2 H). UV/vis [$CH_3OH$/Tris·HCl 20 mM pH = 7,4 ; NaCl 150 mM (1/1, v/v)]: λ nm (ε $M^{-1}$ $cm^{-1}$) = 202 (63 600), 260 (65 600), 368 (4 400).

**Zn(II)-12-Cl :** SM (DIC, $NH_3$) m/z: 370 (LZn(II)Cl). UV/vis ($CH_3OH$): λ nm (ε $M^{-1}$ $cm^{-1}$) = 288 (31 800), 299 (27 900), 369 (3 400), 490 (13 200).

**Zn(II)-13 :** UV/vis [$CH_3OH$/Tris·HCl 20 mM pH = 7,4; NaCl 150 mM (1/1, v/v)]: λ nm (ε $M^{-1}$ $cm^{-1}$) = 232 (55 400); 300 (9 600), 314 (9 200), 364 (700, épaulement).

**Zn(II)-14 :** UV/vis [$CH_3OH$/Tris·HCl 20 mM pH = 7,4; NaCl 150 mM (1/1, v/v)]: λ nm (ε $M^{-1}$ $cm^{-1}$) = 236 (43 900), 304 (8 700), 316 (8 000).

**Zn(II)-16:** SM (électrospray, > 0) m/z: 425 (LZn(II) - 1 H). UV/vis [$CH_3OH$/Tris·HCl 20 mM pH = 7,4; NaCl 150 mM (1/1, v/v)]: λ nm (ε $M^{-1}$ $cm^{-1}$) = 262 (44 700), 276 (22 300, épaulement), 308 (5 900), 390 (2 000).

**Zn(II)-17 :** SM (électrospray, > 0) m/z: 439 (LZn(II) - 1 H). UV/vis [$CH_3OH$/Tris·HCl 20 mM pH = 7,4; NaCl 150 mM (1/1, v/v)]: λ nm (ε $M^{-1}$ $cm^{-1}$) = 202 (66 900); 264 (92 500), 276 (47 100, épaulement), 318 (5 500), 398 (6 700).

**Zn(II)-18 :** SM (électrospray, > 0) m/z: 394 (LZn(II) - 1 H). UV/vis [DMSO/Tris·HCl 20 mM pH = 7,4; NaCl 150 mM (8/2, v/v)]: λ nm (ε $M^{-1}$ $cm^{-1}$) = 273 (27 400); 309 (23 800), 332 (22 000); 4307 (3 800).

**Zn(II)-23 :** UV/vis [$CH_3OH$/Tris·HCl 20 mM pH = 7,4; NaCl 150 mM (1/1, v/v)]: λ nm (ε $M^{-1}$ $cm^{-1}$) = 213 (47 000); 234 (24 800), 274 (25 600), 303 (10 900, épaulement), 330 (11 600), 348 (12 300), 363 (13 100).

***Estimation des constantes d'affinité des ligands pour les ions métalliques :***

Des solutions de ligand à étudier $L_s$, de chélateur en compétition $L_c$ et d'ion métallique M dans le rapport 1/1/1 sont analysées par spectrophotométrie UV-visible à 20°C. La concentration de chaque composant est de 15 μM. Les spectres d'absorption UV-visible des espèces $L_s$, $ML_s$, $L_c$ ou $ML_c$ et les analyses spectrophotométriques des compétitions sont réalisées en présence de tampon Tris·HCl 20 mM ; NaCl 150 mM (pH = 7,4) puisque c'est le solvant utilisé durant les essais de resolubilisation de $A\beta_{1-42}$ décrits par la suite. Un solvant organique ($CH_3OH$, dioxane ou DMSO) a été ajouté afin d'avoir une bonne solubilité de tous les ligands et de tous les complexes métalliques aux concentrations utilisées dans ces expériences. Des contrôles ont montré que tous les ligands et tous les complexes métalliques étudiés donnent des résultats en accord avec la loi de Beer-Lambert dans les conditions expérimentales utilisées.

Les chélateurs d'ions métalliques ($L_c$) utilisés durant les expériences de compétition ont été choisis à partir de "The National Institute of Standards and Technology Standard Reference Data base 46" NIST (Critically Selected Stability Constants of Metal Complexes Database, version 4.0, U. S. Department of Commerce) et L. G. Sillen, A. E. Martell *Stability Constants of Metal-Ion complexes,* The Chemical Society London Publication, 1971.

Les chélateurs compétiteurs $L_c$ ont été sélectionnés afin de disposer d'une gamme de constante de stabilité pour l'ion métallique M présentant des intervalles d'à peu près un ou deux log et aussi afin d'avoir la possibilité pour chaque expérience de compétition de quantifier une des espèces $L_s$, $ML_s$, $L_c$ ou $ML_c$ à une longueur d'onde du spectre UV-vis sans contamination des trois autres espèces (Figure 7). Les constantes de stabilité K = [ML]/([M][L]) choisies pour les chélateurs compétiteurs sont données pour 25°C , une force ionique de 0.1-0.2 ν et un rapport molaire ion métallique/chélateur de 1/1 dans les tableaux 1 et 2. Lorsque plus d'un complexe métallique peut se former entre l'ion

métallique et le chélateur, le chélateur de compétition a seulement été sélectionné si le log K de son complexe métallique avec un rapport ion métallique/chélateur de 1/1 est supérieur à ceux de tous les autres complexes possibles.

Le log K d'un chélateur pour un ion métallique varie avec le pH de la solution comme déterminé par les équations suivantes (C. S. Atwood et al., J. Neurochem. 2000, 75, 1219-1233 ; A. Ringblom, Complexation in Analytical Chemistry, 1963, Interscience, New York; G. Schwarznbach et al., Complexometric Titrations, 1969, Meuthuen, New York):

$$\log K_{app} = \log K - \log \alpha \qquad \text{(Eq. 1)}$$

où:

$$\alpha = [H^+]^n/(K_{a1} \times K_{a2} \times K_{a3} \times ... \times K_{an}) + [H^+]^{n-1}/(K_{a1} \times K_{a2} \times K_{a3} \times ... \times K_{an-1}) + ... + [H^+]/K_{a1} + 1 \qquad \text{(Eq. 2)}$$

$K_a = 10^x$, où $x = -pK_a$ du chélateur (les valeurs $K_a$ sont écrites dans un ordre décroissant des valeurs de $pK_a$), et n = no. de la valeur de $pK_a$. Les valeurs de log K app pour les chélateurs compétiteurs utilisés à pH = 7,4 sont présentées dans les Tableaux 1 et 2.

Lorsque $L_s$, $L_c$ et M sont utilisés dans un rapport 1/1/1, et que $L_s$ forme avec l'ion métallique un complexe MLs et $L_c$ forme avec l'ion métallique un complexe $ML_c$

$$L_s + M \underset{}{\overset{K_s}{\rightleftharpoons}} ML_s \qquad K_s = \frac{[ML_s]}{[L_s]\ [M]} \qquad \text{(Eq. 3)}$$

$$L_c + M \underset{}{\overset{K_c}{\rightleftharpoons}} ML_c \qquad K_c = \frac{[ML_c]}{[L_c]\ [M]} \qquad \text{(Eq. 4)}$$

$K_c$ est la valeur de $K_{app}$ pour le chélateur en compétition $L_c$, déterminée avec les équations 1 et 2.

On peut en déduire qu'à l'équilibre :

$$\frac{K_s}{K_c} = \frac{[ML_s]\ [L_c]}{[ML_c]\ [L_s]} \qquad \text{(Eq. 5)}$$

et

$$K_s = K_c \frac{[ML_s]\ [L_c]}{[ML_c]\ [L_s]} \qquad \text{(Eq. 6)}$$

La concentration initiale C est la même pour $L_s$, $L_c$ et M. Si les complexes $ML_s$ et $ML_c$ sont observés en même temps sur les spectres UV-vis pour log $K_c$> 6 on peut considérer que tout l'ion métallique est complexé sous les formes $ML_s$ ou $ML_c$ et qu'à l'équilibre :

$$[ML_s] + [ML_c] = C = [ML_s] + [L_s] = [ML_c] + [L_c]$$

La concentration $[ML_s]$ = x % de C étant mesurée sur les spectres UV-visible, on en déduit les concentrations $[L_s]$ = (1 - x) % de C, $[ML_c]$ = (1 - x) % de C et $[L_c]$ = x % de C et:

$$K_s = K_c \frac{x^2}{(x-1)^2} \qquad \text{(Eq. 7)}$$

Certaines expériences ont également été faites, dans les mêmes solvants, avec $L_s$ et M dans un rapport 1/1 (15 μM de chaque) mais avec différentes stoechiométries de $L_c$. Pour une concentration initiale C = $L_s$ = M, la concentration de ligand compétiteur [Lc] = y % de C qui, à l'équilibre, permet d'obtenir le rapport de concentration $[ML_s]/[L_s]$ = 1 et donc pour laquelle les concentrations $[ML_s]$ = $[L_s]$ = 50 % de C est déduite de graphes comme ceux obtenus sur la Figure 8. Dans ces conditions $[ML_s]$ = $[ML_c]$; $[ML_c]$ + $[L_c]$= Y, y ≥ 50 et:

$$K_s = K_c \frac{y - 50}{50} \qquad \text{(Eq. 8)}$$

Lorsque log $K_s$ ≤ 6, ces équations ne sont pas utilisées car les pourcentages d'ion métallique libre en solution ou chélaté par le solvant ou le tampon ne sont pas négligeables.

Ainsi, dans le cas de **12** en présence de Zn(II), il a seulement été déterminé, dans le solvant étudié, le nombre d'équivalent de $ZnCl_2$ pour que 15 μM de ligand soit entièrement sous forme de complexe de Zn(II) dans le solvant étudié, le ligand étant en compétition avec le tampon Tris et le DMSO pour la complexation de l'ion métallique. Par comparaison avec d'autres chélateurs dans les mêmes conditions : 2, 2'-bipyridine (log $K_{app}$ ≈ 5 ; 250 eq $ZnCl_2$) < **12** (100 eq $ZnCl_2$)< 1,10-phénanthroline (log $K_{app}$ = 6,3 ; 50 eq $ZnCl_2$). D'où une estimation de log $K_s$ ≈ 6.

De la même façon, dans le tampon Tris/$CH_3OH$ (1/1, v/v), le nombre d'équivalent de Zn(II) est compris entre 100 et 200 eq pour **13,** entre 500 et 1000 eq pour **14,** entre 7,5 et 10 eq pour **18** et entre 100 et 200 eq pour **23.**

Les Tableaux 1 et 2 résument les valeurs observées lors de compétitions et le Tableau 3 présente les valeurs des constantes d'affinité qui en ont été déduites.

Le Tableau 4 met en évidence que log $K_s$ varie peu suivant la composition des différents milieux étudiés.

**Tableau 1:** Réaction de compétition entre les ligands ($L_s$), $CuCl_2$ (M) et un ligand compétiteur ($L_c$) dans un rapport 1/1/1 à 15 μM. Pour chaque ligand $L_s$, les solvants employés sont ceux décrits dans le Tableau 3. Le pourcentage de l'espèce $CuL_s$ à l'équilibre est donné. Les valeurs entre parenthèses correspondent au nombre d'équivalents de $L_c$ permettant la formation de 50 % de $CuL_s$ à partir d'un mélange de $L_s$ (15 μM, 1 équivalent) et $CuCl_2$ (1/1, mol/mol). $K_1$ est la constante de stabilité pour l'espèce $ML_c$. $K_2$ est la constante de stabilité pour l'espèce $M(L_c)_2$.

| | EDA | biPy | HIDA | Dien | EDDA | EDTA | Trien | CTDA | Tetren |
|---|---|---|---|---|---|---|---|---|---|
| log $K_1$ | 10,5 | 8,1 | 11,8 | 15,9 | 16,2 | 18,8 | 20,1 | 22,0 | 22,8 |
| log $K_1$ app | 7,8 | 8,1 | 10,5 | 11,8 | 13,9 | 15,9 | 16,0 | 17,1 | 17,9 |
| log $K_2$ | 9,1 | 5,5 | 4,0 | 5,0 | | | | | |
| **3** | 100 | 100 | 100 | - | 100 | 50 (1 eq) | 30 | 15 | 0 |
| **4** | - | - | 100 | - | 90 | 40 (0,65 eq) | - | - | - |
| **5** | - | - | - | - | - | 45 | - | - | - |
| **6** | - | - | - | - | - | 75 | - | - | - |
| **7** | - | - | - | - | - | 55 (1,1 eq) | - | - | - |

(suite)

| | EDA | biPy | HIDA | Dien | EDDA | EDTA | Trien | CTDA | Tetren |
|---|---|---|---|---|---|---|---|---|---|
| **8** | - | - | - | - | - | 40 (0,65 eq) | - | - | - |
| **9** | - | - | - | 100 | - | 60 | 45 (0,75 eq) | - | 0 |
| **10** | - | - | - | - | - | 45 | - | - | - |
| **12** | - | - | - | - | - | 55 | - | - | - |
| **13** | - | - | - | - | 65 | 25 | - | - | - |
| **17** | - | - | - | - | - | 55 | - | - | - |
| **23** | - | - | - | - | - | 30 | - | - | - |

**Tableau 2:** Réaction de compétition entre les ligands ($L_s$), $ZnCl_2$ (M) et un ligand compétiteur ($L_c$) dans un rapport 1/1/1 à 15 μM.

| log $K_1$<br>log $K_1$ app<br>log $K_2$ | Trien<br>11,9<br>7,8 | NTA<br>10, 5<br>8,0<br>3,8 | EGTA<br>12,6<br>9,4 | EDTA<br>16,5<br>13,7 | DTPA<br>18,2<br>13,9 |
|---|---|---|---|---|---|
| **3** | 100 | 100 | 100 | 30 (0,7 eq) | 20 |
| **4** | - | - | - | 20 | - |
| **5** | - | - | - | 55 | - |
| **6** | - | - | - | 75 | - |
| **7** | - | - | | 55 | - |
| **8** | - | - | - | 30 | - |
| **9** | 100 | 100 | - | 65 | - |
| **10** | - | - | - | 45 | - |
| **17** | - | - | - | 35 | - |

Pour chaque ligand $L_s$, les solvants sont ceux décrits dans le Tableau 3. Le pourcentage de l'espèce $ZnL_s$ à l'équilibre est donné. Les valeurs entre parenthèses correspondent au nombre d'équivalents de $L_c$ permettant la formation de 50 % de $ZnL_s$ à partir d'un mélange de $L_s$ (15 μM, 1 équivalent) et $ZnCl_2$ (1/1, mol/mol). $K_1$ est la constante de stabilité pour l'espèce $ML_c$. $K_2$ est la constante de stabilité pour l'espèce $M(L_c)_2$.

**Tableau 3 :** Espèces formées lors des réactions de titration avec $CuCl_2$ ou $ZnCl_2$ et constantes d'affinité des différents ligands pour les ions Cu(II) ou Zn(II) à pH = 7,4.

| Ligand | Complexe observé par titration avec $Cu^{II}$ (stoechiométrie L/Cu(II) observée) | log $KCu^{II}$ | Complexe observé par titration avec $Zen^{II}$ (stoechiométrie L/Zn(II) observée) | Log $KZn^{II}$ | Solvant |
|---|---|---|---|---|---|
| **3** | $LCu^{II}$ (1/1) | 15,9 ± 1 [a] | $LZn^{II}$ (1/1) | 13,3 ± 1 [a] | Tampon/$CH_3OH$ (1/1) |
| **4** | $LCu^{II}$ (1/1) | 15,4 ± 1[a] | $LZn^{II}$ (1/1) | 12,5 ± 1 [b] | Tampon/dioxane [c] |

(suite)

| Ligand | Complexe observé par titration avec $Cu^{II}$ (stoechiométrie L/Cu(II) observée) | log $KCu^{II}$ | Complexe observé par titration avec $Zen^{II}$ (stoechiométrie L/Zn(II) observée) | Log $KZn^{II}$ | Solvant |
|---|---|---|---|---|---|
| **5** | $LCu^{II}$ (1/1) | 15,7 ± 1 [b] | $LZn^{II}$ (1/1) | 13,9 ± 1 [b] | Tampon/$CH_3OH$ (1/1) |
| **6** | $LCu^{II}$ (1/1) | 16,6 ± 1 [b] | $LZn^{II}$ (1/1) | 14,4 ± 1 [b] | Tampon/$CH_3OH$ (1/1) |
| **7** | $LCu^{II}$ (1/1) | 16,0 ± 1 [a] | $LZn^{II}$ (1/1) | 13,9 ± 1 [b] | Tampon/$CH_3OH$ (1/1) |
| **8** | $LCu^{II}$ (1/1) | 15,4 ± 1 [a] | $LZn^{II}$ (1/1) | 13,0 ± 1[b] | Tampon/dioxane[c] |
| **9** | $LCu^{II}$ (1/1) | 15,7 ± 1 [a] | $LZn^{II}$ (1/1) | 14,2 ± 1[b] | Tampon/DMSO (2/8) |
| **10** | $LCu^{II}$ (1/1) | 15,7 ± 1 [b] | $LZn^{II}$ (1/1) | 13,5 ± 1 [b] | Tampon/$CH_3OH$ (1/1) |
| **12** | $LCu^{II}$ (1/1) | 16,0 ± 1 [b] | $LZn^{II}$ (1/1) | ≈ 6 | Tampon/DMSO (2/8) |
| **13** | $LCu^{II}$ (1/1) | 14,7 ± 1 [b] | - | - | Tampon/$CH_3OH$ (1/1) |
| **17** | $LCu^{II}$ (1/1) | 16,0 ± 1[b] | $LZn^{II}$ (1/1) | 13,2 ± 1 [b] | Tampon/$CH_3OH$ (1/1) |
| **23** | $LCu^{II}$ (1/1) | 15,2 ± 1[b] | - | - | Tampon/$CH_3OH$ (1/1) |

a) D'après l'équation 8.
b) D'après l'équation 7.
c) Dioxane/tampon Tris·HCl 20 mM pH = 7,4 ; NaCl 150 mM = 1/1 et 8/2 pour les expériences avec $CuCl_2$ et $ZnCl_2$, respectivement.

**Tableau 4:** Comparaison des réactions de compétition entre le ligand **3** ($L_s$), $CuCl_2$ ou $ZnCl_2$ (M) et le ligand compétiteur EDTA ($L_c$) dans un rapport 1/1/1 à 15 μM dans les différents solvants utilisés pour la détermination des constantes d'affinité apparentes pour les ions métalliques ($K_s$).

| Ion métallique | solvant | % $ML_s$ | $K_s$[a] |
|---|---|---|---|
| Cu(II) | Tampon/$CH_3OH$ (1/1) | 50 | 15,9 |
| | Tampon/dioxane (1/1) | 45 | 15,7 |
| | Tampon/DMSO (2/8) | 55 | 16,1 |
| Zn(II) | Tampon/$CH_3OH$ (1/1) | 30 | 13,0 |
| | Tampon/dioxane (2/8) | 33 | 13,1 |
| | Tampon/DMSO(2/8) | 35 | 13,1 |
| | $CH_3OH$ | 40 | 13,3 |

Le pourcentage de l'espèce $CuL_s$ à l'équilibre est indiqué.

a) D'après l'équation 7.

**Capacité des composés à augmenter la solubilité de protéines impliquées dans les maladies neurodégénératives :**

Dans le cerveau de personnes atteintes par la maladie d'Alzheimer, les plaques amyloïdes contiennent principalement un peptide (Aβ) comportant 39-43 acides aminés. Il est produit par la digestion de la Protéine Précurseur Amyloïde (APP) par les β- et γ-sécrétases. Les peptides $A\beta_{1-40}$ et $A\beta_{1-42}$ sont les plus nombreux. La pathogénèse est liée à leur accumulation et plus particulièrement à celle de $A\beta_{1-42}$ qui est le plus amyloïdogénique et dont la production est amplifiée par les mutations induisant la maladie d'Alzheimer ou par les facteurs de risque de cette maladie (M. P. Mattson, Nature, 2004, 430, 631-639; M. Citron, Nature Rev. Neurosci. 2004, 5, 677-685).

***Réactifs pour les expériences réalisées en présence du peptide amyloïde***

***$A\beta_{1-42}$:***

Avant utilisation, l'eau [qualité MilliQ (Millipore] et toutes les solutions tampons ont été traitées sur résine Chelex-100 (Biorad) (5 mg/mL) et filtrées à travers des filtres 0,2 μm (Whatman) pour enlever d'éventuelles traces d'ions métalliques ou de particules.

$CuCl_2$, $ZnCl_2$ ou $FeCl_3$, de qualité puriss p.a. proviennent de chez Fluka.

Le peptide β-amyloïde $A\beta_{1-42}$ ($A\beta_{1-42}$) a été synthétisé, purifié (jusqu'à une pureté supérieure à 95 %) et caractérisé par analyse sur HPLC et par spectrométrie de masse MALDI-TOF. Les solutions de travail du peptide ont été préparées en solubilisant 1 mg de peptide lyophilisé dans 500 μL d'eau et 500 μL d'une solution aqueuse de NaOH pH = 12,0 ; sous agitation dans un Thermomixer Comfort (Eppendorf). Les préparations de peptide sont ensuite centrifugées 10 min à 9 000 t/min et le surnageant est utilisé comme solution "stock de Aβ". La concentration en peptide du "stock de Aβ" est déterminée immédiatement par test colorimétrique avec un kit Micro BCA Protein Assays (Pierce) à partir de gammes étalon réalisées avec des quantités connues d'albumine de sérum bovin (BSA) puis la solution de peptide $A\beta_{1-42}$ est aliquotée et congelée rapidement dans de l'azote liquide avant d'être conservée à -20°C jusqu'à son utilisation.

Les ligands sont employés sous la forme de chlorhydrates sauf dans le cas de **1** et **16** qui sont utilisés sous sa forme chlorhydrate obtenue en cours de synthèse , respectivement 2,2'-méthanediyl-bis(8-hydroxy-2-quinoléinium) dichlorure dihydrate et 2,2'-(méthanediyl)-bis(7-méthyloxy-8-hydroxy-2-quinoléinium) dichlorure. Les sels sont générés par addition d'un équivalent d'acide chlorhydrique par équivalent de fonction azotée du ligand dissout dans du DMSO. Après évaporation du solvant, ces sels sont dissouts à la concentration désirée avec du DMSO et conservés à -20°C jusqu'à leur utilisation.

***Analyse de la précipitation de $A\beta_{1-42}$ en fonction du rapport***

***Cu(II)/peptide:*** Le protocole a été établi à partir des travaux de : C. S. Atwood et al., J. Neurochem. 2000, 75, 1219-1233. Les concentrations finales sont indiquées. $A\beta_{1-42}$ (5 μM, 500 μL) est mis à agréger dans du tampon Tris·HCl 20 mM ; NaCl 150 mM (pH = 7,4) durant 2 heures à 37°C sous agitation à 1400 t/min en présence de différentes stoechiométries de $CuCl_2$ dissout dans du DMSO (50 μL). Les échantillons (volume final = 550 μL) sont ensuite centrifugés durant 20 min à 9 000 t/min et 500 μL de surnageant sont prélevés. Le tube contenant le culot de précipitation reçoit alors 450 μL de mélange tampon d'expérience /DMSO (91/9, v/v). Puis la concentration en protéine est déterminée sur le surnageant et le culot par Micro BCA Protein Assays (Pierce): chaque échantillon reçoit un volume de révélateur et est incubé durant 1 heure à 60°C sous agitation à 1 400 t/min puis l'absorbance à 562 nm est mesurée. Les quantifications sont réalisées à partir de gammes étalon de BSA. Les quantités de $A\beta_{1-42}$ réellement contenues dans le culot et le surnageant sont obtenues après une correction due à la présence d'une partie résiduelle du surnageant (50/550 μL) dans la fraction contenant le culot. Pour toutes les expériences, le résultat de l'addition du pourcentage de $A\beta_{1-42}$ dans le surnageant avec le pourcentage de $A\beta_{1-42}$ dans le culot donne des valeurs proches de 100 %.

L'ion Cu(II) est connu pour induire le maximum de peptide-Aβ agrégé insoluble (C. S. Atwood et al., J. Biol. Chem. 1998, 273, 12817-12826). Le rapport Cu(II)/peptide induisant le maximum d'agrégation dans les conditions expérimentales mises en oeuvre a été tout d'abord déterminé. La Figure 9 résume les résultats obtenus. Sans addition de Cu(II), 47 % de $A\beta_{1-42}$ est précipité. L'agrégation augmente quand le rapport Cu(II)/A)/$A\beta_{1-42}$ augmente jusqu'à 2,5 Cu(II) par $A\beta_{1-42}$ puis se stabilise (82 % de peptide agrégé).

***Inhibition de la précipitation de A$\beta_{1-42}$ par l'ajout de ligand :***

Le protocole a été établi à partir des travaux de : C. S. Atwood et al., J. Neurochem. 2000, 75, 1219-1233. Les concentrations finales sont indiquées. 500 $\mu$L de A$\beta_{1-42}$ (5 $\mu$M) est mis à agréger dans du tampon Tris·HCl 20 mM ; NaCl 150 mM (pH = 7,4) durant 1 heure à 37°C sous agitation à 1400 t/min en l'absence ou en présence d'ions métalliques : $CuCl_2$, $ZnCl_2$ ou $FeCl_3$ (20 $\mu$M). Puis 50 $\mu$L de ligand à tester (200 $\mu$M) dissout dans du DMSO sont alors ajoutés et les échantillons sont incubés 1 heure de plus à 37°C sous agitation à 1 400 t/min. Les échantillons sans ligand reçoivent aussi 50 $\mu$L de DMSO. Les échantillons (volume final = 550 $\mu$L) sont ensuite centrifugés durant 20 min à 9 000 t/min et 500 $\mu$L de surnageant sont prélevés. Le tube contenant le culot de précipitation reçoit alors 450 $\mu$L de mélange tampon d'expérience/DMSO (91/9, v/v). Puis la concentration en protéine est déterminée sur le surnageant et le culot par Micro BCA Protein Assays (Pierce): chaque échantillon reçoit un volume de révélateur et est incubé durant 1 heure à 60°C sous agitation à 1 400 t/min puis l'absorbance à 562 nm est mesurée. Les quantifications sont réalisées à partir de gammes étalon de BSA. Les quantités de A$\beta_{1-42}$ réellement contenues dans le culot et le surnageant sont obtenues après une correction due à la présence d'une partie résiduelle du surnageant dans la fraction contenant le culot. Dans le cas de **1, 2, 6, 12, 13, 14, 16, 17, 18, 23,** du 8-hydroxyquinaldine et 8-aminoquinoléine, qui présentent une absorption à 562 nm dans les conditions de dosage, des blancs sont réalisés en présence du ligand testé et de l'ion métallique étudié. Pour toutes les expériences, le résultat de l'addition du pourcentage de A$\beta_{1-42}$ dans le surnageant avec le pourcentage de A$\beta_{1-42}$ dans le culot donne des valeurs proches de 100 %.

Les résultats obtenus sont résumés dans le Tableau 5. Ils montrent la capacité des composés étudiés à augmenter la solubilité de A$\beta_{1-42}$ en présence d'ions métalliques et même, en particulier pour **1, 2, 12, 13, 14, 15, 17, 18, 23** et **24,** en absence de ces ions.

Un test supplémentaire (non présenté) a mis en évidence qu'en présence de $CuCl_2$, le taux de précipitation maximal du peptide était atteint au bout d'une heure d'incubation. On peut donc proposer que tout au moins dans le cas de l'agrégation du peptide en présence de $CuCl_2$, les ligands (qui sont rajoutés seulement après 1 heure d'incubation) ne font pas qu'empêcher l'agrégation du peptide mais peuvent probablement agir sur des agrégats préalablement formés.

**Tableau 5:** Analyse de la solubilisation par les différents ligands de A$\beta_{1-42}$ agrégé en absence ou en présence d'ions métalliques.

| | sans métal<br>% Soluble Aβ | $CuCl_2$<br>% Aβ soluble | $ZnCl_2$<br>% Aβ soluble | $FeCl_3$<br>% Aβ soluble |
|---|---|---|---|---|
| Aβ seul | 55 ± 4 | 18 ± 2 | 29 ± 3 | 47 |
| **1** | 63 ± 4 | 60 ± 5 | 63 | 61 |
| **2** | 66 | 45 ± 5 | 57 | 58 |
| **3** | 56 | 47 ± 3 | 59 | 60 |
| **4** | 55 | 30 | 49 | 45 |
| **5** | 60 | 51 ±1 | 50 | 52 |
| **6** | 56 | 58 ± 3 | 54 | 51 |
| **7** | 59 | 35 | 60 | 40 |
| **8** | 59 | 36 | 58 | 41 |
| **9** | 36 | 19 | 47 | 38 |
| **10** | 52 | 42 | 43 | 43 |
| **11** | 48 | 48 | 45 | 43 |
| **12** | 69 | 69 ± 2 | 52 | 59 |
| **13** | 71 ± 2 | 55 ± 3 | 57 | 66 |
| **14** | 79 ± 3 | 45 ± 3 | 57 | 51 |
| **15** | 72 | 35 | 23 | 42 |
| **16** | 53 | 48 ± 3 | 50 | 44 ± 6 |
| **17** | 63 ± 3 | 38 ± 4 | 42 ± 4 | 38 |
| **18** | 66 | 46 ± 3 | 62 ± 5 | 49 |

(suite)

| | sans métal % Soluble Aβ | $CuCl_2$ % Aβ soluble | $ZnCl_2$ % Aβ soluble | $FeCl_3$ % Aβ soluble |
|---|---|---|---|---|
| **23** | 73 | 60 ± 4 | 58 ± 4 | 62 |
| **24** | 64 | 21 | 21 | 38 |
| 8-hydroxyquinoléine | 85 | 70 ± 3 | 88 | 80 |
| 8-hydroxyquinaldine | 69 | 52 | 62 | 51 |
| Clioquinol | 63 | 55 ± 3 | 37 | 51 |
| 8-aminoquinoléine | 66 ± 3 | 40 | 67 | 58 |
| EDTA | 41 | 65 | - | - |

$A\beta_{1-42}$ (5 µM) est incubé 1 heure à 37°C dans du tampon 20 mM Tris·HCl (pH = 7,4), 150 mM NaCl en présence ou en absence de 20 µM de sel métallique ($CuCl_2$, $ZnCl_2$ ou $FeCl_3$) puis 1 heure de plus en présence de 200 µM ligand puis les mélanges réactionnels sont centrifugés. Les quantités de $A\beta_{1-42}$ soluble et précipité sont déterminées dans le surnageant et le précipité, respectivement, à l'aide d'un kit Micro BCA Protein Assays.

Des expériences ont aussi été réalisées pour $A\beta_{1-42}$ (5 µM), $CuCl_2$ (12,5 µM) et le ligand (12,5 µM) afin d'analyser l'effet, sur la réaction d'agrégation, d'une stoechiométrie minimale (1/1) en ligand par rapport à l'ion métallique pour une concentration en cet ion entraînant un maximum de précipitation du peptide $A\beta_{1-42}$. Pour toutes les expériences, le résultat de l'addition du pourcentage de $A\beta_{1-42}$ dans le surnageant avec le pourcentage de $A\beta_{1-42}$ dans le culot donne des valeurs proches de 100 %.

Les résultats obtenus sont résumés sur la Figure 10.

Une stoechiométrie de 2,5 équivalents de Cu(II) par peptide a été choisie car c'est la valeur minimale induisant le maximum d'agrégation dans les conditions expérimentales employées (Figure 9). Pour les expériences contrôles avec des dérivés comportant un seul résidu quinoléine, 5 équivalents de ligand ont été ajoutés car des complexes de type $L_2Cu$ sont classiquement invoqués dans leur cas.

La Figure 10 résume les résultats observés en les comparant à ceux des expériences précédentes. Remarquer que l'activité de **1, 3, 5, 6, 11** et **18** est statistiquement identique si on considère les valeurs avec un écart type ≤ 5 %, quelles que soient les conditions employées, alors que celle de leurs analogues comportant un seul résidu quinoléine (8-hydroxyquinoléine, clioquinol et 8-hydroxyquinaldine) diminue quand l'excès par rapport au peptide ou à Cu(II) diminue. Pour ces deux conditions expérimentales, les valeurs sont également voisines pour **13** et **14** Dans ces deux conditions expérimentales, **23** est également plus actif que son analogue comportant un seul résidu quinoléine (8-aminoquinoléine).

**Capacité des composés à diminuer le stress oxydatif :**

Il a été proposé que les interactions de Aβ avec les ions Fe et Cu peuvent contribuer à la création des lésions observées dans les cerveaux atteints de la maladie d'Alzheimer. Les peptides Aβ synthétiques exercent une toxicité qui corrèle avec une production de peroxyde d'hydrogène ($H_2O_2$) à partir de $O_2$ par l'intermédiaire d'un mécanisme d'oxydoréduction au niveau de l'ion métallique (M. P. Mattson, Nature 2004, 430, 631-639).

***Dosage du peroxyde d'hydrogène produit par*** $A\beta_{1-42}$ ***en présence de*** $CuCl_2$***, de réducteur, d'air et de ligand:*** Le protocole a été établi à partir des travaux de : X. Huang et al., J. Biol. Chem. 1999, 274, 37111-37116 ; X. Huang et al., Biochemistry 1999, 38, 7609-7616 ; C. Opazo et al., J. Biol. Chem. 2002, 277, 40302-40308; K. J. Barnham et al., J. Biol. Chem. 2003, 278, 42959-42965; G. D. Ciccotosto et al., J. Biol. Chem. 2004, 279, 42528-42534.

Les concentrations finales sont indiquées. Les réactions ont été réalisées dans le noir dans du tampon phosphate de sodium, 50 mM (pH = 7,4). $A\beta_{1-42}$ (0,2 µM) et $CuCl_2$ (0,4 µM) sont préincubés durant 1 heure à 37°C sous agitation à 1 400 t/min dans 375 µL de tampon. Puis 2 µL d'une solution de ligand à étudier (0,2; 0,4 ou 0,8 µM) dans du DMSO sont alors ajoutés. Après incubation durant une heure supplémentaire, 2 µL d'une solution aqueuse d'ascorbate de sodium (10 µM) sont ajoutés et l'incubation est poursuivie durant 5 min toujours sous agitation. La quantité de $H_2O_2$ produite est alors quantifiée à l'aide d'un kit Amplex Red $H_2O_2$/HRP Assay Kit (Molecular Probes) en ajoutant un volume de réactif de dosage sur les échantillons puis en les incubant durant 1 heure à 37°C sous agitation à 500 t/min. Les détections de $H_2O_2$ sont alors réalisées spectrophotométriquement à 563 nm. Les quantifications sont réalisées à partir de courbes étalons obtenues avec de quantités connues de $H_2O_2$. Les résultats obtenus sont résumés dans le Tableau 6 et sur la Figure 11.

Des expériences ont aussi été réalisées en présence d'une quantité connue de $H_2O_2$, ajoutée juste avant ou

juste après l'addition d'ascorbate de sodium, afin d'étudier la stabilité de $H_2O_2$ dans les conditions expérimentales utilisées. Les résultats obtenus sont résumés dans le Tableau 7.

D'autres expériences ont été réalisées en présence de différentes concentrations de peptide $A\beta_{1-42}$ (0,2 à 0,53 μM) et $CuCl_2$ (0,4 μM), avec ou sans addition de ligand (0,4 μM) et de quantités connues de $H_2O_2$, afin d'étudier l'influence de la stoechiométrie $A\beta_{1-42}$/Cu sur la production de $H_2O_2$ par les systèmes étudiés. Les résultats obtenus sont résumés sur la Figure 11.

Le peptide Aβ peut chélater différentes stoechiométries de Cu(II) et ainsi former différents types de complexes. Toutes ces espèces peuvent avoir différentes capacités à produire $H_2O_2$ en présence de dioxygène et de réducteur. La titration de la quantité de $H_2O_2$ produite par 0,4 μM de Cu(II) en fonction de la concentration de $A\beta_{1-42}$ fait apparaître ce phénomène (Figure 11). La quantité de $H_2O_2$ produite augmente quand le rapport Cu/$A\beta_{1-42}$ augmente. Les mêmes expériences ont été réalisées en présence de 1 équivalent de ligand 3 ou 7 par Cu(II). Les deux composées inhibent pratiquement toute la production de $H_2O_2$ quel que soit le rapport Cu/ $A\beta_{1-42}$ testé.

Le Tableau 6 montre que les différents dérivés de quinoléine testés inhibent la production de $H_2O_2$ quelles que soient les conditions expérimentales utilisées.

**Tableau 6:** Comparaison de l'inhibition par les différents ligands (0,4 μM) de la production de $H_2O_2$ réalisée par $A\beta_{1-42}$ (0,2 et 0,5 μM) en présence de $CuCl_2$ (0,4 μM), d' ascorbate (10 μM) et d'air.

| Ligand 1 eq/Cu(II) (quand non précisé) | sans $A\beta_{1-42}$ | 0,2 μM $A\beta_{1-42}$ Cu/Aβ = 2 | 0,5 μM $A\beta_{1-42}$ Cu/Aβ = 0,75 |
|---|---|---|---|
| sans ligand | 2,91 ± 0,22 | 2,83 ± 0,19 | 1,08 |
| **1** | 0,54 | 0,75 ± 0,11 | 0,76 |
| **2** | 0,56 | 0,80 ± 0,23 | 0,61 |
| **3** 0,5 eq/Cu(II)<br>1 eq/Cu(II)<br>1,5 eq/Cu(II) | 2,12<br>0,84<br>- | 0,94<br>0,71 ± 0.02<br>0,49 | -<br>0,55<br>- |
| **4** | 0,12 | 0,37 ± 0,09 | 0,35 |
| **5** | 0,39 | 0,55 ± 0,08 | - |
| **6** | 0,82 | 0,86 ± 0,11 | - |
| **7** | 0,75 | 0,89 ± 0,07 | 0,68 |
| **8** | 0,66 | 0,89 ± 0,09 | 0,64 |
| **9** | 0,66 | 0,82 ± 0,08 | 0,82 |
| **12** | 0,40 | 0,47 ± 0,09 | - |
| **13** | 0,89 | 2,10 | 0,70 |
| **14** | 0,74 | 1,99 | 0,78 |
| **15** | - | 1,02 | - |
| **16** | - | 0,70 | - |
| **17** | - | 0,69 | - |
| **18** | 0,47 | 0,47 | 0,45 |
| **23** | 0,65 | 0,69 | 0,49 |
| **24** | 0,49 | 0,62 | 0,47 |
| 8-hydroxyquinoléine<br>1 eq/Cu(II)<br>2 eq/Cu(II) | <br>2,02<br>0,62 | <br>0,69<br>0,71 ± 0,05 | <br>0,75<br>0,62 |
| Clioquinol<br>1 eq/Cu(II)<br>2 eq/Cu(II) | <br>2,07<br>0,54 | <br>0,89 ± 0,08<br>0.78 ± 0,06 | <br>0,64<br>0,57 |
| sans ligand et sans Cu(II) | 0,49 ± 0,19 | 0,69 | 0,51 |

(suite)

| Ligand 1 eq/Cu(II) (quand non précisé) | sans $A\beta_{1-42}$ | 0,2 µM $A\beta_{1-42}$ Cu/Aβ = 2 | 0,5 µM $A\beta_{1-42}$ Cu/Aβ = 0,75 |
|---|---|---|---|
| sans ligand, sans ascorbate | 0 | 0,06 | - |
| et sans Cu(II) | | | |

Les chiffres de tableau correspondent à la quantité d'$H_2O_2$ (exprimé en nanomoles) dosée à l'aide d'un kit Amplex Red $H_2O_2$/HRP Assay.

Dans une expérience contrôle faite avec uniquement le ligand sans peptide amyloïde $\beta_{1-42}$ et sans Cu, la détermination de la production de $H_2O_2$ due à des traces métalliques présentes dans le milieu d'étude est toujours inférieure à 0,49 ± 19 nanomoles d'$H_2O_2$.

Le Tableau 7 montre que, dans les conditions expérimentales utilisées, les ligands peuvent être divisés en deux catégories : (i) ceux qui inhibent la production de $H_2O_2$ mais ne le dégradent pas (toute la quantité de $H_2O_2$ ajoutée est retrouvée) ; (ii) ceux pour lesquels la diminution de $H_2O_2$ est associée à sa dégradation (comme dans le cas de **12** où toute la quantité de $H_2O_2$ ajoutée n'est pas retrouvée).

**Tableau 7:** Analyse de la stabilité de $H_2O_2$ au cours des expériences d'inhibition par les ligands de la production de $H_2O_2$ réalisée par $A\beta_{1-42}$ (0,2 ou 0,4 µM) en présence de $CuCl_2$ (0,4 µM), d'ascorbate (10 µM) et d'air.

| $A\beta_{1-42}$ 0.2 µM[a] 0,075 nanomole | Cu(II) 0.4 µM 0,15 nanomole | ascorbate 10 µM 3,8 nanomoles | Ligand 0,4 µM[a] 0,15 nanomole | $H_2O_2$ ajouté nanomoles | $H_2O_2$ dosé nanomoles |
|---|---|---|---|---|---|
| - | - | + | - | - | 0,49 ± 0,19 |
| - | - | + | - | 3,0 | 3,2 (3,5) |
| - | + | + | - | - | 2,91 ± 0.22 |
| - | + | + | - | 1,5 | 4,0 (4.4) |
| - | + | + | - | 1,5 [b] | 3,6(4,4) |
| + | + | + | - | - | 2,83 ± 0,19 |
| + | + | + | - | 1,5 | 4,2 (4,3) |
| + | + | + | - | 1,5 [b] | 4,2 (4,3) |
| 0,4 µM | + | + | - | - | 1,6 |
| 0,4 µM | + | + | - | 1,5 | 3,0 (3,1) |
| 0,4 µM | + | + | - | 1,5 [b] | 3,0 (3,1) |
| - | + | - | **3** | 3,0 | 3,1 |
| - | + | + | **3** | - | 0,84 |
| - | + | + | **3** | 3,0 | 3,8 (3,8) |
| + | + | + | **3** | - | 0,71 ± 0,02 |
| + | + | + | **3** | 3,0 | 3,7 (3,7) |
| + | + | + | **3** | 3,0 [b] | 3,8 (3,7) |
| + | + | + | **4** | - | 0,37 ± 0,09 |
| + | + | + | **4** | 3,0 | 3,3 (3,4) |
| + | + | + | **5** | - | 0,55 ± 0,08 |
| + | + | + | **5** | 3,0 | 3,4 (3,5) |
| + | + | + | **6** | - | 0,87 ± 0,11 |
| + | + | + | **6** | 3,0 | 3,6 (3,9) |
| + | + | + | **12** | - | 0,47 ± 0,09 |
| + | + | + | **12** | 3,0 | 1,7 (3,4) |
| + | + | + | **14** | - | 1,99 ± 0,11 |

(suite)

| $A\beta_{1-42}$ 0.2 μM[a] 0,075 nanomole | Cu(II) 0.4 μM 0,15 nanomole | ascorbate 10 μM 3,8 nanomoles | Ligand 0,4 μM[a] 0,15 nanomole | $H_2O_2$ ajouté nanomoles | $H_2O_2$ dosé nanomoles |
|---|---|---|---|---|---|
| + | + | + | **14** | 3,0 [b] | 4,9 (5,0) |
| - | + | + | **18** | - | 0,47 |
| - | + | + | **18** | 3,0 [b] | 3,5 (3,5) |

L'analyse est obtenue en comparant les résultats obtenus avec ou sans addition d'une quantité connue de $H_2O_2$ dans le milieu réactionnel juste après l'addition d'ascorbate si non précisé par b). La valeur théorique en $H_2O_2$ s'il n'y a pas de dégradation est indiquée entre parenthèses. Elle correspond à l'addition du nombre de nanomoles de $H_2O_2$ ajoutés à ceux produits dans une réaction réalisée dans les mêmes conditions expérimentales mais sans ajout de $H_2O_2$. $H_2O_2$ est dosé à l'aide d'un kit AmplexRed $H_2O_2$/HRP Assay.

a) Quand non précisé
b) $H_2O_2$ ajouté juste avant l'ascorbate.

**Modulation de l'hydrophobicité :**

***Détermination des log $D_{7,4}$ :*** La méthode est adaptée de Z.-P Zhuang et al., J. Med. Chem., 2001, 44, 1905-1914. Le ligand (2,0 mg) est dissout dans 2 mL de 1-octanol puis 2 mL de tampon Tris·HCl 20 mM, pH = 7,4 ; NaCl 150 mM sont ajoutés. Après un mélange au vortex durant 3 min à température ambiante suivi d'une centrifugation durant 5 min à 9 000 t/min, la concentration du ligand dans chaque phase est déterminée par spectrophotométrie UV-visible. Les fractions 1-octanol sont repartitionnées ainsi jusqu'à ce que des valeurs reproductibles de coefficient de partition soient obtenues. Ce coefficient est exprimé comme le logarithme décimal de [(la concentration de ligand contenu dans la phase 1-octanol)/(la concentration de ligand contenu dans la phase aqueuse tamponnée)] = log $D_{7,4}$. Les mesures ont été réalisées trois fois.

Le tableau 8 résume les résultats obtenus.

La valeur de log $D_{7,4}$ reflète l'hydrophobicité et donc la lipophilicité des composés qui est un des paramètres employés (avec d'autres tels le poids moléculaire) pour estimer les possibilités de biodistribution des molécules (H. van de Waterbeemd et al., Nature Rev. Drug Discovery 2003, 2, 192-204).

Pour des substitutions identiques des macrocycles aromatiques, les dérivés comportant un seul résidu quinoléine sont plus hydrophiles que ceux en comportant plusieurs. L'hydrophobicité peut être modulée par la longueur du bras de jonction entre les résidus quinoléine (comparer **7** et **10),** par la nature dudit bras (comparer **7** et **18)** et par la substitution des hydrogènes des cycles (comparer respectivement : **1** et **2 ; 3** et **4 ; 7,8, 9** et **17)** ou des bras de jonction (comparer **1, 3, 5** et **6)** par différents groupements.

**Tableau 8:** Valeur du logarithme décimal du coefficient de partage des ligands entre du 1-octanol (phase hydrophobe) et du tampon Tris·HCl 20 mM ; NaCl 150 mM (pH = 7.4) (phase hydrophile) (1/1, v/v).

| Ligand | PM | log $D_{7,4}$ |
|---|---|---|
| **1** | 301 | 3,3 ± 0,1 |
| **2** | 370 | 3,5 ± 0,1 |
| **3** | 330 | 4,4 ± 0,1 |
| **4** | 398 | 4,9 ± 0,1 |
| **5** | 338 | 3,8 ± 0,1 |
| **6** | 316 | 3,9 ± 0,1 |
| **7** | 316 | 3,5 ± 0,1 |
| **8** | 384 | 4,2 ± 0,1 |
| **9** | 636 | 5,6 ± 0,5 |
| **10** | 344 | 3,8 ± 0,1 |

(suite)

| Ligand | PM | log D $_{7,4}$ |
|---|---|---|
| **11** | 474 | 3,7 ± 0,1 |
| **12** | 271 | 3,5 ± 0,1 |
| **13** | 328 | 2,7 ± 0,1 |
| **14** | 342 | 1,8 ± 0,1 |
| **15** | 272 | 2,0 ± 0,1 |
| **17** | 376 | 3,5 ± 0,1 |
| **18** | 331 | 3,0 ± 0,1 |
| **23** | 357 | 2,5 ± 0,1 |
| **24** | 299 | 2,6 ± 0,1 |
| 8-hydroxyquinoléine | 145 | 2,1 ± 0,1 |
| 8-hydroxyquinaldine | 159 | 2,4 ± 0,1 |
| Clioquinol | 305 | 3,8 ± 0,1 |
| 8-aminoquinoléine | 144 | 1,9 ± 0,1 |

**Analyse de la génotoxicité potentielle :**

***Test de AMES :*** La méthode est adaptée de D.M. Maron et B. N. Ames, Mutat. Res. 1983, 113, 173-215 ; D . E. Levin et al., Mutat. Res. 1982, 94, 315-330; D. E. Levin et al. Proc. Natl. USA 1982, 79, 7445-7449.

Deux lignées test (TA98 et TA100) de *Salmonella tiphimurium* mutant (His-) ont été utilisées de par leur déficience à synthétiser l'histidine et de la confirmation des caractéristiques de ces lignées par des tests de marqueurs génétiques. Le test consiste en l'évaluation du potentiel des molécules testées à induire une mutation réverse au locus histidine de ces lignées. Ces deux lignées ont été fournies par le Dr. Bruce N. Ames (University of California, Berkeley, USA). Elles sont résistantes à l'Ampicilline et sensibles à la Tetracycline. Elles ne sont pas mutantes pour rfa, UvγB et UvγA. La lignée TA98 (his D3052) contient une mutation de phase de lecture (GC) tandis que la lignée TA100 (his G46) conteint une substitution de paire de base (GC). Le nombre de révertant spontané est de 37 ± 6 et 166 ± 15 colonies par plaques pour TA98 et TA100, respectivement.

Les composés à tester ont été dissous dans 100 % de DMSO (le solvant) et dilués logarithmiquement (d'un facteur 10) de façon à obtenir 4 concentrations test de 30 000 ; 3 000 ; 300 et 30 μg/mL. Chaque série de 0,1 mL de solution stock à tester, 0,1 mL de lignée test et de milieu de culture avec ou pas 0,5 mL d'homogénat enzymatique (S9) de microsome de foie de rat ont été mélangés avec 2 mL d'agarose fondue (contenant 0,5 mM d'histidine et 0,5 mM de biotine). Ce mélange a été déposé à la surface d'une plaque de milieu agarose glucose minimal (30 mL pour chaque boite de pétrie) de façon à obtenir des concentrations finales en composé à tester de 3 000; 300; 30 et 3 μg/plaque. Les plaques ont alors été incubées à 37°C durant 48 heures. Les cultures ont été traitées ou non en présence d'une activation métabolique exogène par l'ajout de 0,5 mL de mélange S9 qui contenait 8 mM $MgCl_2$, 33 mM KCl, 4 mM NADP, 5 mM glucose-6-phosphate, 100 mM $NaH_2PO_4$ (pH = 7,4) et 4 % (v/v) d'homogénat enzymatique de microsome de foie de rat induit par Acrolor 1254 (S9). Les colonies révertantes des lignées test ont été dénombrées avec un compteur de colonies (Sigma). Les résultats ont été considérés comme significatifs uniquement si les valeurs obtenues pour le solvant seul et des composés de référence étaient dans une fourchette précédemment établie. Un nombre de colonies ≥ 3 fois au nombre de colonies observé pour le solvant seul est considéré comme mutagène de façon significative. Un composé induisant un nombre de colonie < 50 % de celles obtenues pour le solvant seul a été considéré comme toxique pour les bactéries testées. Tous les essais ont été réalisés en triplicate.

L'obtention de résultats attendus avec ces lignées pour 4-NPD, l'azide de sodium, 2-Anthramine et 2-Aminofluorène a permis de confirmer la bonne qualité de l'étude.

Les résultats obtenus sont résumés dans le tableau 9.

**Tableau 9 :** Résultats du test de Ames de mutagénicité de Salmonella sur les souches TA98 et TA100 de salmonelle en absence ou en présence d'homogénat enzymatique (S9) de microsome de foie de rat.

| Composé (valeur par plaque) | Ajout de S9 | TA98 | | TA100 | |
|---|---|---|---|---|---|
| | | Effet mutagène | Cytotoxicité | Effet mutagène | Cytotoxicité |
| DMSO (solvant) | non > | 100 µL | > 100 µL | > 100 µL | > 100 µL |
| | oui | > 100 µL | > 100 µL | > 100 µL | > 100 µL |
| Clioquinol | non | - | 300 µg | - | 30 **µg** |
| | oui | - | 300 µg | - | 30 µg |
| **3** | non | > 3000 µg | > 3000 µg | > 3000 µg | > 3000 µg |
| | oui | > 3000 µg | > 3000 µg | > 3000 µg | > 3000 µg |
| **5** | non | > 3000 µg | > 3000 µg | - | 300 µg |
| | oui | > 3000 µg | > 3000 µg | > 3000 µg | > 3000 µg |
| **12** | non | > 3000 µg | > 3000 µg | > 3000 µg | > 3000 µg |
| | oui | > 3000 µg | > 3000 µg | > 3000 µg | > 3000 µg |

Un effet bactéricide du Clioquinol est observé sur les deux lignées. Aucun effet mutagène n'a été observé pour les composés polyquinoléine testés même pour les plus fortes doses étudiées. Aucun effet bactéricide n'a été observé pour ces composés même pour les plus fortes doses testées à l'exception du composé 5 à partir de 300 µg/plaque, sur la lignée TA100 en absence de S9.

## Revendications

**1.** Composés de formule (I)

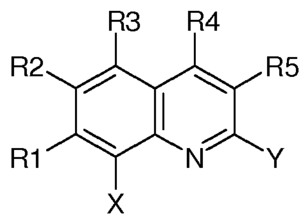

(I)

pour utilisation pour chélater les ions métalliques telle que dans la formule (I)

X représente un groupe -OR, -NRR', -S(O)pR, -OCOR, -OCOOR, et
Y représente un groupe de formule :

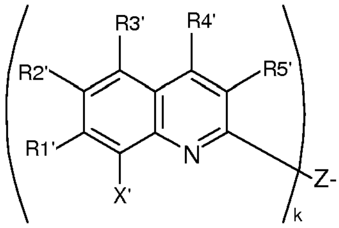

(IY)

dans laquelle X' représente un groupe -OR, -NRR', -S(O)pR, -OCOR, -OCOOR et Z représente un groupe de formule $-(A)_m-(Alk)_n-(A')_{m'}-$ où
m, n, m' identiques ou différents représentent indépendamment 0 ou 1, étant entendu que au moins un des m, n, m' soit égal à 1,
A, A', identiques ou différents, représentent indépendamment un groupe choisi parmi -O-, -C(=O)-, ou un cycle de 4 à 11 chaînons choisi parmi les cycloalkyles, hétérocycles, aryles, hétéroaryles, ledit cycle étant éventuellement substitué par un ou plusieurs substituants choisis parmi Alkyle, OR, NRR', $CF_3$, Hal, CN, S(O)pR, COOR, OCOOR, CONRR', NRCOOR ;
Alk représente indépendamment un groupe -alkyle- éventuellement substitué par un ou plusieurs substituants choisis parmi OR, NRR', $CF_3$, Hal, CN, S(O)pR, COOR, OCOOR, CONRR', NRCOOR ;
et
R et R' identiques ou différents représentent indépendamment un atome d'hydrogène ou un groupe cycloalkyle ou alkyle éventuellement substitué par un ou plusieurs groupes choisis parmi OR, NRR', Hal, -CN, $-CF_3$, S(O)pR, COOR, OCOOR, CONRR', NRCOOR ;
R1, R2, R3, R4, R5, R1', R2', R3', R4', R5', R6' identiques ou différents représente indépendamment un groupe ou atome choisi parmi H, OR, NRR', Hal, -CN, $-CF_3$, S(O)pR, COOR, OCOOR, CONRR', NRCOOR, alkyle éventuellement substitué par un ou plusieurs groupes choisis parmi OR, NRR', Hal, -CN, $-CF_3$, S(O)pR, COOR, OCOOR, CONRR', NRCOOR ;
k représente 1 ;
p représente 0, 1 ou 2 ;
ainsi que leurs stéréoisomères ou leur mélanges, leurs formes tautomères, leurs hydrates, solvates, leurs sels, formes libres et esters pharmaceutiquement acceptables.

**2.** Composés pour utilisation selon la revendication 1 tels que les composés sont choisis parmi :

Diméthyl-bis[8-(acétyloxy)-2-quinoléinyl]propanedioate
2,2'-Méthanediyl-bis(8-hydroxyquinoléine)
2,2'-Méthanediyl-bis(5-chloro-8-hydroxyquinoléine)
2,2'-(2,2-Propanediyl)-bis(8-hydroxyquinoléine)
2,2'-(2,2-Propanediyl)-bis(5-chloro-8-hydroxyquinoléine)
2,2'-(Difluorométhanediyl)-bis(8-hydroxyquinoléine)
Bis(8-hydroxy-2-quinoléinyl)méthanone
2,2'-(1,2-Ethanediyl)-bis[8-(méthyloxy)quinoléine]
2,2'-(1,2-Ethanediyl)-bis(8-hydroxyquinoléine)
2,2'-(1,2-Ethanediyl)-bis(5-chloro-8-hydroxyquinoléine)
2,2'-(1,2-Ethanediyl)-bis(5-chloro-7-iodo-8-hydroxyquinoléine)
2,2'-(1,4-Butanediyl)-bis[8-(méthyloxy)quinoléine]
2,2'-(1,4-Butanediyl)-bis(8-hydroxyquinoléine)
2,2',2''-(1,2,3-Propanetriyl)-tris[8-(méthyloxy)quinoléine]
2,2',2''-(1,2,3-Propanetriyl)-tris(8-hydroxyquinoléine)

ainsi que leurs stéréoisomères ou leur mélanges, leurs formes tautomères, leurs hydrates, solvates, leurs sels, formes libres et esters pharmaceutiquement acceptables.

**3.** Composés pour utilisation selon la revendication 1 ou 2 tels que lesdits métaux sont choisis parmi le zinc, cuivre

et/ou fer.

4. Composés de formule (1)

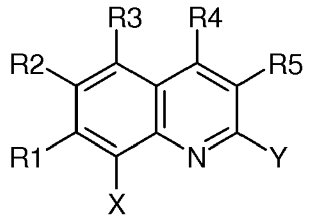

(I)

pour utilisation pour prévenir et/ou traiter les maladies affectant le système nerveux central, telles que les maladies neurodégénératives telle que dans la formule (1) :

X représente un groupe -OR, -NRR’, -S(O)pR, -OCOR, -OCOOR et
Y représente un groupe de formule :

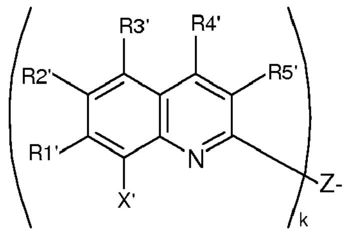

(IY)

dans laquelle X’ représente un groupe -OR, -NRR’, -S(O)pR, -OCOR, -OCOOR et Z représente un groupe de formule $-(A)_m-(Alk)_n-(A')_{m'}-$ où
m, n, m’ identiques ou différents représentent indépendamment 0 ou 1, étant entendu que au moins un des m, n, m’ soit égal à 1,
A, A’ représentent indépendamment un groupe choisi parmi -O-, -C(=O)-, ou un cycle de 4 à 11 chaînons choisi parmi les cycloalkyles, hétérocycles, aryles, hétéroaryles, ledit cycle étant éventuellement substitué par un ou plusieurs substituants choisis parmi Alkyle, OR, NRR’, $CF_3$, Hal, CN, S(O)pR, COOR, OCOOR, CONRR’, NRCOOR ;
Alk représente indépendamment un groupe -alkyle- éventuellement substitué par un ou plusieurs substituants choisis parmi OR, NRR’, $CF_3$, Hal, CN, S(O)pR, COOR, OCOOR, CONRR’, NRCOOR ;
R et R’ identiques ou différents représentent indépendamment un atome d’hydrogène ou un groupe cycloalkyle ou alkyle éventuellement substitué par un ou plusieurs groupes choisis parmi OR, NRR’, Hal, -CN, $-CF_3$, S(O)pR, COOR, OCOOR, CONRR’, NRCOOR ;
R1, R2, R3, R4, R5, R1’, R2’, R3’, R4’, R5’, R6’ identiques ou différents représente indépendamment un groupe ou atome choisi parmi H, OR, NRR’, Hal, -CN, $-CF_3$, S(O)pR, COOR, OCOOR, CONRR’, NRCOOR, alkyle éventuellement substitué par un ou plusieurs substituants choisis parmi OR, NRR’, $CF_3$, Hal, CN,
S(O)pR, COOR, OCOOR, CONRR’, NRCOOR ;
k représente 1 ;
p représente 0, 1 ou 2 ;
ainsi que leurs stéréoisomères ou leur mélanges, leurs formes tautomères, leurs hydrates, solvates, leurs sels, formes libres et esters pharmaceutiquement acceptables,

5. Composés pour utilisation selon la revendication 4, tels que les composés sont choisis parmi :

Diméthyl-bis[8-(acétyloxy)-2-quinoléinyl]propanedioate
2,2’-Méthanediyl-bis(8-hydroxyquinoléine)

2,2'-Méthanediyl-bis(5-chloro-8-hydroxyquinoléine)
2,2'-(2,2-Propanediyl)-bis(8-hydroxyquinoléine)
2,2'-(2,2-Propanediyl)-bis(5-chloro-8-hydroxyquinoléine)
2,2'-(Difluorométhanediyl)-bis(8-hydroxyquinoléine)
Bis(8-hydroxy-2-quinoléinyl)méthanone
2,2'-(1,2-Ethanediyl)-bis[8-(méthyloxy)quinoléine]
2,2'-(1,2-Ethanediyl)-bis(8-hydroxyquinoléine)
2,2'-(1,2-Ethanediyl)-bis(5-chloro-8-hydroxyquinoléine)
2,2'-(1,2-Ethanediyl)-bis(5-chloro-7-iodo-8-hydroxyquinoléine) 2,2'-(1,4-Butanediyl)-bis[8-(méthyloxy)quinoléine]
2,2'-(1,4-Butanediyl)-bis(8-hydroxyquinoléine)
2,2',2''-(1,2,3-Propanetriyl)-tris[8-(méthyloxy)quinoléine]
2,2',2''-(1,2,3-Propanetriyl)-tris(8-hydroxyquinoléine)

ainsi que leurs stéréoisomères ou leur mélanges, leurs formes tautomères, leurs hydrates, solvates, leurs sels, formes libres et esters pharmaceutiquement acceptables.

**6.** Composés pour utilisation selon la revendication 4 ou 5 tels que les maladies neurodégénératives sont choisies parmi la maladie d'Alzheimer, la maladie de Parkinson, la maladie d'Huntington, la sclérose latérale amyotrophique ou la trisomie 21, les encéphalopathies spongiformes, telle que la maladie de Kreutzfeld-Jacob.

**7.** Composition pharmaceutique comprenant un composé de formule (1)

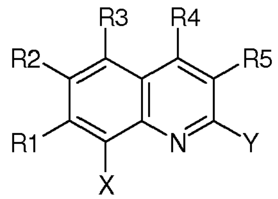

(I)

tels que dans la formule (I)

X représente un groupe -OH, -NRR', -S(O)pR, -OCOOR et
Y représente un groupe de formule :

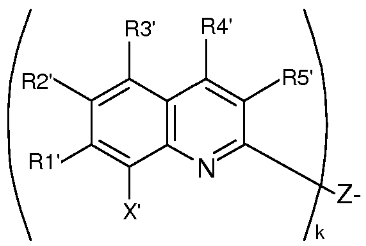

(IY)

dans laquelle X' représente un groupe -OH, -NRR', -S(O)pR, -OCOOR et
Z représente un groupe de formule $-(A)_m-(Alk)_n-(A')_{m'}-$ où m, n, m' identiques ou différents représentent indépendamment 0 ou 1, étant entendu que au moins un des m, n, m', n', m'', n'' soit égal à 1, A, A' identiques ou différents représentent indépendamment un groupe choisi parmi -O-, -C(=O)-, ou un cycle de 4 à 11 chaînons choisi parmi les cycloalkyles, hétérocycles, aryles, hétéroaryles, ledit cycle étant éventuellement substitué par un ou plusieurs substituants choisis parmi Alkyle, OR, NRR', $CF_3$, Hal, CN, S(O)pR, COOR, OCOOR, CONRR', NRCOOR ; Alk

représente indépendamment un groupe -alkyle- éventuellement substitué par un ou plusieurs substituants choisis parmi OR, NRR', $CF_3$, Hal, CN, S(O)pR, COOR, OCOOR, CONRR', NRCOOR ;
et R et R' identiques ou différents représentent indépendamment un atome d'hydrogène ou un groupe cycloalkyle ou alkyle éventuellement substitué par un ou plusieurs groupes choisis parmi OR, NRR', Hal, -CN, -$CF_3$, S(O)pR, COOR, OCOOR, CONRR', NRCOOR ;
R1, R2, R3, R4, R5, R1', R2', R3', R4', R5', R6' identiques ou différents représente indépendamment un groupe ou atome choisi parmi H, OR, NRR', Hal, -CN, -$CF_3$, alkyle éventuellement substitué par un ou plusieurs substituants choisis parmi OR, NRR', $CF_3$, Hal, CN, S(O)pR, COOR, OCOOR, CONRR', NRCOOR ;
k représente 1 ;
p représente 0, 1 ou 2 ;
ainsi que leurs stéréoisomères ou leur mélanges, leurs formes tautomères, leurs hydrates, solvates, leurs sels, formes libres et esters pharmaceutiquement acceptables.

**8.** Composition pharmaceutique selon la revendication 7 telle que les composés sont choisis parmi :

Diméthyl-bis[8-(acétyloxy)-2-quinoléinyl]propanedioate
2,2'-Méthanediyl-bis(8-hydroxyquinoléine)
2,2'-Méthanediyl-bis(5-chloro-8-hydroxyquinoléine)
2,2'-(2,2-Propanediyl)-bis(8-hydroxyquinoléine)
2,2'-(2,2-Propanediyl)-bis(5-chloro-8-hydroxyquinoléine)
2,2'-(Difluorométhanediyl)-bis(8-hydroxyquinoléine)
Bis(8-hydroxy-2-quinoléinyl)méthanone
2,2'-(1,2-Ethanediyl)-bis[8-(méthyloxy)quinoléine]
2,2'-(1,2-Ethanediyl)-bis(8-hydroxyquinoléine)
2,2'-(1,2-Ethanediyl)-bis(5-chloro-8-hydroxyquinoléine)
2,2'-(1,2-Ethanediyl)-bis(5-chloro-7-iodo-8-hydroxyquinoléine) 2,2'-(1,4-Butanediyl)-bis[8-(méthyloxy)quinoléine]
2,2'-(1,4-Butanediyl)-bis(8-hydroxyquinoléine)
2,2',2''-(1,2,3-Propanetriyl)-tris[8-(méthyloxy)quinoléine]
2,2',2''-(1,2,3-Propanetriyl)-tris(8-hydroxyquinoléine)

ainsi que leurs stéréoisomères ou leur mélanges, leurs formes tautomères, leurs hydrates, solvates, leurs sels, formes libres et esters pharmaceutiquement acceptables.

**9.** Composé de formule (I) :

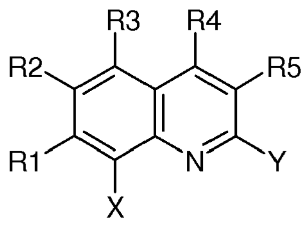

(I)

tels que dans la formule (1)

X représente un groupe -OH, -NRR', -S(O)pR, -OCOOR et
Y représente un groupe de formule :

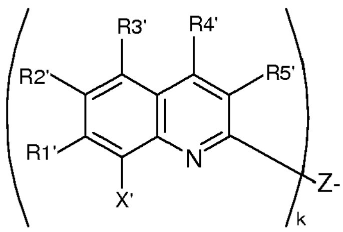

(IY)

dans laquelle X' représente un groupe -OH, -NRR', -S(O)pR, -OCOOR et Z représente un groupe de formule $-(A)_m-(Alk)_n-(A')_{m'}-$ où m, n, m' identiques ou différents représentent indépendamment 0 ou 1, étant entendu que au moins un des m, n, m', n', m", n" soit égal à 1, A, A' identiques ou différents représentent indépendamment un groupe choisi parmi -O-, -C(=O)-, ou un cycle de 4 à 11 chaînons choisi parmi les cycloalkyles, hétérocycles, aryles, hétéroaryles, ledit cycle étant éventuellement substitué par un ou plusieurs substituants choisis parmi Alkyle, OR, NRR', $CF_3$, Hal, CN, S(O)pR, COOR, OCOOR, CONRR', NRCOOR ; Alk représente indépendamment un groupe -alkyle- éventuellement substitué par un ou plusieurs substituants choisis parmi OR, NRR', $CF_3$, Hal, CN, S(O)pR, COOR, OCOOR, CONRR', NRCOOR ;
et
R et R' identiques ou différents représentent indépendamment un atome d'hydrogène ou un groupe cycloalkyle ou alkyle éventuellement substitué par un ou plusieurs groupes choisis parmi OR, NRR', Hal, -CN, $-CF_3$, S(O)pR, COOR, OCOOR, CONRR', NRCOOR ;
R1, R2, R3, R4, R5, R1', R2', R3', R4', R5', R6' identiques ou différents représente indépendamment un groupe ou atome choisi parmi H, OR, NRR', Hal, -CN, $-CF_3$, alkyle éventuellement substitué par un ou plusieurs substituants choisis parmi OR, NRR', $CF_3$, Hal, CN, S(O)pR, COOR, OCOOR, CONRR', NRCOOR ;
k représente 1 ;
p représente 0, 1 ou 2 ; ainsi que leurs stéréoisomères ou leur mélanges, leurs formes tautomères, leurs hydrates, solvates, leurs sels, formes libres et esters pharmaceutiquement acceptables,
à l'exception des composés pour lesquels :

X représente un groupe -OH, et
Y représente un groupe de formule :

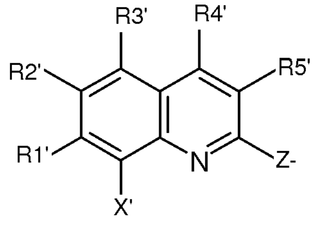

dans laquelle X' représente un groupe -OH, et
Z représente un groupe choisi parmi $-CH_2-$, $-CH_2-CH_2-$, $-(CH_2)_6-$, -CH=CH-, $-C(Me)_2-$, -thienyl-,
R1, R2, R3, R4, R5, R1', R2', R3', R4', R5', R6' sont égaux à H.

**10.** Composé selon la revendication 9 choisi parmi :

2,2'-Méthanediyl-bis(5-chloro-8-hydroxyquinoléine)
2,2'-(2,2-Propanediyl)-bis(5-chloro-8-hydroxyquinoléine)
2,2'-(Difluorométhanediyl)-bis(8-hydroxyquinoléine)
Bis(8-hydroxy-2-quinoléinyl)méthanone

2,2'-(1,2-Ethanediyl)-bis(5-chloro-8-hydroxyquinoléine)
2,2'-(1,2-Ethanediyl)-bis(5-chloro-7-iodo-8-hydroxyquinoléine) 2,2'-(1,4-Butanediyl)-bis[8-(méthyloxy)quinoléine]
2,2'-(1,4-Butanediyl)-bis(8-hydroxyquinoléine)
2,2',2''-(1,2,3-Propanetriyl)-tris[8-(méthyloxy)quinoléine]
2,2',2''-(1,2,3-Propanetriyl)-tris(8-hydroxyquinoléine)

ainsi que leurs stéréoisomères ou leur mélanges, leurs formes tautomères, leurs hydrates, solvates, leurs sels, formes libres et esters pharmaceutiquement acceptables.

**11.** Composé selon l'une quelconque des revendications 9 ou 10 tel que X représente OR et Y représente un groupe de formule (IY) dans laquelle X' représente OR et Z représente A où A représente -C(O)- ou Z représente Alk où Alk représente un groupe alkyle linéaire ou ramifié éventuellement substitué par un ou plusieurs atomes d'halogène ; R=H ou alkyle éventuellement substitué, et
R1, R2, R3, R4, R5, R1', R2', R3', R4', R5', R6' identiques ou différents représente indépendamment un groupe ou atome choisi parmi H, OR, NRR', Hal,- CN, -$CF_3$, alkyle éventuellement substitué par un ou plusieurs substituants choisis parmi OR, NRR', $CF_3$, Hal, CN, S(O)pR, COOR, OCOOR, CONRR', NRCOOR.

**12.** Procédé de préparation d'un composé selon l'une quelconque des revendications 9 à 11 comprenant l'étape de couplage des composés de formule (II) et (II') :

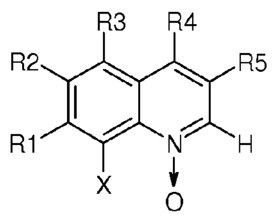

(II)

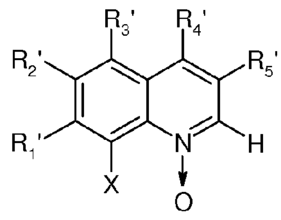

(II')

suivi d'une hydrolyse, et, éventuellement suivi de dérivatisation(s) du produit de formule (1) obtenu pour obtenir le produit de formule (I) souhaité et/ou de l'étape d'isolation du produit obtenu.

**13.** Procédé de préparation d'un composé selon l'une quelconque des revendications 9 à 11 comprenant l'étape de couplage des composés de formule (III) et (III') :

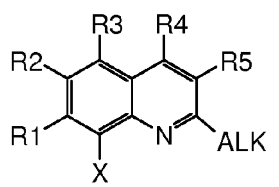

(III)

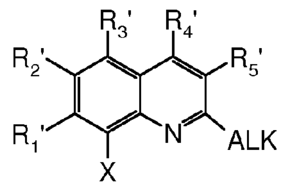

(III')

éventuellement suivi de dérivatisation(s) du produit de formule (I) obtenu pour obtenir le produit de formule (I) souhaité, et/ou de l'étape d'isolation du produit final obtenu.

**14.** Procédé de préparation d'un composé selon l'une quelconque des revendications 9 à 11 comprenant l'étape de couplage des composés de formule (IV) et (IV') :

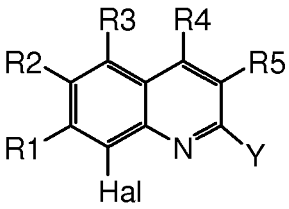

(IV)

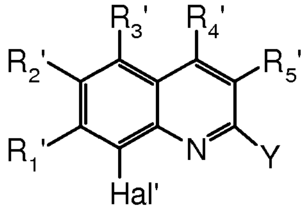

(IV')

dans lesquelles Hal et Hal' représentent des atomes d'halogène en présence d'un groupe de formule HZH, éventuellement suivi de dérivatisation(s) du produit de formule (I) obtenu pour obtenir le produit de formule (I) souhaité et/ou de l'isolation du produit obtenu.

**15.** Composés pour utilisation selon l'une quelconque des revendications 1 à 6 telle que X représente OR et Y représente un groupe de formule (IY) dans laquelle X' représente OR et Z représente A où A représente -C(O)- ou Z représente Alk où Alk représente un groupe alkyle linéaire ou ramifié éventuellement substitué par un ou plusieurs atomes d'halogène ; R=H ou alkyle éventuellement substitué, et
R1, R2, R3, R4, R5, R1', R2', R3', R4', R5', R6' identiques ou différents représente indépendamment un groupe ou atome choisi parmi H, OR, NRR', Hal, -CN, -$CF_3$, alkyle éventuellement substitué par un ou plusieurs substituants choisis parmi OR, NRR', $CF_3$, Hal, CN, S(O)pR, COOR, OCOOR, CONRR', NRCOOR.

**16.** Composition pharmaceutique selon l'une quelconque des revendications 7 ou 8 telle que X représente OR et Y représente un groupe de formule (IY) dans laquelle X' représente OR et Z représente A où A représente -C(O)- ou Z représente Alk où Alk représente un groupe alkyle linéaire ou ramifié éventuellement substitué par un ou plusieurs atomes d'halogène ; R=H ou alkyle éventuellement substitué, et
R1, R2, R3, R4, R5, R1', R2', R3', R4', R5', R6' identiques ou différents représente indépendamment un groupe ou atome choisi parmi H, OR, NRR', Hal, -CN, -$CF_3$, alkyle éventuellement substitué par un ou plusieurs substituants choisis parmi OR, NRR', $CF_3$, Hal, CN, S(O)pR, COOR, OCOOR, CONRR', NRCOOR.

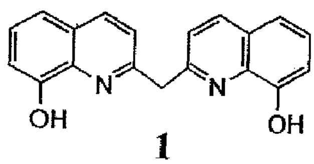

1

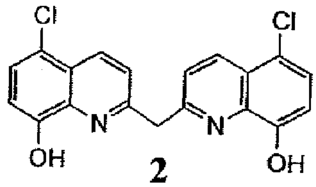

2

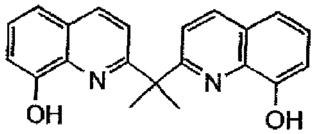

3

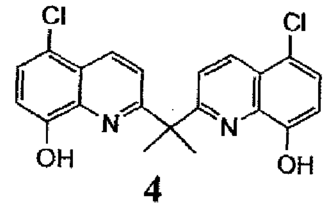

4

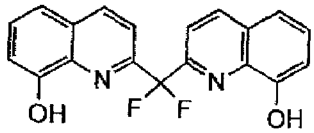

5

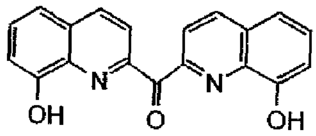

6

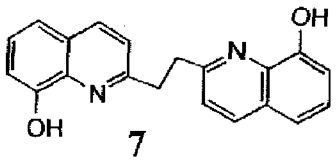

7

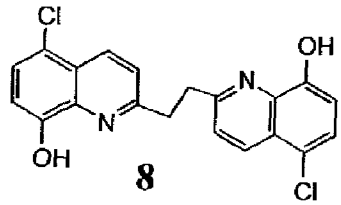

8

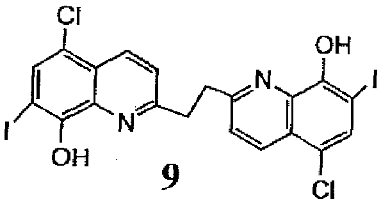

9

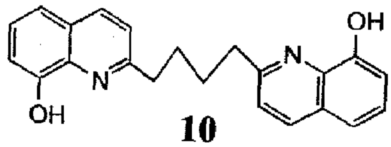

10

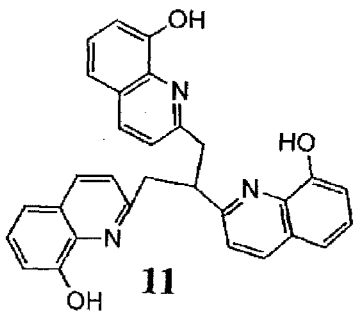

11

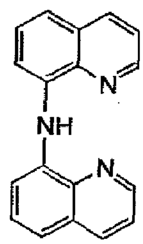

12

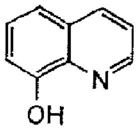

8-hydroxyquinoléine

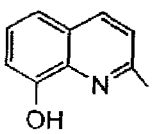

8-hydroxyqunaldine

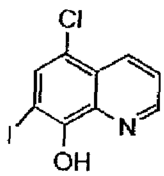

Clioquinol

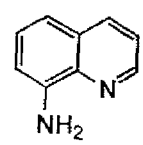

8-aminoquinoléine

FIG.1 (début)

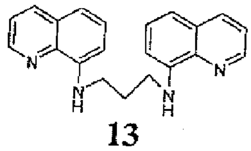
13

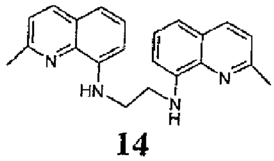
14

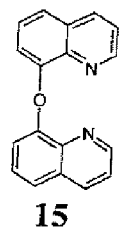
15

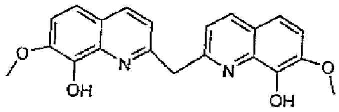
16

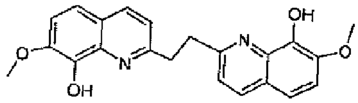
17

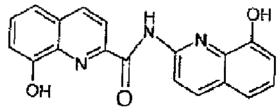
18

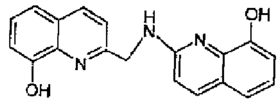
19

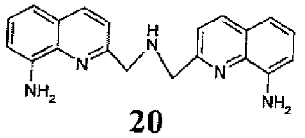
20

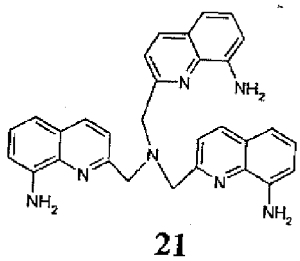
21

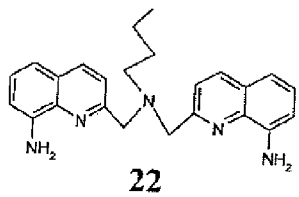
22

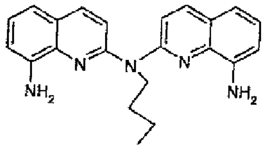
23

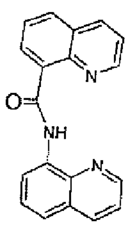
24

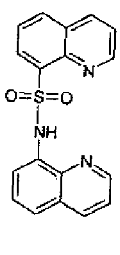
25

FIG.1 (fin)

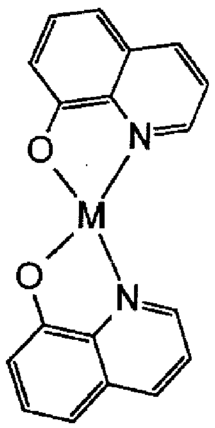

cis

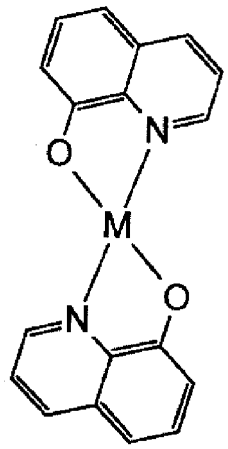

trans

FIG.2

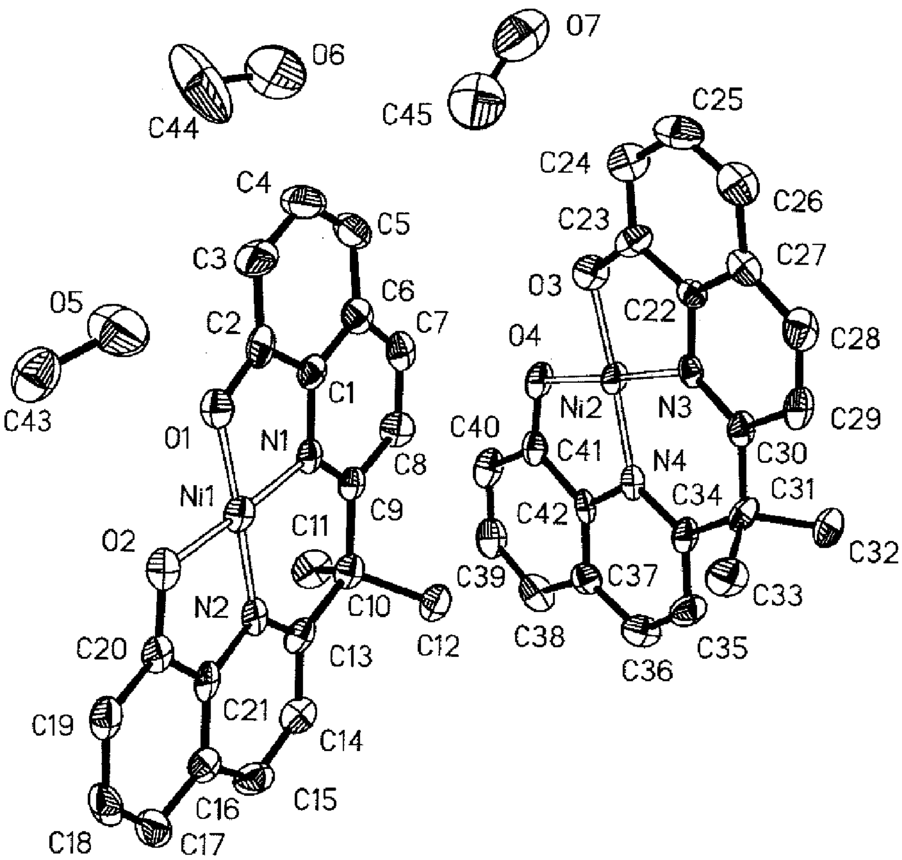

FIG.3

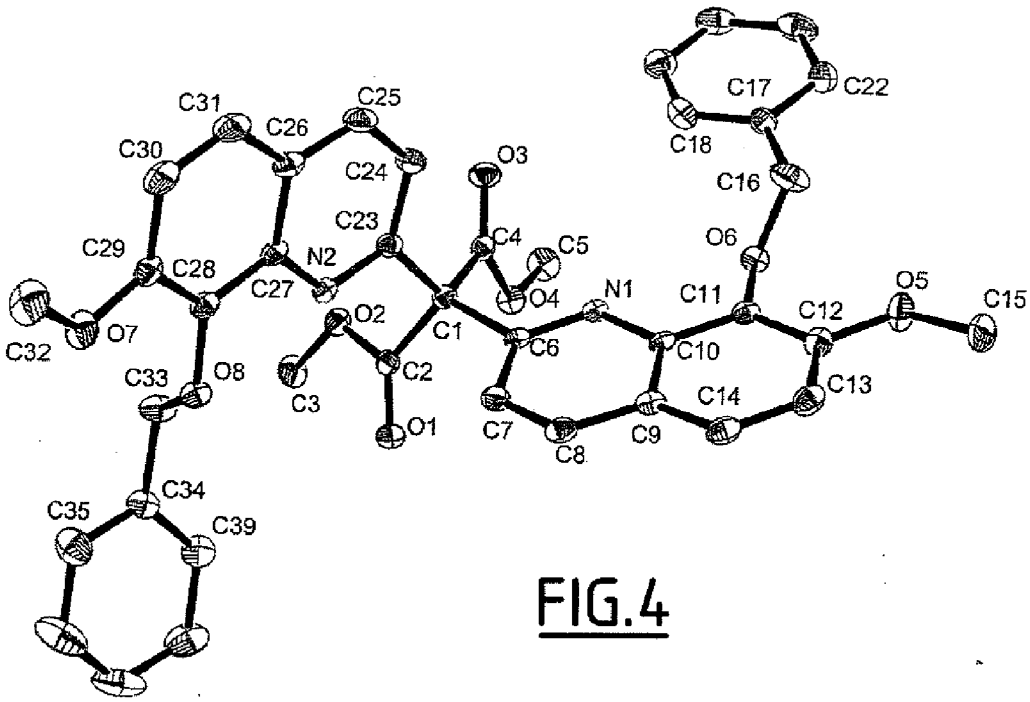

FIG.4

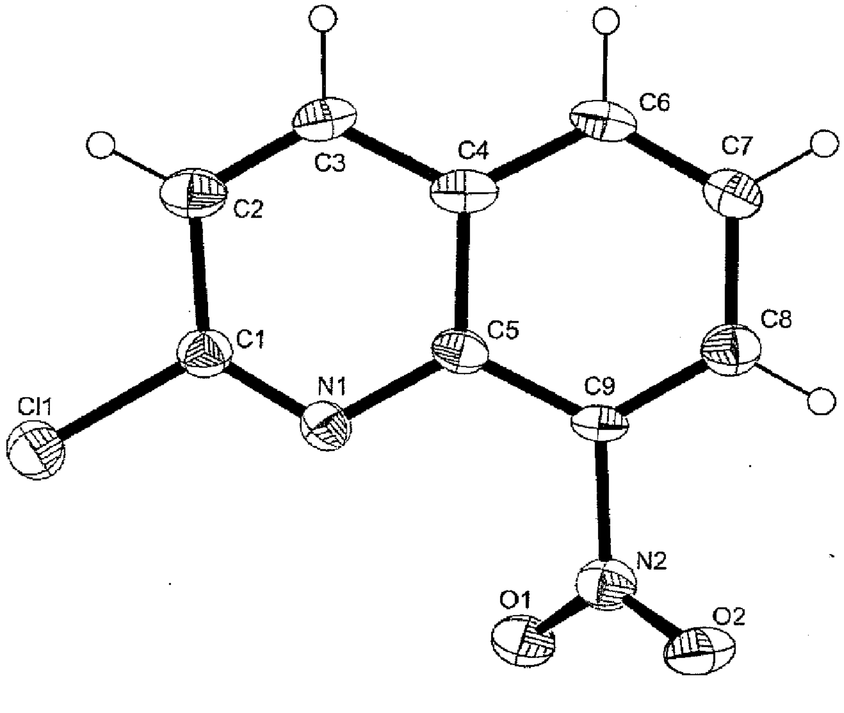

FIG.5

FIG.6

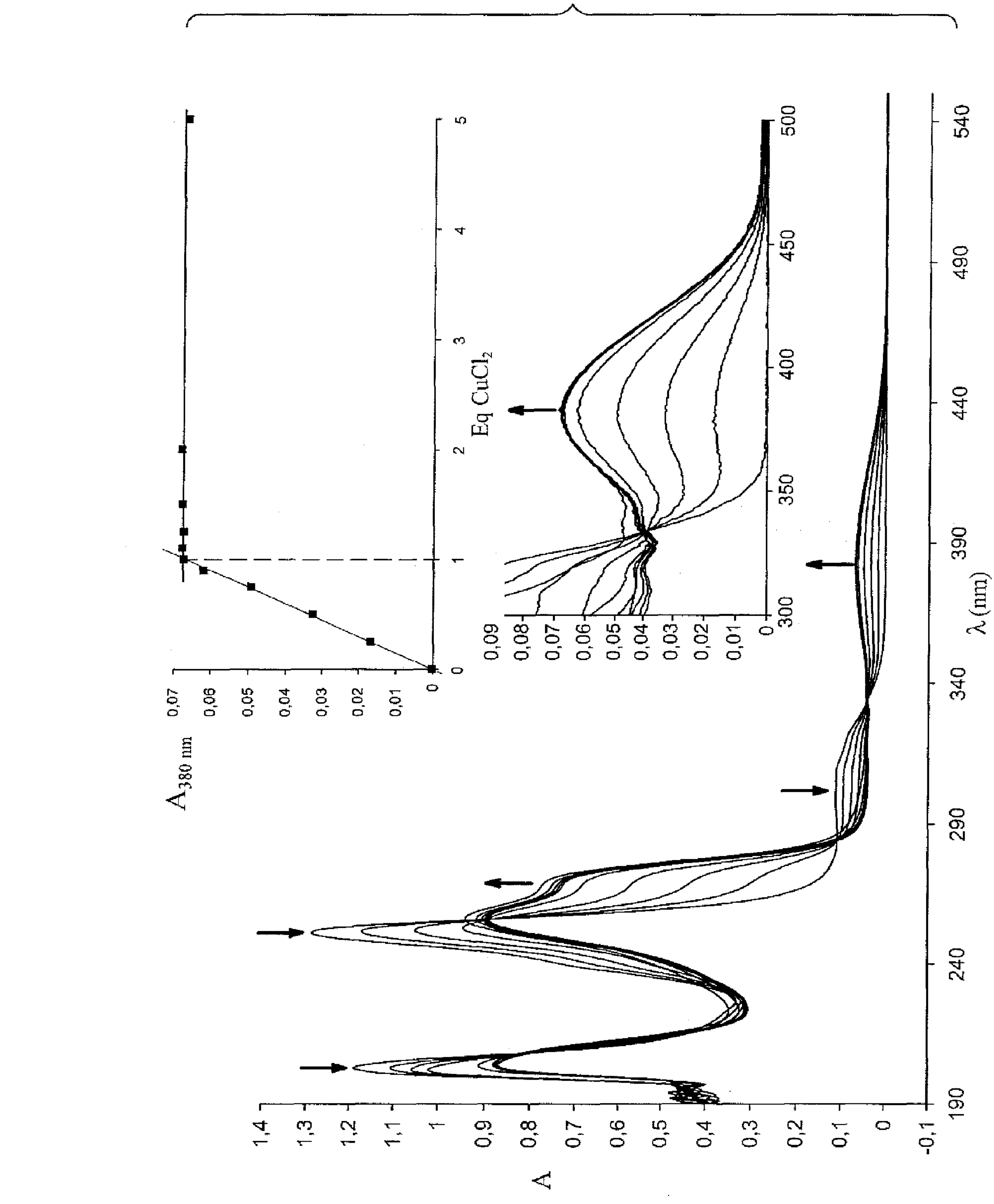

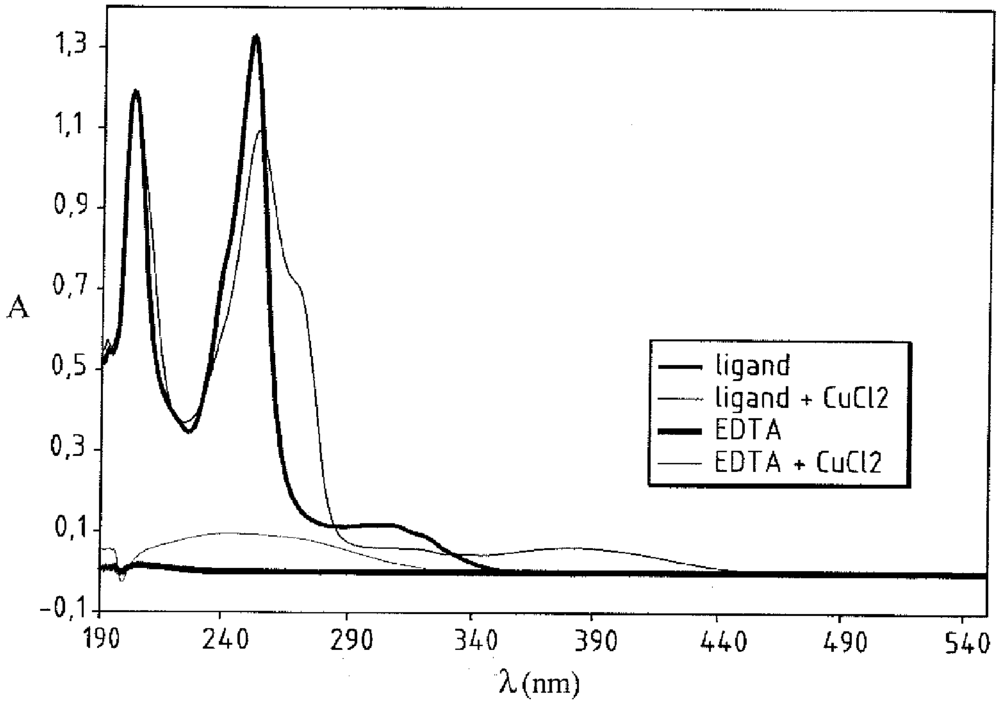
A
1,3
1,1
0,9
0,7
0,5
0,3
0,1
-0,1
190
240
290
340
390
440
490
540
λ (nm)
ligand
ligand + CuCl2
EDTA
EDTA + CuCl2

FIG.7

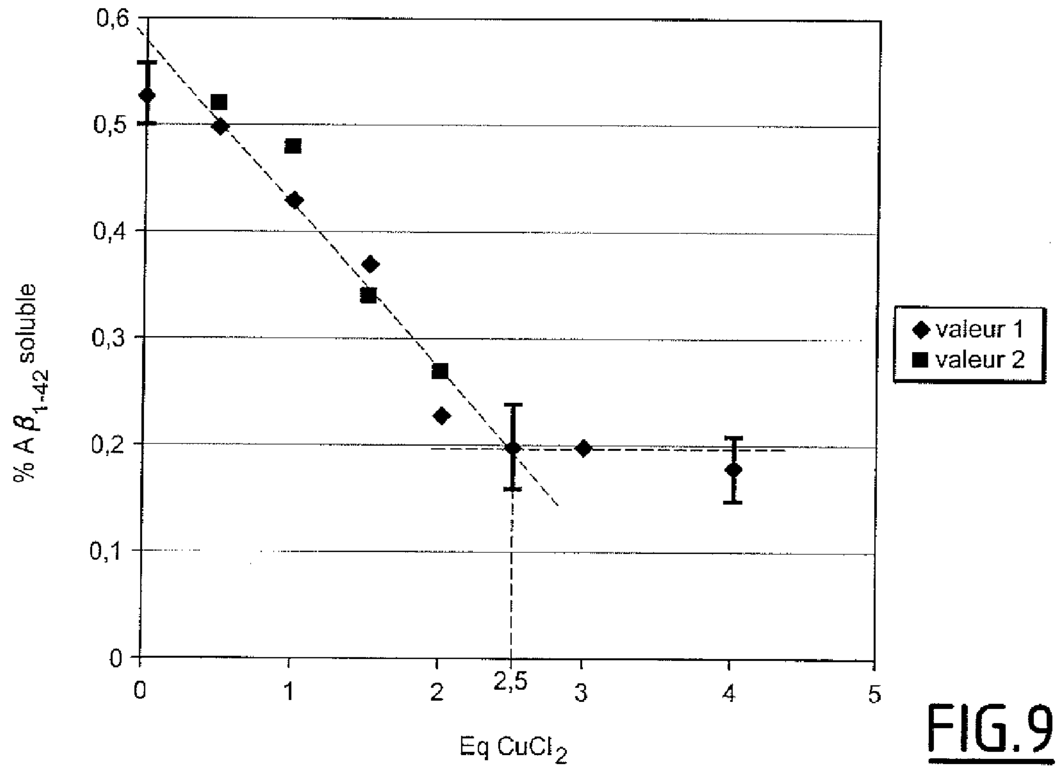
0,6
0,5
0,4
0,3
0,2
0,1
0
% A $\beta_{1-42}$ soluble
0
1
2
2,5
3
4
5
Eq $CuCl_2$
valeur 1
valeur 2

FIG.9

FIG.8

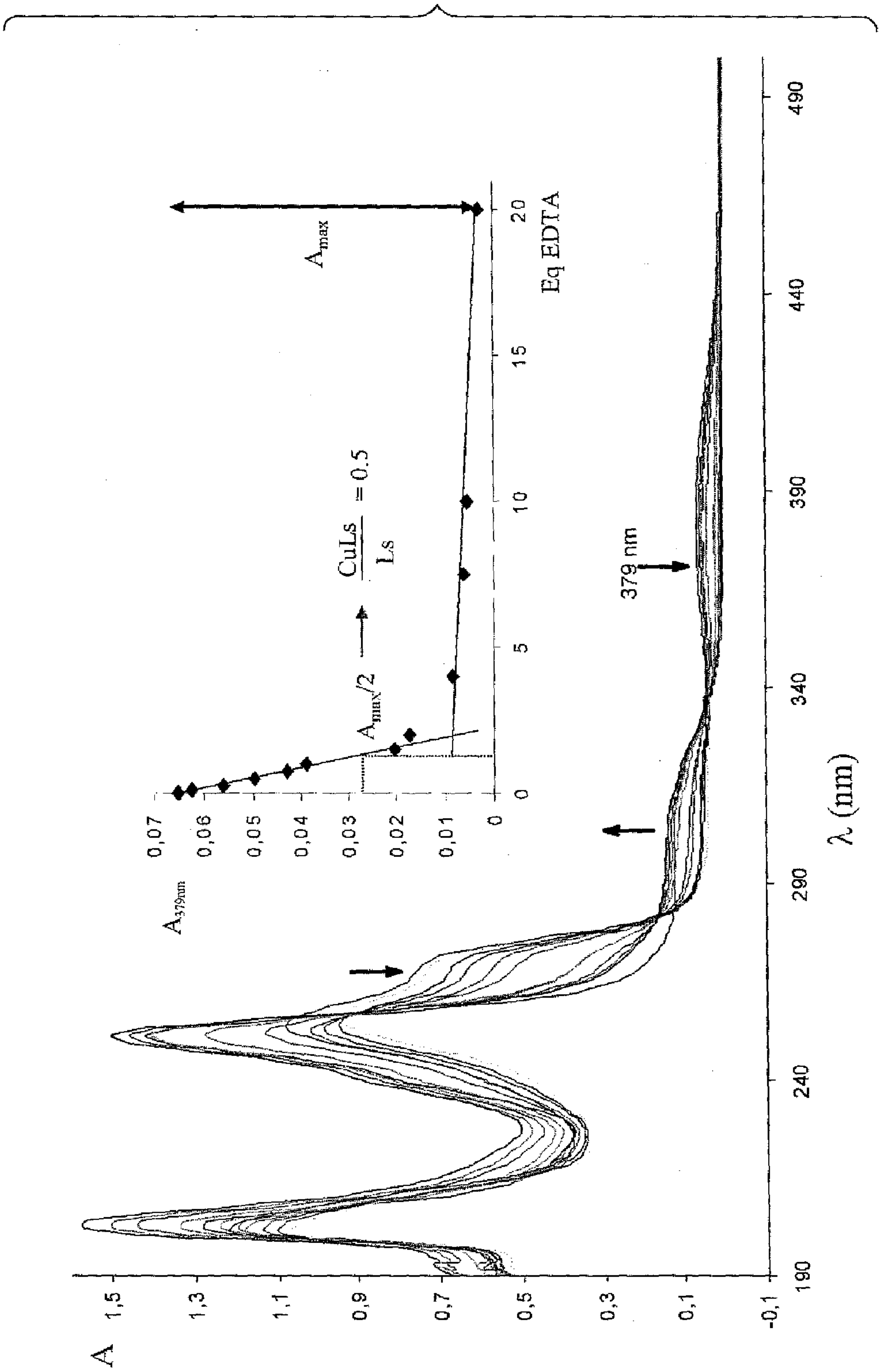
A
1,5
1,3
1,1
0,9
0,7
0,5
0,3
0,1
-0,1
190
240
290
340
390
440
490
λ (nm)
379 nm
$A_{379nm}$
0,07
0,06
0,05
0,04
0,03
0,02
0,01
0
0
5
10
15
20
Eq EDTA
$A_{max}$
$A_{max}/2$
CuLs / Ls = 0.5

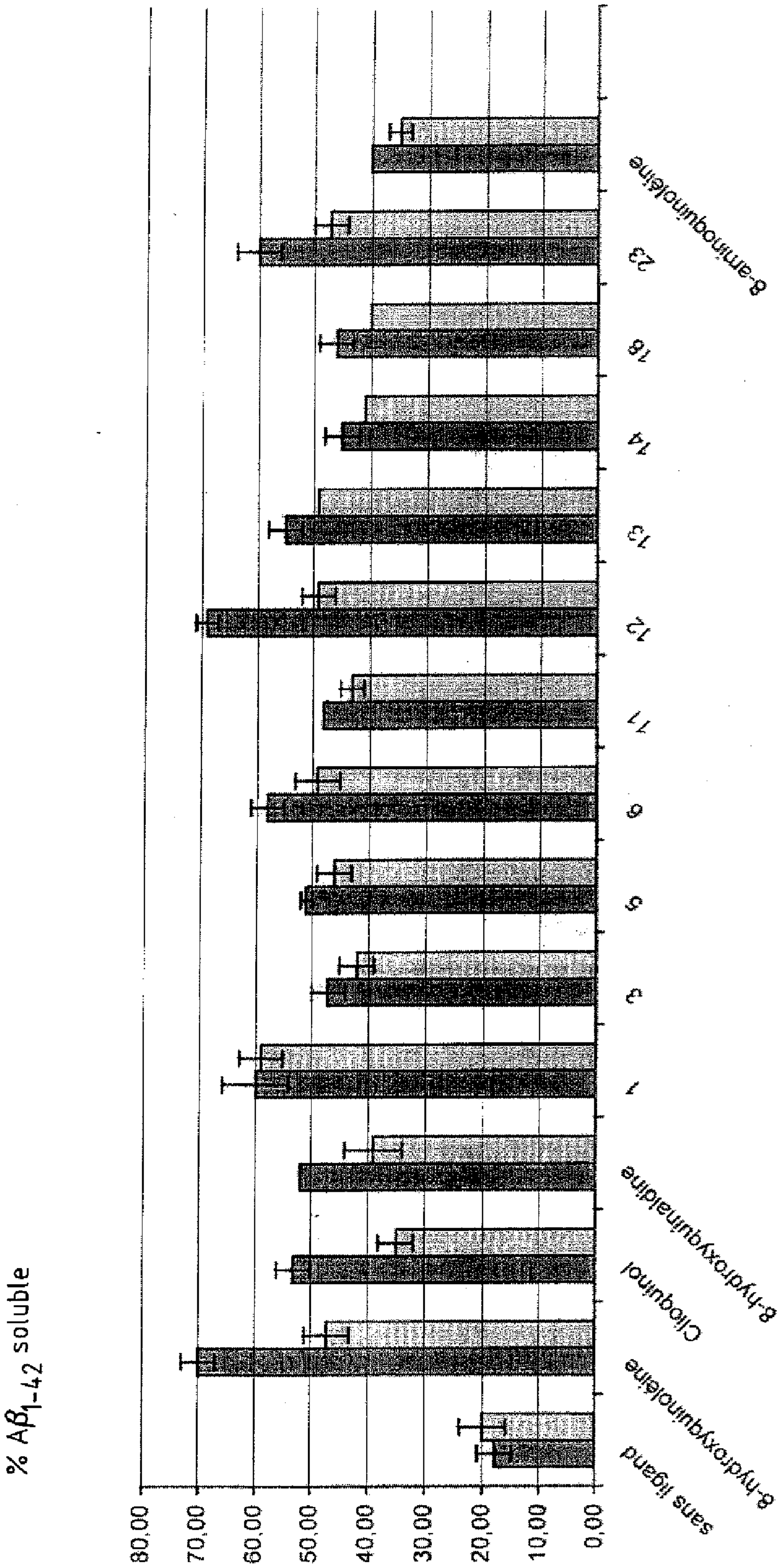
% $A\beta_{1-42}$ soluble
80,00
70,00
60,00
50,00
40,00
30,00
20,00
10,00
0,00
sans ligand
8-hydroxyquinoléine
Clioquinol
8-hydroxyquinaldine
1
3
5
9
11
12
13
14
18
23
8-aminoquinoléine

FIG.10

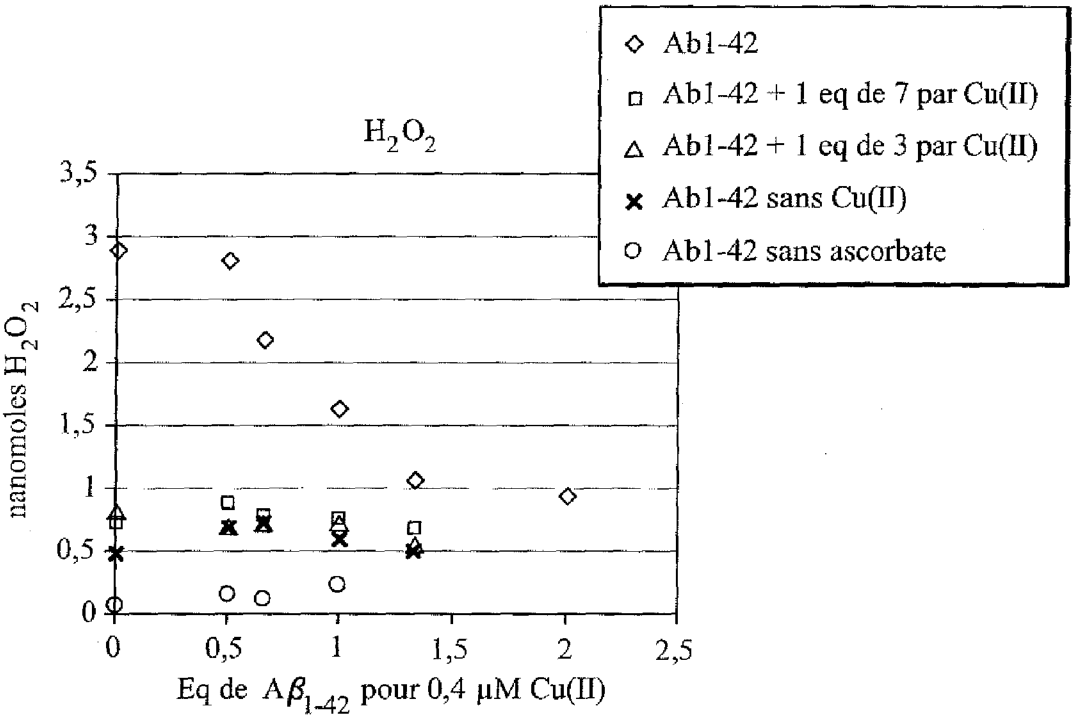
H2O2
Ab1-42
Ab1-42 + 1 eq de 7 par Cu(II)
Ab1-42 + 1 eq de 3 par Cu(II)
Ab1-42 sans Cu(II)
Ab1-42 sans ascorbate
3,5
3
2,5
2
1,5
1
0,5
0
nanomoles H2O2
0
0,5
1
1,5
2
2,5
Eq de Aβ1-42 pour 0,4 µM Cu(II)

FIG.11

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2004007461 A
- EP 0443862 A


### Littérature non-brevet citée dans la description


- **E. Bossy-Wetzel et al.** *Nature Medecine,* 2004, S2-S9
- **K. J. Barnham et al.** *Nature Rev. Drug Discov.,* 2004, vol. 3, 205-214
- **M. P. Mattson.** *Nature,* 2004, vol. 430, 631-639
- **P. M. Doraiswamy et al.** *The Lancet Neurol.,* 2004, vol. 3, 431-434
- **E. Quaglio et al.** *J. Biol. Chem.,* 2001, vol. 276, 11432-11438
- **D. Kaur et al.** *Neuron,* 2003, vol. 37, 899-909
- **M. Citron.** *Nature Rev. Neurosci.,* 2004, vol. 5, 677-685
- **M. P. Cuajungco et al.** *Ann. N. Y. Acad. Sci.,* 2000, vol. 920, 292-304
- **C. S. Atwood et al.** *Met. Ions Biol. Syst.,* 1999, vol. 36, 309-364
- **R. A. Cherny et al.** *Neuron,* 2001, vol. 30, 665-676
- **Sillen, L. G. et al.** Stability Constants of Metal-Ion Complexes. The Chemical Society London Publication, 1971
- **Stockwell et al.** *J. Am. Chem. Soc.,* 1999, 10662-10663
- **E. Scarpini et al.** *The Lancet Neurology,* 2003, vol. 2, 539-547
- **E. Gaggeli et al.** *Chem. Rev.,* 2006, vol. 106, 1995-2044
- **A. B. Clippingdale et al.** *J. Peptide Sc.,* 2001, vol. 7, 227-249
- **D.M. Maron ; B. N. Ames.** *Mutat. Res.,* 1983, vol. 113, 173-215
- **D . E. Levin et al.** *Mutat. Res.,* 1982, vol. 94, 315-330
- **D. E. Levin et al.** *Proc. Natl. USA,* 1982, vol. 79, 7445-7449
- **S.M. Berge et al.** *Pharmaceutical Salts, J. Pharm. Sci,* 1977, vol. 66, 1-19
- **Larock.** Comprehensive Organic Transformations. VCH Pub, 1989
- **T.W. Greene ; P.G.M. Wuts.** Protective Groups in Organic Chemistry. John Wiley and Sons, 1991
- **J.F.W. McOmie.** Protective Groups in Organic Chemistry. Plenum Press, 1973
- **C. Kitamura et al.** *J. Chem. Soc., Perkin Trans. 1,* 2000, 781-785
- **H. Jiang et al.** *Tetrahedron,* 2003, vol. 59, 8365-8374
- **Xue et al.** *Tetrahedron,* 2001, vol. 57, 7623-7628
- **P. Belser et al.** *Tetrahedron,* 1996, vol. 52, 2937-2944
- **D.E. Pearson et al.** *J. Org. Chem.,* 1967, vol. 32, 2358-2360
- **G. E. Collis et al.** *Acta Cryst.,* 2003, vol. C59, o443-o444
- **G. Xue et al.** *Tetrahedron,* 2001, vol. 57, 7623-7628
- **M. C. Kimber et al.** *Aust. J. Chem.,* 2003, vol. 56, 39-44
- **Y. Yamamoto et al.** *Bull. Chem. Soc. Jpn.,* 1978, vol. 51, 3489-3495
- **H. Gershon ; M. W. Mc Neil.** *J. Heterocycl. Chem.,* 1972, vol. 9, 659-666
- **H. Gershon et al.** *J. Heterocycl. Chem.,* 1972, vol. 9, 659-666
- **T. Garber et al.** *Inorg. Chem.,* 1990, vol. 29, 2863-2868
- **M. Albrecht et al.** *Synthesis,* 1999, vol. 10, 1819-1829
- **C.Kitamura et al.** *J. Chem. Soc., Perkin Trans 1,* 2000, 781-785
- **J. C. Peters et al.** *Inorg. Chem.,* 2001, vol. 40, 5083-5091
- **V. M. Dziomko et al.** *Yakugaku Zasshi,* 1951, vol. 71, 452-455
- **D. Planchenault et al.** *Tetrahedron,* 1995, vol. 51, 5823-5830
- **D. Nobel.** *J. Chem. Soc., Chem. Commun.,* 1993, 419-420
- **M. Numazawa et al.** *J. Chem. Soc., Chem. Commun.,* 1983, 533-534
- **D. E. Pearson et al.** *J. Org. Chem.,* 1967, 2358-2360
- **C. S. Atwood et al.** *J. Neurochem.,* 2000, vol. 75, 1219-1233

- **A. Ringblom.** Complexation in Analytical Chemistry. Interscience, 1963
- **G. Schwarznbach et al.** Complexometric Titrations. Meuthuen, 1969
- **C. S. Atwood et al.** *J. Biol. Chem,* 1998, vol. 273, 12817-12826
- **X. Huang et al.** *J. Biol. Chem.,* 1999, vol. 274, 37111-37116
- **X. Huang et al.** *Biochemistry,* 1999, vol. 38, 7609-7616
- **C. Opazo et al.** *J. Biol. Chem.,* 2002, vol. 277, 40302-40308
- **K. J. Barnham et al.** *J. Biol. Chem.,* 2003, vol. 278, 42959-42965
- **G. D. Ciccotosto et al.** *J. Biol. Chem.,* 2004, vol. 279, 42528-42534
- **Z.-P Zhuang et al.** *J. Med. Chem.,* 2001, vol. 44, 1905-1914
- **H. van de Waterbeemd et al.** *Nature Rev. Drug Discovery,* 2003, vol. 2, 192-204